# EUROPEAN PATENT SPECIFICATION

(11) **EP 1 140 209 B1**
(45) Date of publication and mention of the grant of the patent: **20.08.2008**
(21) Application number: 99963556.8
(22) Date of filing: 16.12.1999
(51) Int. Cl.: A61K 49/00

(54) **BLOOD POOL AGENTS FOR NUCLEAR MAGNETIC RESONANCE DIAGNOSTICS**
BLUT- POOL KONTRASTMITTEL ZUR VERWENDUNG IN DER MAGNETISCHEN RESONANZS-DIAGNOSTIK
AGENTS DE COMPARTIMENT SANGUIN POUR ETABLIR DES DIAGNOSTICS PAR RESONANCE MAGNETIQUE NUCLEAIRE

(30) Priority: 23.12.1998 IT MI982802
(43) Date of publication of application: 10.10.2001
(60) Divisional application: 08004643.6
(73) Proprietor: BRACCO IMAGING S.p.A., 20134 Milano (IT)
(72) Inventor: ANELLI, Pier Lucio, I-20134 Milano (IT); BROCCHETTA, Marino, I-20134 Milano (IT); DE HAËN, Christoph, I-20134 Milano (IT); GAZZOTTI, Ornella, I-20134 Milano (IT); LATTUADA, Luciano, I-20134 Milano (IT); LUX, Giovanna, I-20134 Milano (IT); MANFREDI, Giuseppe, I-20134 Milano (IT); MOROSINI, Pierfrancesco, I-20134 Milano (IT); PALANO, Daniela, I-20134 Milano (IT); SERLETI, Michele, I-20134 Milano (IT); UGGERI, Fulvio, I-20134 Milano (IT); VISIGALLI, Massimo, I-20134 Milano (IT)
(74) Representative: Minoja, Fabrizio
(86) International application number: PCT/EP1999/010002
(87) International publication number: WO 2000/038738

(56) References cited:
- EP-A- 0 279 307
- EP-A- 0 417 725
- WO-A-95/19186
- WO-A-95/32741
- BETEBENNER, DAVID A. ET AL: "Hepatobiliary delivery of polyaminopolycarboxylate chelates: synthesis and characterization of a cholic acid conjugate of EDTA and biodistribution and imaging studies with its indium-111 chelate" BIOCONJUGATE CHEM. (1991), 2(2), 117-23 , XP002137988

## Description

The present invention relates to the novel use of metal ion complexes of conjugates of bile acids with molecules having chelating activity as contrast agents in the diagnostic technique known as "magnetic resonance imaging" (M.R.I.), in particular as blood pool agents.

Complexes formed of chelating agents and suitable metals are already used as contrast agents in the following diagnostic techniques: X-ray imaging, nuclear magnetic resonance imaging (M.R.I.) and scintigraphy.

In particular, medical diagnosis using magnetic resonance imaging (M.R.I.), a recognized powerful diagnostic technique in clinical practice (Stark, D.D., Bradley, W. G., Jr., Eds. "Magnetic Resonance Imaging" The C. V. Mosby Company, St. Louis, Missouri (USA), 1988), mainly employs paramagnetic pharmaceutical compositions, preferably containing chelated complexes of bi-trivalent paramagnetic metal ions with polyaminopolycarboxylic ligands and/or their derivatives or analogues.

The images, basically deriving from the NMR signal of water protons, are the result of a complex interaction between different parameters, such as proton density and T₁ and T₂ relaxation times. A contrast enhancement can be obtained through the administration of exogenous chemical substances which significantly change the resonance properties of nearby water protons (see Lauffer, R.B. Chem. Rev. 1987, 87, 901).

The paramagnetic contrast agents used for N.M.R imaging act modifying the relaxation times of the water protons present in the tissues in which said contrast agents are concentrated, and they can therefore increase the contrast between different tissues, or between healthy and diseased tissues.

Gadolinium paramagnetic complexes, due to their high capability of reducing relaxation times of the protons of nearby water molecules through dipolar interaction, have been the object of studies, publications and patents.

Some of them are at present in clinical use as M.R.I. contrast agents:
Gd-DTPA, N-methylglucamine salt of the gadolinium complex with diethylenetriaminopentaacetic acid, MAGNEVIST^{®}; Gd-DOTA, N-methylglucamine salt of the gadolinium complex with 1,4,7,10-tetraazacyclododecane-1,4,7,10-tetraacetic acid, DOTAREM^{®}; Gd-HPDO3A, gadolinium complex with [10-(2-hydroxypropyl)-1,4,7,10-tetraazacyclododecane-1,4,7-triacetic acid, PROHANCE^{®}; Gd-DTPA-BMA, gadolinium complex with diethylenetriaminopentaacetic acid bis(methylamide), OMISCAN^{®}.

The contrast agents listed above and commercially available are intended for a general use. In fact, after administration, the M.R.I. said contrast agents diffuse in blood and in the extracellular areas of various parts of the body before being excreted. They are therefore similar, in this respect, to the iodinated compounds used for X-ray medical diagnosis.

At present, the medical profession is in need of contrast agents that are aimed at specific organs or for imaging of the blood system, which cannot be well defined by means of the products already commercially available. The first approach to obtain the latter consisted in covalently linking the contrast agent to macromolecules, such as proteins, or in inglobating it inside stable molecular aggregates such as liposomes, or still in using the so-called superparamagnetic particles.

For example, the gadolinium complex with diethylenetriaminopentaacetic acid (Gd-DTPA) was linked to human albumin (HSA), polylysine or dextran (Oksendal A. N. et al., J. Magn. Reson. Imaging, 157, 1993; Rocklage S. M., "Contrast Agents, Magn. Res. Imaging, Mosby Year Book, 372-437, 1992) in order to minimize or even suppress the diffusion from blood into the extracellular fluid thus providing a higher retention of the agent in the blood system. Such an approach, although attaining the desired effect, suffers from disagreeable side effects as the agent itself is excreted with difficulty.

A different strategy is the use of superparamagnetic particles coated with polyethylene, glycols or hydrocarbons in order to reduce the hepatic uptake by endothelial reticulum or by other systems (Tilcock C., Biochim. Biophys. Acta, 77, 1993; Bogdanoy A. A. et al., Radiology, 701,1993), thus prolonging the permanence of said agents in blood. In this case, also the above mentioned side effects occur, as well as problems due to the high production cost.

The demand for an efficient blood pool agent, having low toxicity and reasonably economic costs, is therefore still unmet.

The present invention relates to the novel use as blood pool agents of specifically selected compounds, already previously described by the Applicant in international patent application WO-A-95/32741 for the MR imaging of the hepatabiliary system. Said compounds result from the conjugation of a bile acid with a chelating agent, which are capable of chelating the ions of paramagnetic bi- trivalent metals.

It has surprisingly been found that a specific class of said compounds remains in the vascular system for a sufficiently long time so as to be valuable for use as contrast agents for the imaging of the vascular system, particularly of coronaries.

This effect can be clearly evidenced carrying out in vivo tests in animals (such as rabbit, monkey). The permanence in the vascular system can in fact be immediately visualized when plotting the proton relaxation values (1/T₁) of blood samples of the animal, taken at appropriate time intervals after administration of the contrast agent.

As Gd(III) complexes are paramagnetic species, high 1/T₁ values are evidence of high concentrations of the contrast agent in blood.

The difference between a conventional extracellular contrast agent and a blood pool agent, is well clarified in the paper by Lauffer et al., Radiology, 529,1998 in which T₁ profiles in blood as a function of time elapsed after the administration of the contrast agent are reported.

In particular, the complexes of the present invention, when administered for example to the rabbit at a dosage compatible with a reasonable safety index, are capable of inducing changes in relaxation rates (measured in Δ1/T₁) in blood higher than 5 s⁻¹ 10 minutes after administration, thus being promising for use as contrast agents for the imaging of the vascular system.

It has been found that this type of effect cannot be solely related to the presence of bile acids, but it depends on the chemical structure of said complexes. It seems, in fact, that the chelating unit should be preferably linked to the steroidal skeleton through a bond at the 3,7 or 12 positions of the bile acid.

It has, in fact, been proved that any linkage between the chelating unit and the bile acid involving the carboxylic group at the 24 position would yield complexes having unsatisfactory permanence in the vascular system.

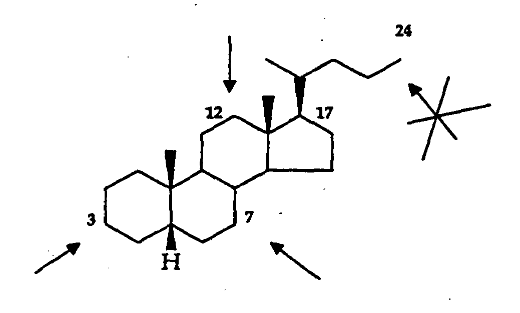

It is therefore an object of the present invention the use as blood pool agents of the complexes of the compounds of general formula (I) with paramagnetic bi-trivalent metal ions selected from the group consisting of Fe⁽²⁺⁾, Fe⁽³⁺⁾, Cu⁽²⁺⁾, Cr⁽³⁺⁾, Gd⁽³⁺⁾, Eu⁽³⁺⁾, Dy⁽³⁺⁾, Yb⁽³⁺⁾ or Mn⁽²⁺⁾,

X-L-Y (I),

in which
- x: is the residue of a polyaminopolycarboxylic ligand or derivatives thereof, selected from the group consisting of:
ethylenediaminotetraacetic acid (EDTA), diethylene-triaminopentaacetic acid (DTPA), 1,4,7,10-tetraaza-cyclododecane-1,4,7,10-tetraacetic acid (DOTA), 1,4,7,10-tetraazacyclododecane-1,4,7-triacetic acid (D03A), [10-(2=hydroxypropyl)-1,4,7,10-tetraaza-cyclododecane-1,4,7-triacetic acid (HPDO3A), 4-carboxy-5,8,11-tris(carboxymethyl)-1-phenyl-2-oxa-5,8,11-triazatridecan-13-oic acid (BOPTA);
- Y: is the derivative of a bile acid selected from the group consisting of residues of cholic, chenodeoxycholic, deoxycholic, ursodeoxycholic, lithocholic acids, 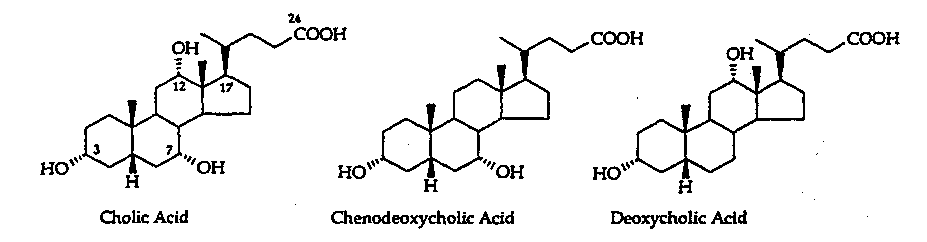 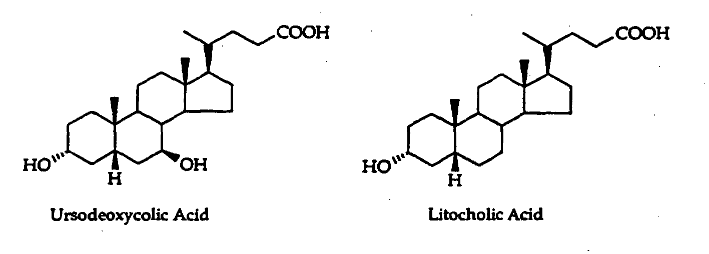 both as they are and functionalized at the positions having the hydroxy group as the reactive group, independently of the stereochemistry of the final products,
said derivative also comprising the conjugate of the acid group at the 24 position with taurine and glycine;
- L: is a chain linked to any position of X, optionally comprising one of the carboxylic groups which is thus transformed into an amido group, and to the C-3, C-7, C-12 positions of Y, and it has the following formula (II) 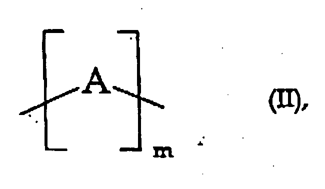 in which
- m: is an integer ranging from 1 to 10, wherein for values above 1 A can have different meanings,
- A: has the following formula (III),

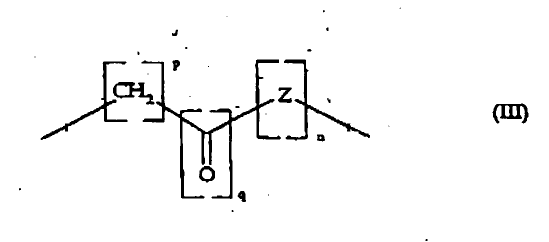

- n and q: can be 0 or 1, but they are not at the same time zero,
- p: can range from 0 to 10,
- z: is an oxygen atom or a -NR group,
in which
- R: is a hydrogen atom, or a (C₁-C₅) alkyl group either unsubstituted or substituted by a -COOH group. particularly preferred compounds are those in which the spacing chains L have the following general formulae (IIIa) and (IIIb).

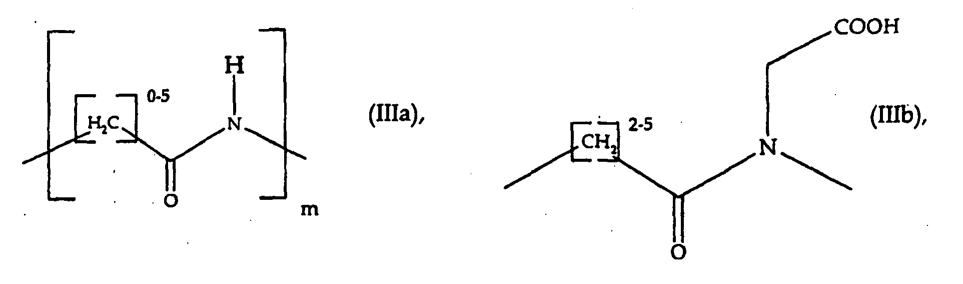

Also preferred are the structures in which Z is the oxygen atom and therefore L is formed through the hydroxy groups present at the 3, 7, 12 positions, independently of the stereochemistry of the final products.

Particularly preferred are the compounds of formula (I) in which the residue X is selected from the group consisting of: EDTA, DTPA, DOTA, DO3A, BOPTA; L is selected from the group consisting of (IIIa), (IIIb);

Y is preferably selected from the group consisting of residues of cholic, deoxycholic, chenodeoxycholic, lithocholic acids, linked to L by an amino group at the 3- position and the acidic group at the 24 position is present as it is or as its taurine or glycine derivative.

Y can also be differently functionalized, for example through conversion of one or more hydroxy groups into keto groups.

Particularly preferred complexes with paramagnetic metal ions defined above are the complexes with gadolinium or with manganese.

Preferred are the compounds of general formula (IV), in which in general formula (I) the residue X is DTPA, substituted on the central chain, and in which R₁ is a hydrogen atom or the -COOH group, 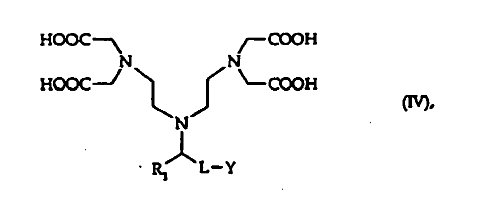
- Y: is selected from the group consisting of cholic, deoxycholic, chenodeoxycholic, lithocholic residues and L has the structure of formula (III).

Particularly preferred are compounds of general formula (IVa) 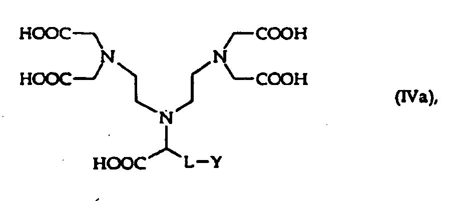 in which R₁ is the -COOH group and Y has the meanings defined above for the compounds of general formula (IV) and L has the structures (IIIa) or (IIIb).

Further object of the present invention is the use of the following compounds, belonging to the class of general formula (IVa)
[3β(S)5β]-3-[[4-[bis(2-[bis(carboxymetbyl)amino]ethyl] amino]-4-carboxy-1-oxo-butyl](carboxymethyl)amino]cholan-24-oic acid 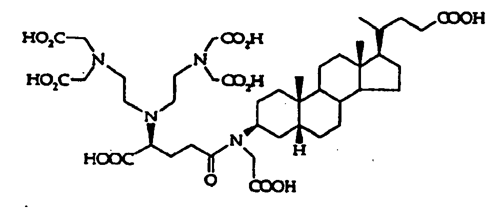 [3β(S),5β] -3- [[4- [bis [2- [bis (carboxymethyl) amino] ethyl] amino]-4-carboxy-1-oxobutyl]amino]cholan-24-oic acid; 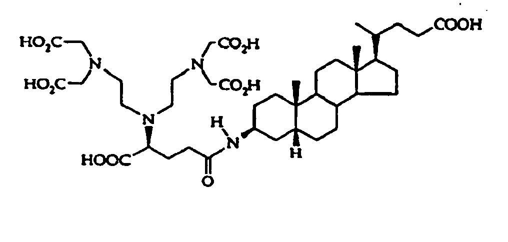 [3β(S),5β,7β]-3- [[4-[bis [2- [bis(carboxymethyl) amino] ethyl]amino]-4-carboxy-1-oxobutyl]amino]-7-hydroxycholan-24-oic acid; 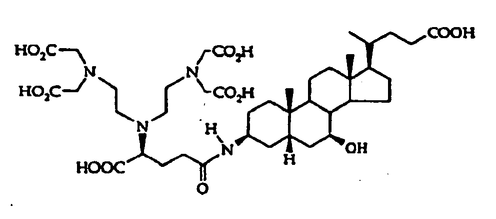 [3α(S) 5β] -3-[[4- [bis[2-[bis(carboxymethyl)amino] ethyl]amino]-4-carboxy-1-oxobutyl-amino]cholan-24-oic acid; 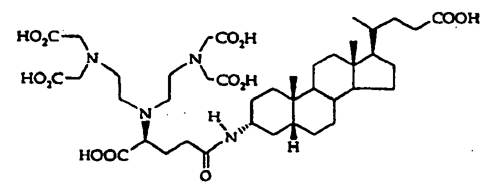 [3β(S),5β]-3-[[4-[bis[2-[bis(carboxymethyl)amino]ethyl] amino]-4-carboxy-1-oxobutyl]amino]-12-oxocholan-24-oic acid; 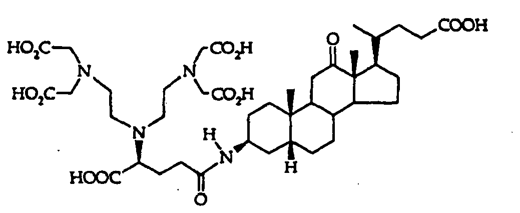 [3β(S),5β,7α]-3-[[4-[bis[2-[bis(carboxymethyl)amino] ethyl]amino]-4-carboxy-1-oxobutyl]amino]-7-hydroxycholan-24-oic acid; 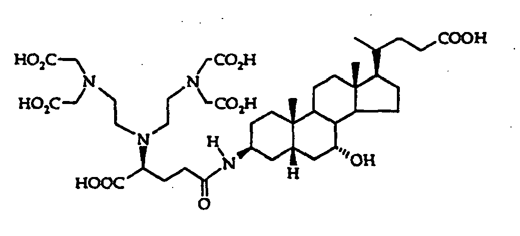 N²-bis[2-[bis(carboxymethyl)aminolethyl]-N-[(3β,5β,-7α,12α)-7,12-dihydroxy-24-oxo-24-[(2-sulfoethyl)amino] cholan-3-yl]-L-glutamine 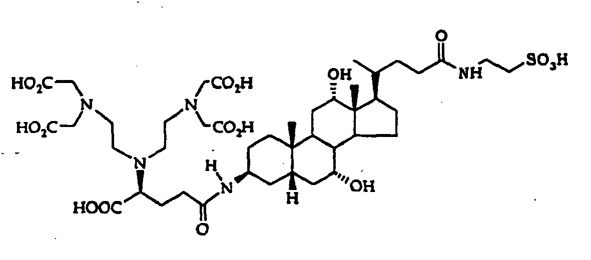 N²-bis[2-[bis(carboxymethyl)amino]ethyl]-N-[(3β,5β)-24-oxo-24-[(2-sulfoethyl)amino]cholan-3-yl]-L-glutamine; 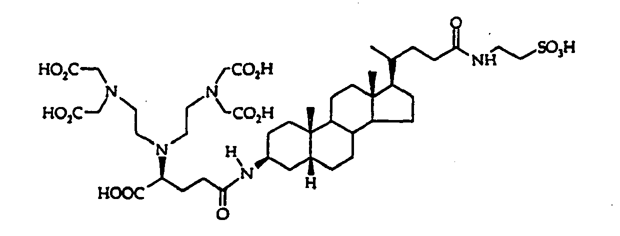 [3β(S),5β,12α]-3-[[4-[bis[2-[bis(carboxymethyl)amino] ethyl]amino]-4-carboxy-1-oxobutyl]amino]-12-hydroxycho-lan-24-oic acid; 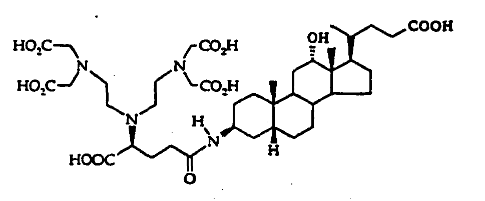 [3β(R),5β,12α]-3-[[4-[bis[2-[bis(carboxymethyl)amino] ethyl]amino]-4-carboxy-l-oxobutyl]amino]-12-hydroxycho-lan-24-oic acid; 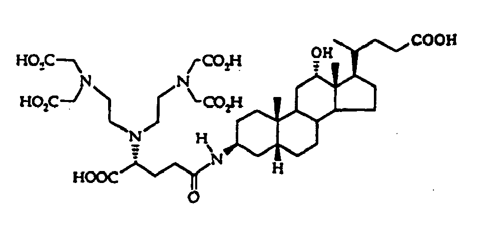 [3β(RS),5β,12α]-3-[[4-[bis[2-[bis(carboxymethyl)amino] ethyl]amino]-4-carboxy-1-oxobutyl]amino]-12-hydroxycho-lan-24-oic acid; 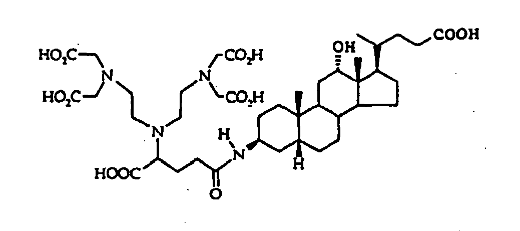 [3α(S),5β,12α]-3-[[4-[bis[2-[bis(carboxymethyl)amino] ethyl]amino]-4-carboxy-1-oxobutyl]amino]-12-hydroxycho-lan-24-oic acid; 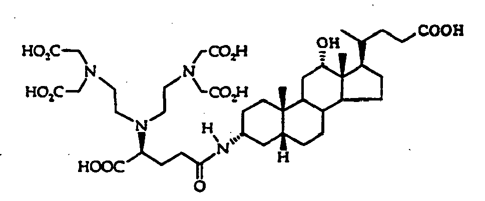 [3β(RS)5β,7α,12α]-3-[[4-[bis(2-[bis(carboxymethyl) amino]ethyl]amino]-4-carboxy-1-oxobutyl]amino]-7,12-dihydroxycholan-24-oic acid; 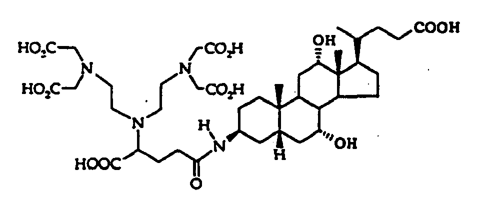 [3α(S),Sβ,7α,12α]-3-[[[[5-[bis[2-[bis(carboxymethyl) amino] ethyl] amino] -5-carboxypentyl] amino] carbonyl] oxyl - 7,12-dihydroxycholan-24-oic acid; 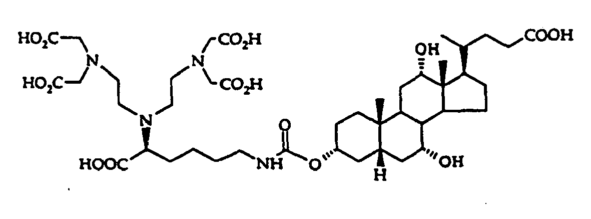 [3α(S),5β]-3-[2-[[5-[bis[2-[bis(carboxymethyl)amino] ethyl]amino]-5-carboxypentyl]amino]-2-oxoethoxy]cholan-24-oic acid.

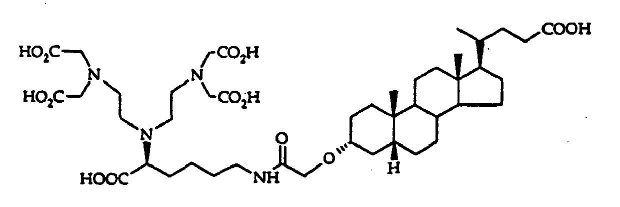

Other compounds belonging to this class, whose complexes with gadolinium have been described in patent application WO-A-95/32741, are the following:
[3β(S),5β,7α,12α]-3-[[4-[bis[2-[bis(carboxymethyl)amino] ethyl]amino]-4-carboxy-l-oxobutyl]amino]-7,12-dihydroxy-cholan-24-oic acid; 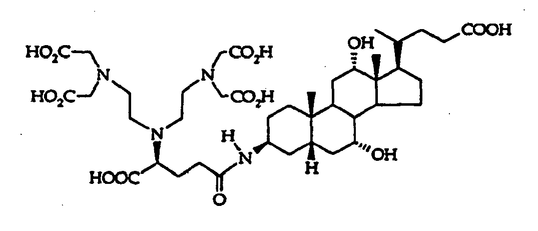
[3β(S),5β,7α,12α]-3-[[4-[[5-[bis[2-[bis(carboxymethyl) amino]ethyl]amino]-5-carboxypentyl]amino]-1,4-dioxo-butyl]amino]-7,12-dihydroxycholan-24-oic acid.

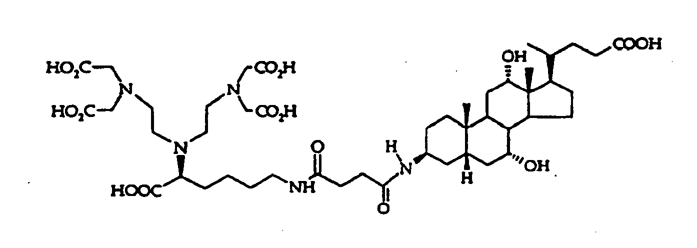

Also preferred are the compounds of general formula (IVb), which also are DTPA derivatives substituted at the central position, 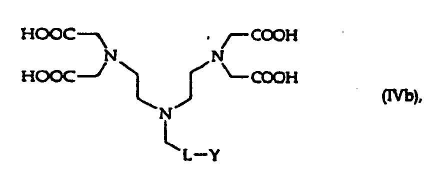 in which Y has the meanings defined above for compounds of formula (IV) and L has the structure (IIIa).

The present invention further relates to the use of the following compounds, that belong to the class of general formula (IVb)
(3β,5β,7α,12α)-3- [[[bis[2-[bis(carboxymethyl)amino] ethyl]amino]acetyl]amino]-7,12-dihydroxycholan-24-oic acid 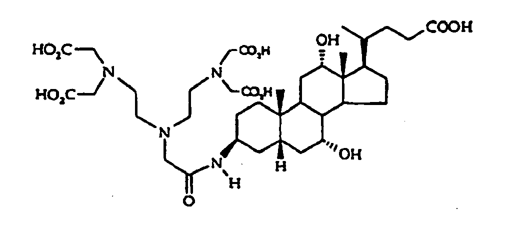
(3β,5β)-3-[[[([bis[2-[bis(carboxymethyl)amino]ethyl] amino]acetyl]amino]acetyl]amino]cholan-24-oic acid

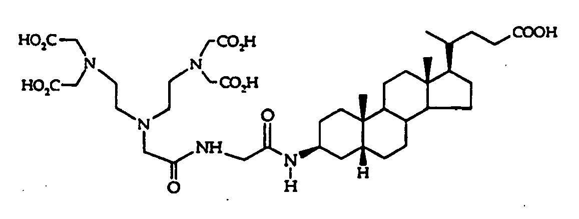

Other compounds belonging to this class, whose complexes with gadolinium have been described in patent application WO-A-95/32741, are the following :
(3β,5β,7α,12α)-3-[[[[[bis[2-[bis(carboxymethyl)amino] ethyl]amino]acetyl]amino]acetyl]amino]-7,12-dihydroxy-cholan-24-oic acid; 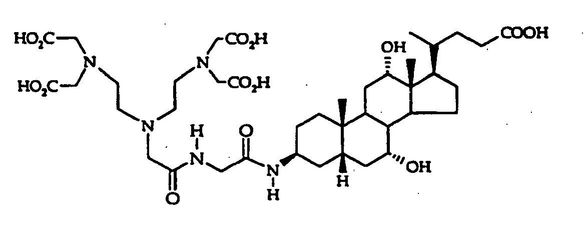
(3β,5β,7α,12α)-3-[[6-[[[bis[2-[bis(carboxymethyl)amino] ethyl]amino]acetyl]amino]-1-oxohexyl]amino]-7,12-dihydro-xycholan-24-oic acid.

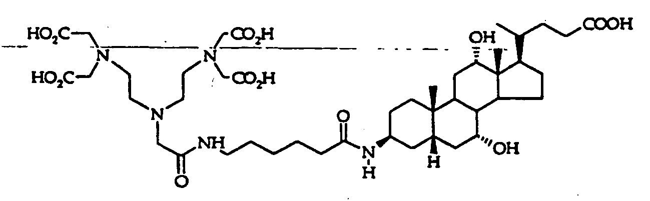

Particularly preferred also are the compounds of general formula (V), in which in general formula (I) the residue X is DTPA, Y has the meanings defined above for compounds (IV) and L has the structure of formula (IIIa).

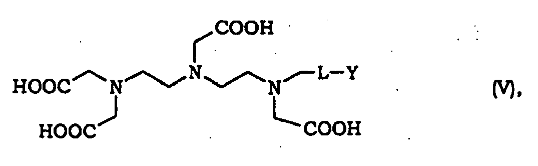

Other compounds belonging to this class, whose complexes with gadolinium have been described in patent application WO-A-9S/32741, are the following:
(3β,5β,7α,12α)-3-[[N-[N-[2-[[2-[bis(carboxymethyl)amino] ethyl](carboxymethyl)amino]ethyl]-N-(carboxymethyl)glycyl]glycyl]amino]-7,12-dihydroxycholan-24-oic acid; 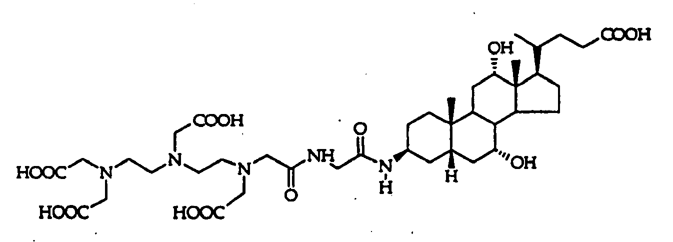
18-[[(3β,5β,7α,12α)-23-carboxy-7,12-hydroxy-24-norcholan-3-yl]amino]-3,6,9-tris(carboxymethyl)-11,18-dioxo-3,6,9,12-tetraazaoctadecanoic acid.

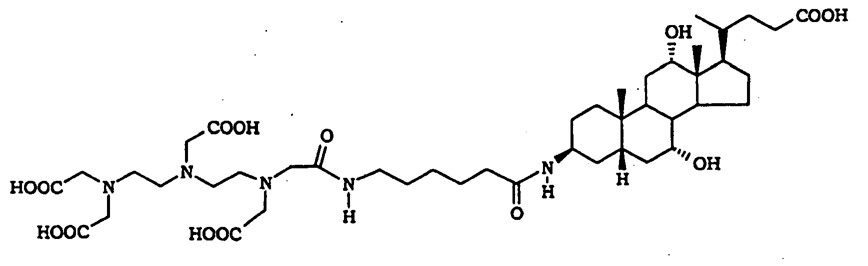

Also preferred are the compounds of formula (VI), in which in formula (I) the residue X is DO3A, Y has the meanings defined above for compounds of formula (IV) and L is selected from the structures (IIIa) and (IIIb).

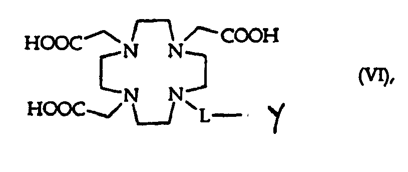

Among the compounds of formula (VI), particularly preferred is 10-[3-[((3α,5β,7α,12β)-23-carboxy-7',12-di-hydroxy-24-norcholan-3-yl]oxy]-2-hydroxypropyl]-1,4,7,10-tetraazacyclododecane-1,4,7-triacetic acid, whose complex with gadolinium has been described in patent application WO-A-95/32741.

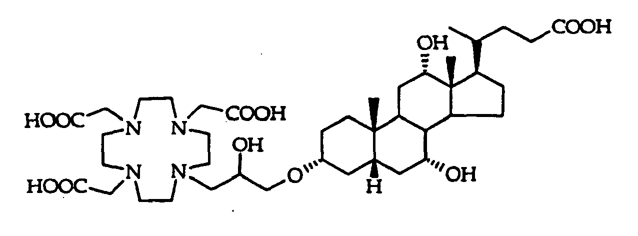

Similarly, the compounds of general formula (VII) are preferred, in which in formula (I) the residue X is EDTA, Y has the meanings defined above for compounds of formula (IV) and L has the structure of formula (III).

Particularly preferred are the complexes of compounds of formula (VII) with manganese.

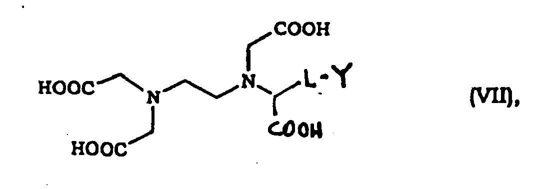

Among the compounds of formula (VII) particularly preferred are the following:
[3α(S),5α,12α]-3-[[[[5-[[2-[bis(carboxymethyl)amino] ethyl](carboxymethyl)amino]-5-carboxypentyl]amino]carbonyl]oxy]-12-hydroxycholan-24-oic acid 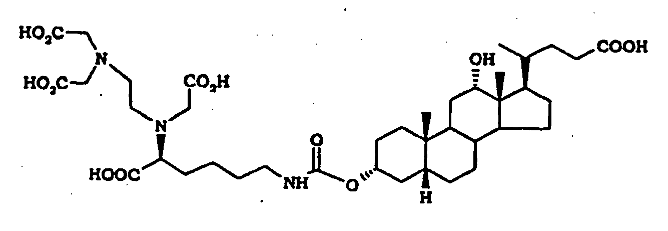
[3β(S),5β,7α,12α]-3-[[4-[[5-[[2-[bis(carboxymethyl)amino]ethyl](carboxymethyl)amino]-5-carboxypentyl]amino]-1,4-dioxobutyl]amino]-7,12-dihydroxycholan-24-oic acid 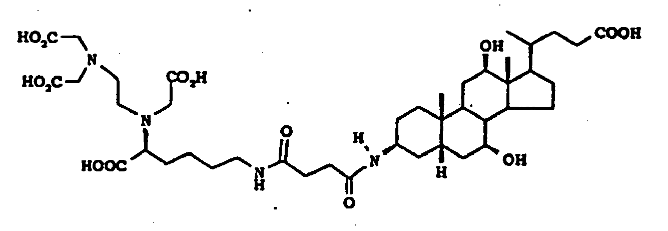
[3α(S),5β]-3-[2-[[5-[[2-[bis(carboxymethyl)amino] ethyl](carboxymethyl)amino]-5-carboxypentyl]amino]-2-oxoethoxy]cholan-24-oic acid 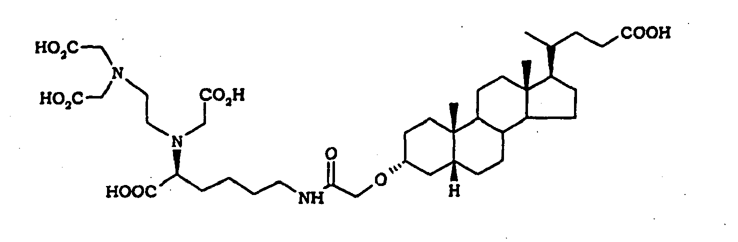
[3β(S),5β,12α]-3- [[4- [[2-[[bis (carboxymethyl) amino] ethyl] (carboxymethyl) amino] -4-carboxy-1-oxobutyl] amino] 12-hydroxycholan-24-oic acid 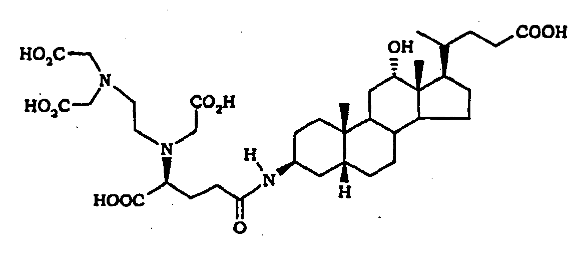
[3β(S),5β]-3-[[4-[[2-[[bis(carboxymethyl)amino]ethyl] (carboxymethyl)amino]-4-carboxy-1-oxobutyl]amino]-12-oxocholan-24-oic acid

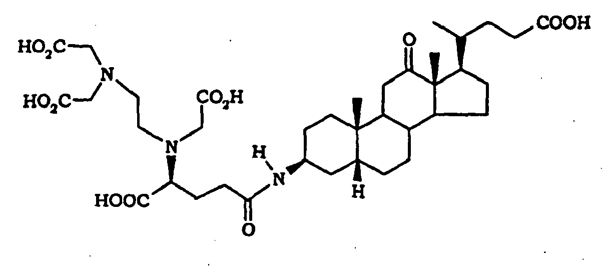

The compounds of general formula (I) can be prepared with the method of convergent synthesis which comprises:
1) synthesis of a functionalized ligand, i.e. of a ligand capable of coordinating one paramagnetic metal ion while binding stably to the bile acid by means of a suitable functional group;
2) synthesis of a functionalized bile acid;
3) coupling reaction between the two different syntons;
4) cleavage of any protective groups;
5) complexation of the paramagnetic metal ion;
illustrated in detail in the above cited patent application WO-A-95/32741.

Some of the preferred processes for the preparation of the ligands of the present invention involve the formation of an amide bond between two syntons, one of them being the precursor of the chelating system of the paramagnetic ion (Synton A), the other being the precursor of the bile acid residue contained in the final complex (Synton B).

The methods described hereinbelow should not be considered limiting the synthesis of the compounds of the present invention.

The amide bond can be formed:
a) by reacting Syntons A containing a carboxylic function with Syntons B containing a primary or secondary amino function;
b) by reacting Syntons A containing a primary or secondary amino function with Syntons B containing a carboxylic function;
c) by reacting DTPA dianhydride (commercially available product) with a Synton B containing a primary or secondary amino function.

A list of some of the syntons A and B used for this invention is reported in the following Table.

**Table**

| Synton A | Synton B |
|---|---|
| 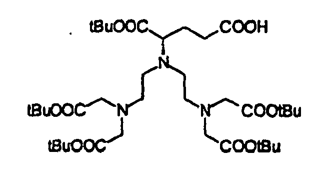 | 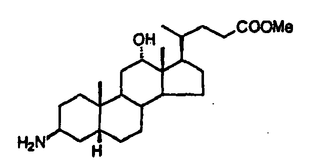 |
| 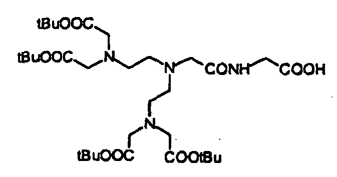 | 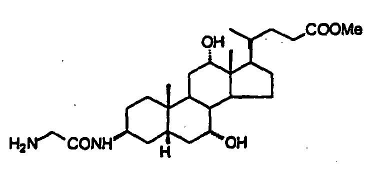 |
| 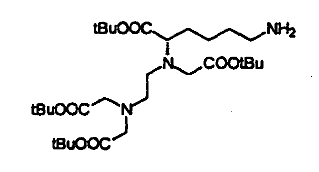 | 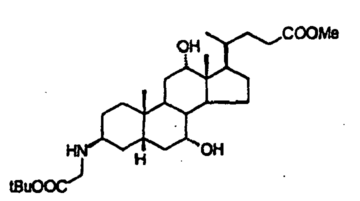 |
| 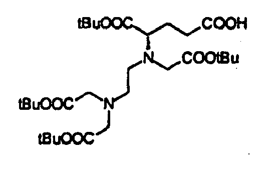 | 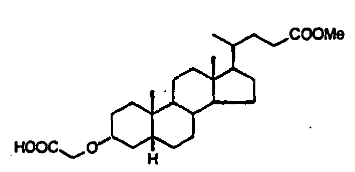 |
| | 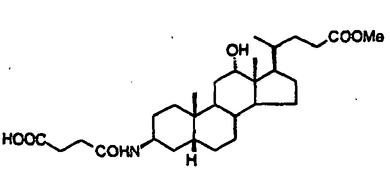 |

The Syntons used are of course suitably protected at those groups which could give rise to parasitic reactions under the conditions used for the formation of the amide bond. After formation of the bond between the two syntons, one or more deprotection steps for restoring the original groups should be contemplated.

Alternatively to this type of processes, the chelating subunit can be introduced by multi-step reactions starting from a bile acid derivative, as in the case of the synthesis of the compound described in Example 3 of the Experimental section, illustrated in the following Scheme 1.

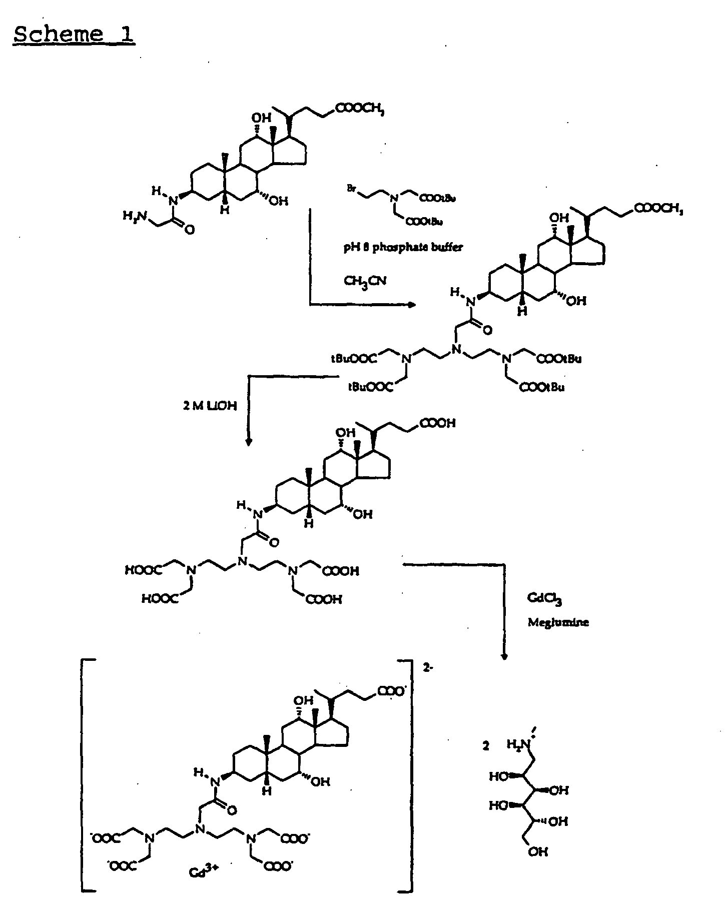

The present invention also relates to a novel process illustrated in the following Scheme 2: 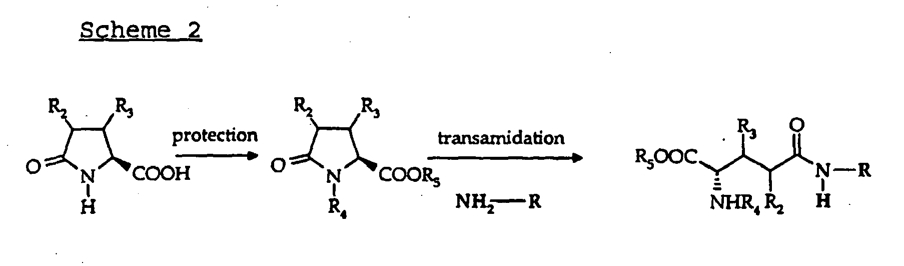 in which
- R₄: is an amino-protecting group;
- R₅: is a straight or branched C₁-C₁₀ alkyl or aryl,
- R₂ and R₃: are independently a hydrogen atom, straight or branched C₁-C₂₀ alkyl, unsubstituted or substituted by aryl groups, or said groups form a C₃-C₁₀ cycle;
which process makes use of a transamidation reaction and allows to maintain the stereochemistry at the chiral centre adjacent to the nitrogen atom of the starting pyrrolidinone and to afford a secondary amide. The combined selection of groups R₄ and R₅ is important in that the cleavage should take place under diversified conditions. Possible examples of R₄ are the carbobenzyloxy (Cbz) group and of R₅ are the methyl group or the t-butyl group.

This process is applicable generally in order to obtain glutamic acid γ-amides and it is very useful and advantageous for the preparation of the compounds of the invention, in particular glutamic acid γ-amides with 3-amino derivatives of residues Y, as defined above. In fact, it allows to obtain the final compound avoiding the use of the expensive condensing agents for the formation of the γ-amido bond between glutamic acid and the corresponding amine.

An example of the application of this entirely novel synthetic procedure is the synthesis of [3β(S),5β,12α]-3-[[4-[bis[2-[bis(carboxymethyl)-amino]ethyl]amino]-4-car-boxy-1-oxobutyl]amino]-12-hydroxycholan-24-oic acid, whose conventional synthesis is reported in Example 4 of the Experimental section, whereas the alternative one is reported in Example 5 and the complete synthetic scheme is shown in the following Scheme 3.

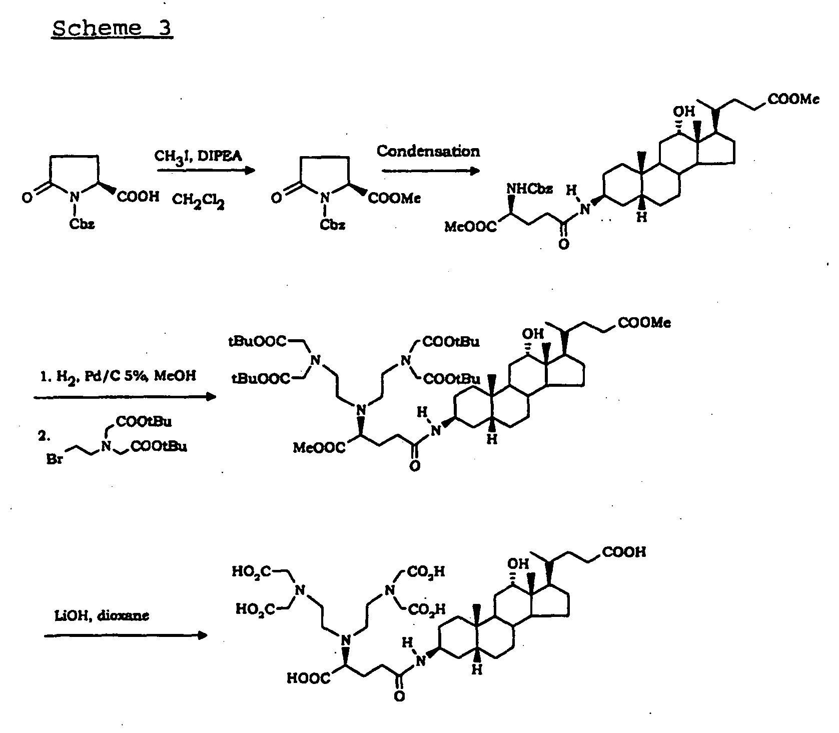

Analogously was prepared the cholic acid derivative already described in patent application WO-A-95/32741, [3β(S),5β,7α,12α]-3-[[4-[bis[2-[bis(carboxymethyl)amino]-ethyl]amino]-4-carboxy-1-oxobutyl]amino]-7,12-dihydroxy-cholan-24-oic acid.

Metal ions suitable to form complex salts with the chelating agents of general formula (I) are the bivalent or trivalent ions of the elements selected from the group consisting of: Fe⁽²⁺⁾, Fe⁽³⁺⁾, Cu⁽²⁺⁾, Cr⁽³⁺⁾, Gd⁽³⁺⁾, Eu⁽³⁺⁾, Dy⁽³⁺⁾, Yb⁽³⁺⁾ or Mn⁽²⁺⁾.

As for the diagnostic use of the novel chelated complexes of the invention, they can be used as contrast agents, particularly for use as blood pool agents in the imaging diagnostic technique by means of magnetic resonance.

The preparation of the complexes is carried out conventionally, according to a process in which the oxide or the suitable salt of the paramagnetic metal, dissolved in water or suspended in a water-alcohol solution, is added to an aqueous or water-alcohol solution of the chelating agent, under stirring and, if necessary, heated mildly or to the boiling temperature, until completion of the reaction. If the complex is insoluble in the reaction solvent, it can be filtered. If it is soluble, it can be recovered by evaporating off the solvent to a residue, for example through spray drying.

In case the resulting complex still contains free acidic groups, it is transformed into a neutral salt by reaction with an organic or inorganic base which forms physiologically compatible cations in solutions.

For the preparation of these neutral salts, a sufficient amount of the base can be added to the complexes containing free acidic groups in aqueous solution or suspension to neutrality. The resulting solution can thus be conveniently evaporated or a suitable solvent can be added to crystallize the complex salt.

Preferred inorganic cations suitable for salifying the chelated complexes of the invention comprise in particular alkali or alkaline-earth metal ions such as potassium, sodium, calcium, magnesium, and the mixtures thereof. Particularly preferred is the sodium ion.

Preferred cations deriving from organic bases suitable for the above mentioned purpose comprise, inter alia, those of primary, secondary and tertiary amines, such as ethanolamine, diethanolamine, morpholine, glucamine, N-methylglucamine, N,N-dimethylglucamine, N-methylglucamine being particularly preferred.

Preferred cations deriving from amino acids comprise, for example, those of taurine, glycine, lysine, arginine or ornithine.

An alternative to this process consists in preparing the injectable formulation without isolating the complex salt. In this case it is mandatory for the final solutions to contain no free metal ions, which are toxic for the body.

This can be checked by titration, for example, with colored indicators such as Xylenol Orange. A final purification step of the complex salt could also be envisaged.

In this type of process the chelating agent, the salt or the metal oxide, and any salifying bases, are reacted in the stoichiometric ratios in water for injections, then, after completion of the reaction, pyrogens are filtered off and the product is distributed in suitable containers and then thermally sterilised.

The injectable pharmaceutical formulations are typically prepared by dissolving the active ingredient, prepared as described above, and the excipients in water of suitable purity from the pharmacological point of view, so as to provide a pharmaceutical formulation suitable for the enteral or parenteral administration, in concentrations ranging from 0.01 to 1.0 molar. The resulting contrast agent is suitably sterilised.

The contrast agents are administered, depending on the diagnostic requirements, at a dose of 0.01 - 0.3 mmol/kg body weight.

In principle, the doses for the parenteral administration range from 0.001 to about 1.0 mmol/kg body weight. The preferred doses for the parenteral administration range from 0.01 to about 0.5 mmol/kg body weight.

The doses for the enteral administration range, in general, from 0.5 to about 10 mmol/kg, preferably from about 1.0 to about 10 mmol/kg body weight.

The novel formulations of the present invention show good tolerability; moreover their water solubility is a further, important feature which makes them particularly suitable for use in nuclear magnetic resonance.

The diagnostic compositions of the present invention are used conventionally. The compositions can be administered to a patient, typically a hot-blooded animal, both systemically and topically in the organ or in the tissue to be visualized by magnetic resonance.

The analysis protocols and the apparatuses can be found in works such as Stark, D. D., Bradley, W. G., Magnetic Resonance Imaging, Mosby Year Book, St. Louis, Mo, 1992.

The experimental conditions used will be illustrated in detail in the Experimental section.

### Experimental section

### EXAMPLE 1

### Gadolinium complex of [3β(S),5β]-3-[[4-[bis[2-[bis-(carboxymethyl)amino]ethyl]amino]-4-carboxy-1-oxobutyl] amino]cholan-24-oic acid salified with 1-deoxy-1-(methylamino)-D-glucitol (1:3)

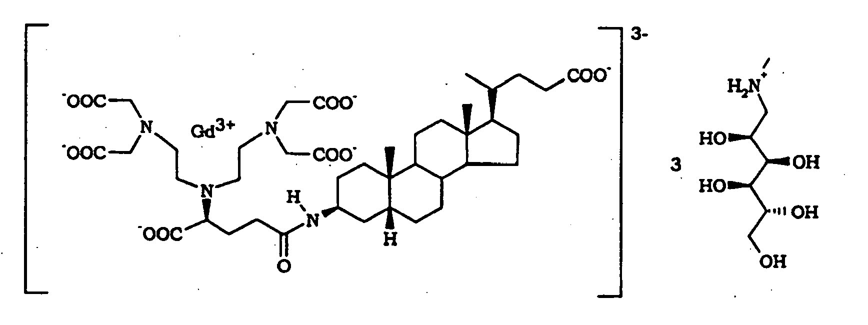

### A) [3β(S),5β]-3-[[5-(1,1-Dimethylethoxy)-4-[bis[2-[bis[2-(1,1-dimethylethoxy)-2-oxoethyl]amino] ethyl]amino]-1,5-dioxopentyl]amino]cholan-24-oic acid methyl ester

3.6 g of (3β,5β)-3-aminocholan-24-oic acid methyl
ester (prepared analogously to the procedure described in WO-A-95/32741: example 5) (9.24 mmol), 8.5 g of N,N-bis[2-[bis[2-(1,1-dimethylethoxy)-2-oxoethyl)amino)-ethyl]-L-glutamic acid t-butyl ester (prepared as described in WO-A-95/32741: example 15) (11.39 mmol) and 1.64 g of diethyl cyanophosphonate (9.39 mmol) are dissolved in 160 mL of DMF. The solution is cooled to 0°C, 1.28 mL of Et₃N (9.24 mmol) are dropped therein and the reaction mixture is left for 30 min at room temperature. After 1 h the solution is evaporated under reduced pressure, the residue is taken up with EtOAc, washed with 5% NaHCO₃ and then with brine. The organic phase is separated, dried over Na₂SO₄ and then evaporated under reduced pressure. The crude is purified by flash-chromatography to obtain 9.5 g of the desired product (8.50 mmol).
Yield: 92%
K.F.: 3.47%

| Elemental analysis | C | H | N |
|---|---|---|---|
| % calc.: | 66.63 | 9.74 | 5.01 |
| % found:^{a} | 67.42 | 10.08 | 5.07 |

| | | | |
|---|---|---|---|
| *^{a}* after drying at 120 °C under vacuum | | | |

TLC: Stationary phase: silica gel plate 60F 254 Merck
Eluent = 4 : 6 EtOAc/n-hexane
Detection: 0.5% KMnO₄ in 1 M NaOH R_{f}= 0.46
The ¹H-NMR, ¹³C-NMR, IR and MS spectra are consistent with the indicated structure.

### B) [3β(S),5β]-3-[[4-Carboxy-4-[bis[2-[bis(carboxyethyl) amino]ethyl]amino]-1-oxobutyl]amino]cholan-24-oic acid methyl ester

A stirred solution of 9.3 g of the compound prepared at step A) (8.32 mmol) in 50 mL of CH₂Cl₂ is added with 5.1 mL of CF₃COOH (66.6 mmol); after 10 min at a temperature of 0-5 °C the solution is evaporated. The residue is taken up into 50 mL of CF₃COOH and, after 24 h at room temperature, added with further 30 mL of CF₃COOH to complete the reaction. After 5 h the reaction mixture is evaporated and the residue is treated with CH₂Cl₂, evaporating each time the solvent under reduced pressure until obtaining a powder. The solid is washed with H₂O, filtered and dried to obtain the desired product (6.9 g; 8.24 mmol).
Yield: 99% m.p.: 205°C
K.F.: 7.78%

| Elemental analysis | C | H | N |
|---|---|---|---|
| % calc.: | 60.27 | 8.18 | 6.69 |
| % found:^{a} | 59.28 | 8.11 | 6.68 |

| | | | |
|---|---|---|---|
| *^{a}* after drying at 120 °C under vacuum | | | |

TLC: Stationary phase: silica gel plate 60F 254 Merck
Eluent = 6 : 4 : 1 CHCl₃/MeOH/25% NH₄OH.
Detection: 0.5% KMnO₄ in 1 M NaOH R_{f}= 0.28
The ¹H-NMR, ¹³C-NMR, IR and MS spectra are consistent with the indicated structure.

### C) [3β(S),5β] -3-[[4-[Bis [2-[bis(carboxymethyl)amino] ethyl]amino]-4-carboxy-l-oxobutyl]amino]cholan-24-oic acid

A suspension of 6.14 g of the compound prepared at step B) (7.33 mmol) in 50 mL of H₂O is added with 50 mL of 1 M NaOH (50 mmol) keeping pH 13 by means of a pH-stat apparatus. After 2 h at room temperature the reaction mixture is acidified (pH 0.5) with 12 M HCl to give a suspension which is filtered, washed with H₂O and dried to afford the desired product (5.64 g; 6.85 mmol).
Yield: 93% m.p.: 205°C
K.F.: 9.02%

| Elemental anal. | C | H | N | Cl,Na |
|---|---|---|---|---|
| % calc.: | 59.84 | 8.08 | 6.81 | |
| % found:^{a} | 59.56 | 8.15 | 6.80 | <0.1 |

| | | | | |
|---|---|---|---|---|
| *^{a}* after drying at 120 °C under vacuum | | | | |

TLC: Stationary phase: silica gel plate 60F 254 Merck
Eluent: 6 : 4 : 1 CHCl₃/MeOH/25% NH₄OH
Detection: 0.5% KMnO₄ in 1 M NaOH R_{f}= 0.25
The ¹H-NMR, ¹³C-NMR, IR and MS spectra are consistent with the indicated structure.

### D) Gadolinium complex of [3ß(S),5ß]-3-[[4-[bis[2-[bis(carboxymethyl)amino]ethyl]amino]-4-carboxy-1-oxobutyl]amino]cholan-24-oic acid salified with 1-deoxy-1-(methylamino)-D-glucitol (1:3)

4.53 g of the compound prepared at step C) (5.5 mmol) are suspended in 50 mL of H₂O and solubilised with 10 mL of 2 M meglumine aqueous solution (20 mmol) obtaining a solution at pH 6.8. After that, 11 mL of 0.5 M GdCl₃ aqueous solution (5.5 mmol) are added in 1 h, keeping pH 6.8 by addition of 6.5 mL of 2 M meglumine aqueous solution (13 mmol). The progress of the reaction is monitored by capillary electrophoresis. After 2 h the solution is filtered through a Millipore® membrane, nanofiltered and evaporated. The residue is dried to give the desired compound (6.15 g; 4.17 mmol).
Yield: 76% m.p.: 220°C
K.F.: 8.44%
CE assay: 100% (area%)

| | | |
|---|---|---|
| Capillary: | fused silica 0.56 m x 50 mm with a bulb cell | |
| Voltage: | 25 kV | |
| Buffer: | 0.05 M borate pH 9.3, 0.3 mM EDTA | |
| Temperature: | 40°C | |
| Stop time: | 20 min | |
| Detection (UV): | 200-210 nm | |
| Injection: | hydrostatic (50 mbar, 5 s) | |
| Concentration sample: | 1 mg mL⁻¹; | |
| Instrumentation: | Hewlett PacKard 3D HPCE | |
| Preconditioning: | t (min) | action |
| | 2 | washing with H₂O |
| | 2 | washing with 0.1 M NaOH |
| | 1 | washing with H₂O |
| | 5 | washing with buffer |
| | 9 | start analysis |

| Elemental anal. | C | H | Gd | N | Cl,Na |
|---|---|---|---|---|---|
| % calc.: | 47.65 | 7.35 | 10.06 | 6.27 | |
| % found:^{a} | 47.83 | 7.36 | 10.01 | 6.24 | < 0.1 |

| | | | | | |
|---|---|---|---|---|---|
| *^{a}* after drying at 120 °C under vacuum | | | | | |

The IR and MS spectra are consistent with the indicated structure.

The following compounds and the related gadolinium complexes are prepared analogously:
Gadolinium complex of [3β(S), 5β,7β] -3- [ [4- [bis [2-[bis(carboxymethyl)amino]ethyl]amino]-4-carboxy-1-oxobutyl]amino]-7-hydroxycholan-24-oic acid salified with 1-deoxy-1-(methylamino)-D-glucitol (1:3);
Gadolinium complex of [3α(S),5β]-3-[2[-[5-[bis[2-[bis(carboxymethyl)amino]ethyl]amino]-5-carboxypentyl]-amino]-2-oxoethoxy]cholan-24-oic acid salified with 1-deoxy-1-(methylamino)-D-glucitol (1:3);

### EXAMPLE 2

### Gadolinium complex of [3β(S),5β]-3-[[4-[bis[2-[bis(carboxymethyl)amino]ethyl]amino]-4-carboxy-1-oxobutyl]amino]-12-oxocholan-24-oic acid salified with 1-deoxy-1-(methylamino)-D-glucitol (1:3)

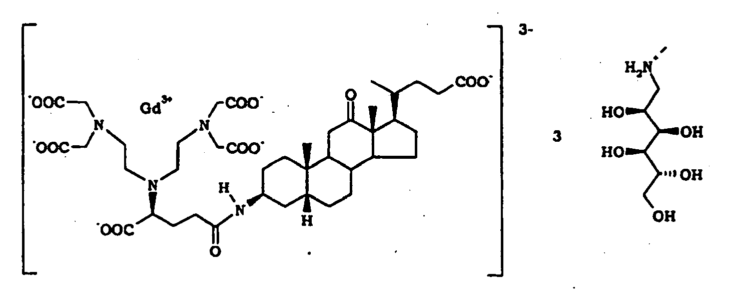

### A) (3β,5β)-3-Azido-12-oxocholan-24-oic acid methyl ester

12.5 mL of Jones reagent [33.3 mmol Cr(VI)] are dropped into a solution of 17.8 g of (3β,5β,12α)-3-azido-12-hydroxycholan-24-oic acid methyl ester (41.1 mmol) (prepared analogously to the method described for (3β,5β,7α,12α)-3-azido-7,12-dihydroxycholan-24-oic acid methyl ester in WO-A-95/32741: example 5) in acetone (600 mL) in 90 min at room temperature. After 20 h the mixture is filtered and the solution is evaporated. The residue is dissolved in CHCl₃ (400 mL) and the solution is washed with saturated aq. NaHCO₃ then with H₂O. The solution is dried and evaporated to give a crude which is crystallized from 96% EtOH to obtain 14.1 g of the desired product (32.9 mmol).
Yield: 84% m.p.: 153°C
K.F.: < 0.1%
[α]²⁰_{D} = + 83.25 (c 2.1, CHCl₃)

| Elemental analysis | C | H | N |
|---|---|---|---|
| % calc.: | 69.90 | 9.15 | 9.78 |
| % found: | 69.98 | 9.32 | 9.69 |

TLC: Stationary phase: silica gel plate 60F 254 Merck
Eluent: 8 : 2 *n*-hexane/EtOAc
Detection: 0.5% KMnO₄ in 1 M NaOH R_{f}= 0.43

The ¹H-NMR, ¹³C-NMR, IR and MS spectra are consistent with the indicated structure.

### B) (3β,5β)-3-Amino-12-oxocholan-24-oic acid methyl ester

A solution of 16.4 g of compound A) (38.2 mmol) in THF (130 mL) is hydrogenated in the presence of 5 % Pd/C (1.6 g) at room temperature and at 40 bar for 15 h in a Parr^{®} autoclave. The reaction mixture is filtered (paper and FH 0.5 µm Millipore® membrane) and evaporated. The crude is purified by flash-chromatography to afford 11.8 g of the desired product (29.2 mmol).
Yield: 77% m.p.: 129-130°C
K.F.: 1.04%
[α]²⁰D = + 87.8 (c 2.02, CHCl₃)

| Elemental analysis | C | H | N |
|---|---|---|---|
| % calc.: | 74.40 | 10.24 | 3.47 |
| % found: | 72.72 | 10.00 | 3.35 |

TLC: Stationary phase: silica gel plate 60F 254 Merck
Eluent: 95 : 5 MeOH/Et₃N
Detection: 0.5% KMnO₄ in 1 M NaOH R_{f}= 0.31

The ¹H-NMR, ¹³C-NMR, IR and MS spectra are consistent with the indicated structure.

### C) [3β(S),5β]-3-[[4-[Bis[2-[bis[2-(1,1-dimethyl ethoxy)-2-oxoethyl]amino]ethyl]amino]-5-(1,1-dimethylethoxy)-1,5-dioxopentyl]amino]-12-oxocholan-24-oic acid methyl ester

A solution of DCC (6.24 g; 30.3 mmol) in CH₂Cl₂ (25 mL) is dropped in 30 min into a solution of N,N-bis[2-[bis[2-(1,1-dimethylethoxy)-2-oxoethyl]amino]ethyl]-L-glutamic acid 1-(1,1-dimethylethyl) ester (prepared as described in WO-A-95/32741: example 15) (21.5 g; 28.9 mmol), compound B) (11.1 g; 27.5 mmol) and HOBT (1-hydroxybenzotriazole) (3.72 g; 27.5 mmol) in CH₂Cl₂ (300 mL) at 0 °C under nitrogen. The mixture is left to warm to room temperature. After 21 h the reaction mixture is filtered and the solution is washed with NaHCO₃ saturated aqueous solution, then H₂O, and subsequently evaporated. The crude is purified by flash-chromatography to afford 24.5 g of the desired product (21.7 mmol).
Yield: 79%
[α]²⁰D= + 12.17 (c 2.07, CHCl₃)
TLC: Stationary phase: silica gel plate 60F 254 Merck
Eluent: 1 : 1 EtOAc/n-hexane
Detection: 0.5% KMnO₄ in 1 M NaOH R_{f}= 0.45

The ¹H-NMR, ¹³C-NMR, IR and MS spectra are consistent with the indicated structure.

### D) [3β(S),5β]-3-[[4-[Bis[2-[bis(carboxymethyl)amino] ethyl]amino]-4-carboxy-1-oxobutyl]amino]-12-oxocholan-24-oic acid

80 mL of TFA (1.0 mol) are dropped into a solution of 23.8 g of the compound prepared at step C) (21.0 mmol) in CH₂Cl₂ (50 mL) at 0°C, in 1 h. The reaction mixture is stirred at room temperature then, after 2 h, evaporated. The residue is taken up with TFA (100 mL; 1.3 mol) and the solution stirred for further 24 h. The reaction mixture is then evaporated, taken up with CH₂Cl₂ and evaporated again. The crude is dissolved in 150 mL of 1 M NaOH, cooling with an ice-bath, and the solution is stirred for 15 h (pH 10) at room temperature. The reaction mixture is adjusted to pH 13 with 3.30 mL of 30% NaOH and, after 4 h, filtered through a Millipore® membrane (HAS 0.45 µm). The filtrate is acidified with 12.5 mL of 30% HCl and 19 mL of 1 M HCl to pH 1.60. The precipitate is filtered, washed with H₂O and dried to give 15.8 g of the desired product (18.9 mmol).
Yield: 90% m.p.: 172-175°C
K.F.: 1.98%
[α]²⁰D=+ 43.54 (c 2.02, 1 M NaOH)
HPLC: 97% (area%)
- Stationary phase:: Zorbax ECLIPSE XDB-C8 3.5 µm; 150 x 4.6 mm.;
- Temperature:: 40°C;
- Mobile phase:: gradient elution;
A = 0.017 M H₃PO₄, 0.3 mM EDTA in H₂O;
B = CH₃CN

| Gradient: | min | % A | % B |
|---|---|---|---|
| | 0 | 85 | 15 |
| | 40 | 65 | 35 |
| | 50 | 65 | 35 |

Flow rate: 1.5 mL min⁻¹;
Detection (UV): 210 nm;

| Elemental analysis | C | H | N | Cl | Na |
|---|---|---|---|---|---|
| % calc.: | 58.84 | 7.71 | 6.69 | | |
| % found: | 56.57 | 7.68 | 6.37 | 0.25 | 0.18 |

TLC: Stationary phase: silica gel plate 60F 254 Merck
Eluent: 5 : 4 : 2 CHCl₃/MeOH/25% NH₄OH
Detection: 0.5% KMnO₄ in 1 M NaOH R_{f}= 0.28

The ¹H-NMR, ¹³C-NMR, IR and MS spectra are consistent with the indicated structure.

### E) Gadolinium complex of [3β(S),5β] -3- [[4-[bis [2-[bis(carboxymethyl)amino]ethyl]amino]-4-carboxy-1-oxobutyl]amino]-12-oxocholan-24-oic acid salified with 1-deoxy-1-(methylamino)-D-glucitol (1:3)

49.0 mL of 0.918 M meglumine aqueous solution (45.0 mmol) are dropped into a suspension of 14.0 g of the compound prepared at step D) (16.7 mmol) in H₂O (100 mL), at room temperature, to give a clear solution (pH 6.5). 31.6 mL of 0.503 M GdCl₃ aqueous solution (15.9 mmol) are dropped therein keeping pH 6.5 by addition of 55.7 mL of 0.918 M meglumine aqueous solution (51.1 mmol) by means of a pH-stat. At the end of the additions the mixture is filtered through a Millipore^{®} membrane (HAWP 0.45 µm), nanofiltered, adjusted to pH 7.0 with 0.100 mL of 0.918 M meglumine aqueous solution (0.092 mmol) and evaporated. The residue is dried to give 22.0 g of the desired product (14.0 mmol).
Yield: 84% m.p.: 100-105 °C
K.F.: 5.06%
HPLC assay: 97% (area%)
- Stationary phase:: HYPURITY™ Elite C18 5 µm; 250 x 4.6 mm column of Hypersil;
- Temperature:: 40°C;
- Mobile phase:: gradient elution;
A = 0.01 M KH₂PO₄ in water;
B = CH₃CN

| Gradient: | min | % A | % B |
|---|---|---|---|
| | 0 | 95 | 5 |
| | 40 | 65 | 35 |
| | 50 | 65 | 35 |

Flow rate: 1 mL min⁻¹;
Detection (UV): 210 nm;

| Elemental analysis | C | H | N | Gd | Na,Cl |
|---|---|---|---|---|---|
| % calc.: | 47.23 | 7.16 | 6.22 | 9.97 | |
| % found: | 45.70 | 7.32 | 6.00 | 9.41 | < 0.1 |

The IR and MS spectra are consistent with the indicated structure.

### EXAMPLE 3

### Gadolinium complex of (3β,5β,7α,12α)-3-[[[bis[2-[bis(carboxymethyl)amino]ethyl]amino]acetyl]amino]-7,12-dihydroxycholan-24-oic acid salified with 1-deoxy-1-(methylamino)-D-glucitol (1:2)

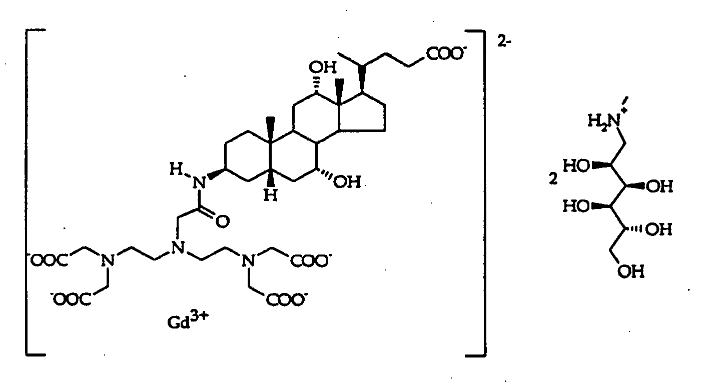

### A) (3β,5β,7α,12α)-3-[[[Bis[2-[bis[2-(1,1-dimethyl-ethoxy)-2-oxoethyl]amino]ethyl]amino]acetyl]amino]-7,12-dihydroxycholan-24-oic acid methyl ester

24.8 g of (3β,5β,7α,12α)-3-[(aminoacetyl)amino]-7,12-dihydroxycholan-24-oic acid methyl ester (prepared according to the procedure described in WO-A-95/32741: example 5) (51.9 mmol) are suspended in a stirred solution of 38.7 g of N-(2-bromoethyl)-N-[2-(1,1-dimethylethoxy)-2-oxoethyl]glycine 1,1-dimethylethyl ester (prepared according to the procedure described in WO-A-95/32741: example 15) (110 mmol) in 390 mL of CH₃CN. The addition of 245 mL of 2 M phosphate buffer pH 8 gives a biphasic solution which is vigorously stirred at room temperature for 144 h. The organic phase is separated and evaporated and the residual oil is dissolved in 250 mL of CH₂Cl₂. The solution is washed with H₂O, dried (Na₂SO₄) and evaporated. The crude is purified by flash-chromatography (eluent = 95 : 5 CHCl₃/CH₃OH) to give the desired product (24.8 g; 24.3 mmol).
Yield: 47%
[α]²⁰_{D}= + 9.45 (c 1.5, CHCl₃)

| Elemental analysis | C | H | N |
|---|---|---|---|
| % calc.: | 64.68 | 9.47 | 5.49 |
| % found: | 64.55 | 9.44 | 5.46 |

TLC: Stationary phase: silica gel plate 60F 254 Merck
Eluent: 88 : 12 CHCl₃/MeOH
Detection: 0.5% KMnO₄ in 1 M NaOH R_{f}= 0.57
The ¹H-NMR, ¹³C-NMR, IR and MS spectra are consistent with the indicated structure.

### B) (3β,5β,7α,12α)-3-[[[Bis[2-[bis(carboxymetliyl)amino]-ethyl]amino]acetyl]amino]-7,12-dihydroxycholan-24oic acid

318 mL of 2 M LiOH aqueous solution (636 mmol) are added dropwise, in 15 min, to a solution of the compound prepared at step A) (21.6 g; 21.1 mmol) in 310 mL of EtOH. After 23 h, EtOH is evaporated and the reaction mixture is stirred for further 2 h. The solution is dropped into 255 mL of 2.6 M HCl and the pH is adjusted to 1.4 with 30% NaOH. After 1.5 h, the precipitate is filtered, washed with 300 mL of 0.1 M HCl and dried to give the desired product (13.1 g; 16.7 mmol).
Yield: 78% m.p.: 129-132 °C dec
HPLC assay: 97.8% (area%)
- Stationary phase:: Lichrosorb RP-Select B 5 µm; 250 x 4 mm column Merck KGaA;
- Temperature:: 45°C;
- Mobile phase:: gradient elution;
A = 0.01 M KH₂PO₄ and 0.017 M H₃PO₄ in H₂O
B = CH₃CN

| min | % A | % B |
|---|---|---|
| 0 | 95 | 5 |
| 5 | 20 | 80 |
| 4.5 | 20 | 80 |

Flow rate: 1 mL min⁻¹;
Detection (UV): 210 nm;
Complexometric titer: 95.5% (0.1 M GdCl₃)
[α]²⁰_{D}= + 13.03 (c 5, 1 M NaOH)

| Elemental analysis | C | H | N | Cl | |
|---|---|---|---|---|---|
| % calc.: | 58.30 | 7.98 | 7.16 | | |
| % found: | 54.63 | 8.12 | 6.64 | 1.82 | H₂O 4.89 |

### C) Gadolinium complex of (3β,5β,7α,12α)-3-[[[bis[2-[bis(carboxymethyl)amino]ethyl]amino]acetyl]amino]-7,12-dihydroxycholan-24-oic acid salified with 1-deoxy-1-(methylamino)-D-glucitol (1:2)

11.3 g of the compound prepared at step B) (13.8 mmol) are suspended in 40 mL of H₂O and dissolved by addition of 44.7 mL of 1 M meglumine aqueous solution (44.7 mmol) up to pH 6. 13.7 mL of 1 M GdCl₃ aqueous solution (13.7 mmol) are dropped into the mixture in 1 h keeping pH 6.5 by addition of 73.5 mL of 1 M meglumine aqueous solution (73.5 mmol). The reaction mixture is nanofiltered and pH is adjusted to 6.8 with 0.3 mL of 0.1 M meglumine. After evaporation and drying the desired product is obtained (17.2 g; 12.9 mmol).
Yield: 93% m.p.: 245-249 °C dec.
Free ligand: < 0.1% (0.001 M GdCl₃)
HPLC assay: 100% (area%)
- Stationary phase:: Lichrospher 100 RP-8 5 µm; 250 x 4 mm column Merck KGaA;
- Temperature:: 40 °C;
- Mobile phase:: isocratic elution with premixed phase: 1 g of n-octylamine and 0.3 mmol of Na₂EDTA are added to 260 mL of CH₃CN and 740 mL H₂O. The solution is buffered to pH 7 with H₃PO₄.
- Flow rate:: 1 mL min⁻¹;
- Detection (UV):: 210 nm;

| Elemental analysis | C | H | Gd | N | |
|---|---|---|---|---|---|
| % calc.: | 47.05 | 7.06 | 11.84 | 6.33 | |
| % found: | 45.19 | 7.21 | 11.22 | 6.07 | H₂O 4.16 |

The IR and MS spectra are consistent with the indicated structure.

### EXAMPLE 4

### Gadolinium complex of [3β(S),5β,12α]-3-[[4-[bis(2-[bis(carboxymethyl)amino]ethyl]amino]-4-carboxy-1-oxobutyl]amino]-12-hydroxycholan-24-oic acid salified with 1-deoxy-1-(methylamino)-D-glucitol (1:3)

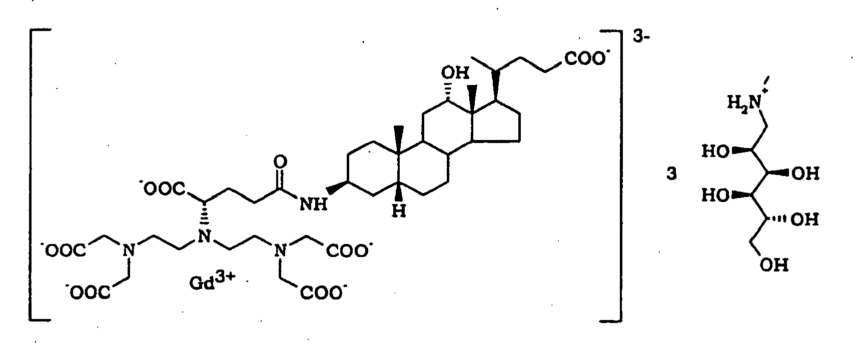

### A) [3β(S),5β,12α]-3-[[4-[Bis[2-[bis[2-(1,1-dimethyl-ethoxy)-2-oxoethyl]amino]ethyl]amino]-5-(1,1-dimethylethoxy)-1,5-dioxopentyl]amino]-12-hydroxy-cholan-24-oic acid methyl ester

Triethylamine (2.23 g; 22 mmol) is added to a solution of 8.93 g of (3β,5β,12α)-3-amino-12-hydroxycholan-24-oic acid methyl ester (prepared analogously to the cholic acid derivative described in WO-A-95/32741: example 5) (22 mmol), 16.41 g of N,N-bis[2-[bis[2-(1,1-dimethylethoxy)-2-oxoethyl]amino]-ethyl]-L-glutamic acid 1-(1,1-dimethylethyl) ester (prepared as described in WO-A-95/32741: example 15) (22 mmol) and 3.91 g of diethyl cyanophosphonate (24 mmol) in 300 mL of DMF at 0 °C. After 1.5 h at 0 °C and 18 h at room temperature, the reaction mixture is evaporated and the residue is dissolved in EtOAc. The solution is washed with NaHCO₃ saturated aqueous solution and H₂O, dried (Na₂SO₄) and evaporated. The crude is purified by flash-chromatography to give the desired product (20.67 g; 18.2 mmol).
Yield: 83%
[α]²⁰_{D}= - 6.75 (c 2.0, CHCl₃)

| Elemental analysis | C | H | N |
|---|---|---|---|
| % calc.: | 65.69 | 9.60 | 4.94 |
| % found: | 66.54 | 9.95 | 4.99 |

TLC: Stationary phase: silica gel plate 60F 254 Merck
Eluent: 1 : 1 n-hexane/EtOAc R_{f}= 0.09
Detection: Ce (SO₄) ₂·4H₂O (0.18%) and (NH₄)₆Mo₇O₂₄·4H₂O (3.83%) in 10% H₂SO₄
The ¹H-NMR, ¹³C-NMR, IR and MS spectra are consistent with the indicated structure.

### B) [3β(S),5β,12α]-3-[[4-[Bis[2-[bis(carboxymethyl) amino]ethyl]amino]-4-carboxy-1-oxobutyl]amino]-12-hydroxycholan-24-oic acid

The compound prepared at step A) (19.72 g; 17.4 mmol) is dissolved in 105 mL CF₃CO₂H at room temperature. After 26 h the solution is evaporated and the residue is treated with H₂O; the solid is filtered, washed with H₂O and partially dried under vacuum. The resulting intermediate is suspended in H₂O and dissolved with 1 M. NaOH up to pH 13. After 5 h at room temperature 0.5 M HCl is dropped into the solution up to pH 1.4. After 15 h at room temperature the precipitate is filtered, washed with H₂O and dried to give a crude which is purified by chromatography over resin Amberlite® XAD 1600 resin to obtain the desired product (9.92 g; 11.8 mmol).
Yield: 68% m.p.: 184 °C (dec.)
Complexometric titer (0.1 M GdCl₃): 99.3%
Acidic titer (0.1 N NaOH): 99.8%
[α]20λ (*c* 2.0; 1 M NaOH)

| | | | | | | | | | | |
|---|---|---|---|---|---|---|---|---|---|---|
| λ(nm) | 589 | | 578 | | 546 | | 436 | | 405 | 365 |
| [α] + | 23.61 | + | 24.59 | + | 27.90 | + | 46.67 | + | 55.61+ | 71.40 |

| Elemental analysis | C | H | N | |
|---|---|---|---|---|
| % calc.: | 58.70 | 7.93 | 6.68 | |
| % found: | 58.22 | 8.16 | 6.59 | H₂O 0.70% |

TLC: Stationary phase: silica gel plate 60F 254 Merck
Eluent: 5 : 4 : 2 CHCl₃/MeOH/25% NH₄OH R_{f}= 0.13
Detection: Ce (SO₄)₂·4H₂O (0.18%) and (NH₄) ₆Mo₇O₂₄·4H₂O (3.83%) in 10% H₂SO₄
The ¹H-NMR, ¹³C-NMR, IR and MS spectra are consistent with the indicated structure.

### C) Gadolinium complex of [3β(S),5β,12α]-3-[[4-[bis[2-[bis(carboxymethyl)amino]ethyl]amino]-4-carboxy-1-oxobutyl]amino]-12-hydroxycholan-24-oic acid salified with 1-deoxy-1-(methylamino)-D-glucitol (1:3)

The compound prepared at step B) (8.39 g; 10 mmol) is suspended in H₂O (30 mL) and dissolved by addition of 1 M aqueous meglumine (36.5 mL; 36.5 mmol) up to pH 6. A 1.025 M GdCl₃ aqueous solution (9.85 mL; 10.1 mmol) is added in 1 h keeping pH 6 by addition of 1 M aqueous meglumine (19.3 mL; 19.3 mmol). The solution is nanofiltered and the pH is adjusted to 7.0 with 1 M aq. meglumine. After evaporation and drying the desired product is obtained (8.57 g; 5.4 mmol).
Yield: 54% m.p.: 150-166 °C (170 °C dec.)

| Elemental analysis | C | H | N | Gd | |
|---|---|---|---|---|---|
| % calc.: | 47.17 | 7.28 | 6.21 | 9.96 | |
| % found: | 43.40 | 7.31 | 5.68 | 9.31 | H₂O 7.14% |

The IR and MS spectra are consistent with the indicated structure.

### D) Analogously to the compound prepared at step C), the gadolinium complex of [3β(S),5β,12α]-3-[[4-[bis[2-[bis(carboxymethyl)amino]ethyl]amino]-4-carboxy-1-oxobutyl]amino]-12-hydroxycholan-24-oic acid salified with sodium (1:3) is prepared

The compound prepared at step B) (26.92 g; 32.08 mmol) is suspended in H₂O (100 mL) and dissolved by addition of 2 M NaOH (56 mL; 112 mmol) to pH 6. A 0.512 M GdCl₃ aqueous solution (58.2 mL; 29.77 mmol) is added in 3 h keeping pH 6 by addition of 2 M NaOH (28.95 mL; 57.9 mmol). The pH of the solution is adjusted to 6.7 with 2 M NaOH (4 mL; 8 mmol) and the solution is nanofiltered. After freeze-drying, the desired product is obtained (29.86 g; 28.2 mmol).
Yield: 88% m.p.: > 300 °C

| Elemental anal. | C | H | N | Gd | Na | | |
|---|---|---|---|---|---|---|---|
| % calc.: | 46.49 | 5.71 | 5.29 | 14.85 | 6.51 | | |
| % found: | 43.98 | 6.34 | 4.92 | 13.86 | 6.16 | H₂O | 4.63% |

The IR and MS spectra are consistent with the indicated
structure.

### EXAMPLE 5

### Alternative method for the preparation of [3β(S),5β,12α]-3-([4-[bis[2-[bis(carboxymethyl)amino]ethyl]amino]-4-carboxy-1-oxobutyl]amino]-12-hydroxycholan-24-oic acid according to scheme 3

### A) (S)-5-Oxo-1,2-pyrrolidinedicarboxylic acid 2-methyl 1-(phenylmethyl) diester

7.1 g of CH₃I (50 mmol) are added to a solution of 6.58 g of (S)-5-oxo-1,2-pyrrolidinedicarboxylic acid 1-(phenylmethyl) ester (25 mmol) and N,N-diisopropylethylamine (3.55 g; 27.5 mmol) in CH₂Cl₂ (33 mL) and the reaction mixture is refluxed for 6.5 h. After cooling to room temperature and dilution with CH₂Cl₂ (50 mL) the reaction mixture is washed with H₂O, 2 % aq. Na₂CO₃, 0.2 M HCl and H₂O. After drying over Na₂SO₄ and evaporation, the desired product (6.8 g; 24.5 mmol) is obtained.
Yield: 98%
HPLC assay: 98.5% (area%)
- Stationary phase:: Lichrosorb RP-Select B 5 µm; 250 x 4 mm column Merck KGaA;
- Temperature:: 45°C;
- Mobile phase:: gradient elution;
A = 0.017 M H₃PO₄ in water
B = CH₃CN

| Gradient: | min | % A | % B |
|---|---|---|---|
| | 0 | 80 | 20 |
| | 15 | 80 | 20 |
| | 35 | 40 | 60 |

Flow rate: 1 mL min⁻¹;
Detection (UV): 210 nm.
[α]²⁰λ (c 2; CHCl₃)

| | | | | |
|---|---|---|---|---|
| λ (nm) | 589 | 578 | 546 | 365 |
| [α] | -42.97 | -44.79 | - 50.09 | -100.43 |

| Elemental analysis | C | H | N |
|---|---|---|---|
| % calc.: | 60.64 | 5.45 | 5.05 |
| % found: | 60.94 | 5.54 | 5.00 |

The ¹H-NMR, ¹³C-NMR, IR and MS spectra are consistent with the indicated structure.

### B) [3β(S),5β,12α]-3-[[5-Methoxy-1,5-dioxo-4-[[(phenyl-methoxy)carbonyl]amino]pentyl]amino]-12-hydroxycho-lan-24-oic acid methyl ester

8.92 g of (3β,5β,12α)-3-amino-12-hydroxycholan-24-oic acid methyl ester (prepared analogously to the cholic acid derivative described in WO-A-95/32741: example 5) (22 mmol) are added to a solution of compound A) (6.1 g; 22 mmol) in dioxane (55 mL) and the resulting mixture is heated to 50 °C for 24 h, then to 105 °C for 29 h. The solvent is evaporated under reduced pressure and the residue is purified by-flash-chromatography (gradient elution with EtOAc/n-hexane) followed by crystallization with a 1 : 1 EtOAc/n-hexane mixture, to give the desired product (11.2 g; 16.4 mmol).
- Yield: 75%: m.p.: 140°C
- HPLC assay: 99.2%: (area%)
- Stationary phase:: Lichrosorb RP-Select B 5 µm; 250 x 4 mm column Merck KGaA;
- Temperature:: 45°C;
- Mobile phase:: gradient elution;
A = 0.017 M H₃PO₄ in water
B = CH₃CN

| Gradient: | min | % A | % B |
|---|---|---|---|
| | 0 | 65 | 35 |
| | 25 | 15 | 85 |
| | 30 | 15 | 85 |

Flow rate: 1 mL min⁻¹;
Detection(UV): 210 nm.
[α]²⁰λ(c 2.01; CHCl₃)

| | | | | | | | |
|---|---|---|---|---|---|---|---|
| λ(nm) | 589 | | 578 | | 546 | | 365 |
| [α] | + 24.14 | + | 25.13 | + | 28.51 | + | 73.81 |

| Elemental analysis | C | H | N |
|---|---|---|---|
| % calc.: | 68.70 | 8.43 | 4.11 |
| % found: | 69.36 | 8.72 | 4.13 |

TLC: Stationary phase: silica gel plate 60F 254 Merck
Eluent: EtOAc
Detection: Ce (SO₄) ₂·4H₂O (0.2 %) and (NH₄) ₆Mo₇O₂₄·4H₂O (3.8%) in 10 % H₂SO₄ Rf = 0.11
The ¹H-NMR, ¹³C-NMR, IR and MS spectra are consistent with the indicated structure.

### C) [3β(S),5β,12α]-3-[[4-[Bis[2-[bis[2-(1,1-dimethyl-ethoxy)-2-oxoethyl]amino]ethyl]amino]-5-methoxy-1,5-dioxopentyl]amino]-12-hydroxycholan-24-oic acid methyl ester

1 g of 5 % Pd/C is added to a solution of compound B) (10.4 g; 15.3 mmol) in MeOH (100 mL); the suspension is stirred for 3.5 h under hydrogen atmosphere (absorbed H₂: 348 mL; 15.5 mmol) at room temperature. After filtration over a Millipore^{®} FH filter (0.45 µm), the solution is evaporated under reduced pressure to give a residue which is dissolved in CH₃CN (60 mL). 2 M Aqueous pH 8 phosphate buffer (60 mL) is added, then a solution of N-(2-bromoethyl)-N-[2-(1,1-dimethylethoxy)-2-oxoethyl]glycine 1,1-dimethylethyl ester (prepared according to the procedure described in WO-A-95/32741: example 15) (11.86 g; 33.7 mmol) in CH₃CN (15 mL) is added dropwise in 10 min at room temperature. The mixture is stirred for 39 h. After separation, the organic phase is evaporated under reduced pressure and the residue is dissolved in AcOEt (200 mL). The solution is washed with H₂O, dried (Na₂SO₄) and evaporated. The crude is purified by flash-chromatography (gradient elution with EtOAc/n-hexane) to give the desired product (11.36 g; 10.4 mmol).
Yield: 68% m.p.: 55-58°C
HPLC assay: 100% (area%)
[α]²⁰λ (c 2.01; CHCl₃)

| | | | | |
|---|---|---|---|---|
| λ (nm) | 589 | 578 | 546 | 365 |
| [α] | - 6.97 | - 7.41 | - 8.61 | - 32.89 |

| Elemental analysis | C | H | N |
|---|---|---|---|
| % calc.: | 64.93 | 9.42 | 5.13 |
| % found: | 65.06 | 9.36 | 5.11 |

TLC: Stationary phase: silica gel plate 60F 254 Merck
Eluent: EtOAc
Detection: Ce (SO₄)₂·4H₂O (0.2 %) and (NH₄)₆MO₇O₂₄·4H₂O (3.8%) in 10 % H₂SO₄ Rf= 0.45
The ¹H-NMR, ¹³C-NMR, IR and MS spectra are consistent with the indicated structure.

### D) [3β(S),5β,12α]-3-[4-[Bis[2-[bis(carboxymethyl)amino]-ethyl]amino]-4-carboxy-1-oxobutyl]amino]-12-hydroxy-cholan-24-oic acid

A solution of compound C) (8.5 g; 7.8 mmol) in dioxane (50 mL) is added with 2 M LiOH aqueous solution (117 mL; 234 mmol). The resulting mixture is stirred at room temperature for 72 h, then acidified to pH 6 by slow addition of 37% HCl. The solution is concentrated to 50 g by evaporation under reduced pressure and diluted with H₂O (40 mL). The solution is acidified to pH 2.5 by addition of 37 % HCl, heated to 50-55 °C and, under strong stirring, acidified very slowly to pH 1.3 with 2 N HCl. After 5 min the heterogeneous mixture is left to slowly cool to room temperature, under stirring, for 15 h. The precipitate is filtered, washed with H₂O and dried to give the desired product (5.92 g; 7 mmol).
Yield: 90% m.p.: 180-198°C
HPLC assay: 99.9% (area%)
- Stationary phase:: Zorbax ECLIPSE XDB-C8 3.5 µm; 150 x 4.6 mm column Rockland Technologies, Inc.;
- Temperature:: 40°C;
- Mobile phase:: gradient elution;
A = 0.017 M H₃PO₄, 0.3 mM EDTA in water;
B = CH₃CN

| Gradient: | min | % A | % B |
|---|---|---|---|
| | 0 | 85 | 15 |
| | 40 | 65 | 35 |
| | 50 | 65 | 35 |

Flow rate: 1.5 mL min⁻¹;
Detection (UV): 210 nm;
Acidic titer (0.1 N NaOH): 99%
[α]²⁰λ (c 2.04; 1 N NaOH)

| | | | | | | |
|---|---|---|---|---|---|---|
| λ (nm) | 589 | 578 | 546 | 436 | 405 | 365 |
| [a] | +24.80 | +25.83 | +29.22 | +49.02 | +58.43 | +75.59 |

| Elemental anal. | C | H | N | Cl,Li | |
|---|---|---|---|---|---|
| % calc.: | 58,70 | 7,93 | 6,68 | | |
| % found | 57,90 | 7,97 | 6,57 | <0.1 | H₂O 0.95 |

The ¹H-NMR, ¹³C-NMR, IR and MS spectra are consistent with the indicated structure.

### EXAMPLE 6

### Gadolinium complex of (3β,5β,7α,12α)-3-[[[[[bis[2-[bis(carboxymethyl)amino]ethyl]amino]acetyl]amino]acetyl] amino]-7,12-dihydroxycholan-24-oic acid salified with 1-deoxy-1-(methylamino)-D-glucitol (1:2)

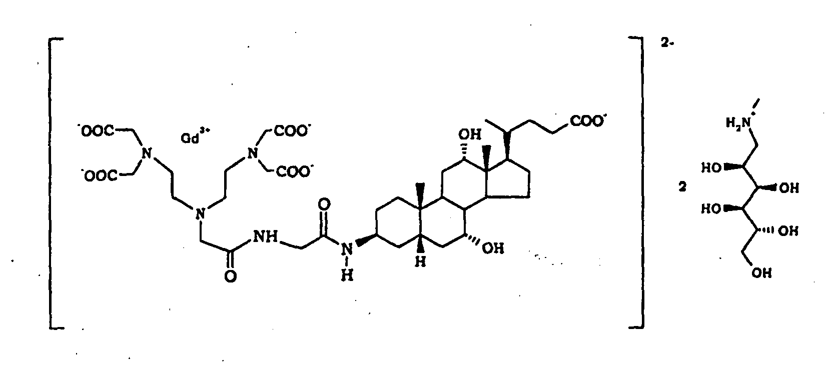

### A) N-[[Bis[2-[bis[2-(1,1-dimethylethoxy-2-oxoethyl] amino]ethyl]amino]acetyl]glycine

6.5 g of glycylglycine (49.3 mmol) are suspended in 100 mL of a 1 : 1 H₂O/EtOH mixture and dissolved at pH 10 with 10 M NaOH (4.8 mL). N-(2-Bromoethyl)-N[2-(1,1-dimethylethoxy)-2-oxoethyl]glycine 1,1-dimethylethyl ester (42 g; 110.9 mmol) in 40 mL of EtOH is dropped therein in 2 h, keeping pH 10.5 with 10 M NaOH (5.8 mL). The solution rapidly turns into an emulsion, which is dissolved after 2.5 h by addition of 10 M NaOH. After 22 h the solvent is evaporated, the mixture is diluted with water and extracted with CH₂Cl₂. The organic phase is washed with H₂O, dried and evaporated, to give a residue which is purified by flash-chromatography. The residue is dissolved in water, the pH is adjusted to 4.5 by addition of 1 M HCl and the solution is extracted with chloroform. The organic phase is washed with H₂O, dried and evaporated, to give 13 g of the desired product (19.3 mmol).
Yield: 39%

| Elemental analysis | C | H | N |
|---|---|---|---|
| %.calc.: | 56.95 | 8.66 | 8.30 |
| % found: | 56.67 | 8.68 | 8.30 |

TLC: Stationary phase: silica gel plate 60F 254 Merck
Eluent: 6 : 3 : 1 CHCl₃/MeOH/25% NH₄OH
R_{f}= 0.65
Detection: 1% KMnO₄ in 1 M NaOH
The ¹H-NMR, ¹³C-NMR,IR and MS spectra are consistent with the indicated structure.

### B) (3β,5β,7α,12α)-3-[([[[Bis[2-[bis[2-(1,1-dimethyl-ethoxy)-2-oxoethyl]amino]ethyl]amino]acetyl]amino] acetyl]amino]-7,12-dihydroxycholan-24-oic acid methyl ester

2.8 mL of TEA (20.2 mmol) are added dropwise in 5 min into a solution containing 13.6 g of the compound prepared at step A) (20.2 mmol), 8.52 g of (3β,5β,7α,12α)-3-amino-7,12-dihydroxycholan-24-oic acid methyl ester (20.2 mmol) and DEPC (3.4 mL; 22.2 mmol) in DMF (290 mL) stirred at 0 °C. After 1 h the reaction is warmed to room temperature and the solution is stirred for 6.5 h. 0.3 mL of DEPC (2 mmol) are added and the solution is stirred for further 15.5 h. DMF is evaporated, the residue is dissolved in EtOAc, washed with aq. NaHCO₃ then with water and finally dried. After purification by flash-chromatography, 13.7 g of the desired product are obtained (12.7 mmol).
Yield: 63%
[α]²⁰D = +5.26 (c 1.5; CHCl₃)

| Elemental analysis | C | H | N |
|---|---|---|---|
| % calc.: | 63.48 | 9.25 | 6.49 |
| % found: | 63.22 | 9.40 | 6.40 |

The ¹H-NMR, ¹³C-NMR, IR and MS spectra are consistent with the indicated structure.

### C) (3β,5β,7α,12α)-3-[([[[Bis[2-[bis(carboxymethyl) amino]ethyl]amino]acetyl]amino]acetyl]amino]-7,12-dihydroxycholan-24-oic acid

12.85 g of the compound prepared at step B) (12 mmol) are dissolved in TFA (210 mL) stirring at -5/0 °C. After 16 h, TFA is evaporated to give a residue which is dissolved in 90 mL of 0.8 M NaOH at pH 13 and stirred at room temperature for 15 h. The solution is concentrated to 50 mL, dropped into 105 mL of 0.6 M HCl and stirred for 2 h. The solid is filtered, washed with 0.1 M HCl and dried to obtain a crude which is purified by chromatography. The fractions containing the desired compound in the salified form are evaporated to give a residue which is dissolved in water and dropped into 1 M HCl, maintaining pH 1.45. The precipitate is filtered, washed with 0.1 M HCl and dried to give 2.6 g of the desired product (3.1 mmol).
Yield: 26% m.p.: 120-125 °C
HPLC assay: 98% (area%)
The ¹H-NMR, ¹³C-NMR, IR and MS spectra are consistent with the indicated structure.

### D) Gadolinium complex of (3β,5β,7α,12α)-3-[[[[[bis[2-[bis(carboxyroethyl)amino]ethyl]amino]acetyl]amino]ac etyl]amino]-7,12-di-hydroxycholan-24-oic acid salified with 1-deoxy-1-(methylamino)-D-glucitol (1:2)

2.59 g of the compound prepared at step C) (3.08 mmol) are suspended in water (20 mL) and dissolved by addition of 1 M meglumine aqueous solution (3.08 mL; 3.08 mmol) to pH 5. Gd₂O₃ (0.501 g; 2.77 mmol) is added to the mixture heating to 50 °C. After 1 h, 1 M meglumine (2.8 mL; 2.8 mmol) is added to dissolve the precipitate. After 24 h the reaction mixture is filtered and the pH is adjusted to 6.8 with 1 M aqueous meglumine (0.4 mL). After evaporation and drying, 4.2 g (3.00 mmol) of the desired product are obtained.
Yield: 99% m.p.: 209-213 °C dec.
HPLC assay: 99.7% (area%)
Free ligand: < 0.1% (0.001 GdCl₃)

| Elemental anal. | C | H | N | Gd | |
|---|---|---|---|---|---|
| % calc.: | 46.84 | 6.99 | 7.08 | 11.36 | |
| % found: | 44.01 | 7.35 | 6.68 | 10.39 | H₂O 4.95 |

The IR and MS spectra are consistent with the indicated structure.

### EXAMPLE 7

### Gadolinium complex of [3β(S),5β] -3- [[4- [bis [2- [bis-(carboxymethyl)amino]ethyl]amino]-4-carboxy-1-oxobutyl] (carboxymethyl)amino]cholan-24-oic acid salified with 1-deoxy-1-(methylamino)-D-glucitol (1:4)

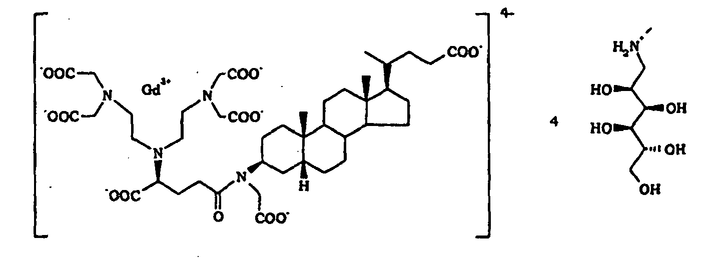

### A) (3β,5β)-3-[[2-(1,1-Dimethylethoxy)-2-oxoethyl]amino]-cholan-24-oic acid methyl ester

40.0 g of (3β,5β)-3-Aminocholan-24-oic acid methyl ester (prepared as in Example 1 above) (103 mmol) are suspended in DMF (1.0 L) at room temperature under nitrogen. Triethylamine (13.0 g; 129 mmol) is added, then a solution of bromoacetic acid 1,1-dimethylethyl ester (24.0 g; 123 mmol) in DMF (30 mL) is dropped into the reaction mixture in 1 h until dissolution. After 3 days the mixture is concentrated and diluted with 4 % NaHCO₃ aqueous solution. The resulting suspension is filtered, the precipitate is washed with H₂O and dried to give 33.7 g of the desired product (66.9 mmol).
Yield: 65 % m.p.: 62-64 °C
[α]²⁰D = + 23.55 (c 1.96, MeOH)

| Elemental analysis | C | H | N | |
|---|---|---|---|---|
| % calc.: | 73.91 | 10.60 | 2.78 | |
| % found: | 74.67 | 10.85 | 2.78 | H₂O < 0.1 |

TLC: Stationary phase: silica gel plate 60F 254 Merck
Eluent: 7 : 3 n-hexane/EtOAc R_{f}= 0.31
The ¹H-NMR, ¹³C-NMR, IR and MS spectra are consistent with the indicated structure.

### B) [3β(S),5β]-3-[[4-[Bis[2-[bis[2-(1,1-dimethylethoxy)-2-oxoethyl]amino]ethyl]amino]-5-(1,1-dimethyletho-xy)-1,5-dioxopentyl][2-(1,1-dimethylethoxy)-2-oxoethyl]amino]cholan-24-oic acid methyl ester

Diisopropylethylamine (19.5 g; 151 mmol) is added dropwise in 20 min into a solution of compound A) (33.0 g; 65.5 mmol) , N,N-bis[2-[bis[2-(1,1-dimethylethoxy)-2-oxoethyl]amino]ethyl]-L-glutamic acid 1-(1,1-dimethylethyl) ester (prepared as described in WO-A-95/32741: example 15) (53.8 g; 72.1 mmol) and (benzotriazol-1-yloxy)tris(dimethylanamino)phosphonium hexafluorophosphate (BOP) (40.6 g; 91.8 mmol) in DMF (400 mL), stirred at room temperature under nitrogen. After 2 days the reaction mixture is concentrated and taken up with EtOAc. The solution is washed with H₂O, dried (Na₂SO₄) and evaporated. The crude is purified twice by flash-chromatography to give 38.7 g of the desired product (31.4 mmol).
Yield: 48 %.
[α]²⁰D= - 54.50 (c 2.51, CHCl₃)
TLC: Stationary phase: silica gel plate 60F 254 Merck
Eluent: 7 : 3 n-hexane/EtOAc R_{f}= 0.22
The ¹H-NMR, ¹³C-NMR, IR and MS spectra are consistent with the indicated structure.

### C) [3β(S),5β]-3-[[4-[Bis[2-[bis(carboxymethyl)amino] ethyl]amino]-4-carboxy-1-oxobutyl](carboxymethyl) amino]cholan-24-oic acid

Into a solution of compound B) (38.7 g; 31.4 mmol) in EtOH (350 mL), stirred at room temperature, 500 mL of 2 M LiOH solution are added dropwise in 1 h. After 16 h the reaction mixture is concentrated to 500 mL and 2 M LiOH solution (350 mL) is added again, heating to 50°C. After 24 h the reaction mixture is cooled to room temperature and dropped into 320 mL of 6 M HCl vigorously stirred at 5 °C. The pH of the resulting suspension is adjusted to 1.0 with 55 mL of 2 M NaOH. The solid is filtered, washed with 0.1 M HCl and dried. The crude is suspended in H₂O and solubilised by addition of 1 M NaOH, then the basic solution is dropped into 0.5 M HCl solution. The precipitate is filtered, washed with 0.05 M HCl, H₂O and dried to give 23.5 g of the desired product (26.7 mmol).
Yield: 85 % m.p. : 178-182 °C
[α]⁴⁰⁵₂₀= + 24.10 (c 1.49, 1 M NaOH)
HPLC assay : 100% (area%)
- Stationary phase:: Hypurity Elite C-18 5 µm; column 250 x 4.6 mm;
- Temperature:: 40°C;
- Mobile phase:: gradient elution;
A = 0.01 M KH₂PO₄, 0.3 mM EDTA in water
B = CH₃CN

| Gradient: | min | % A | % B |
|---|---|---|---|
| | 0 | 95 | 5 |
| | 40 | 65 | 35 |
| | 50 | 65 | 35 |

Flow rate: 1 mL min⁻¹;
Detection (UV): 210 nm.

| Elemental anal. | C | H | N | Na | Cl | |
|---|---|---|---|---|---|---|
| % calc.: | 58.62 | 7.78 | 6.36 | | | |
| % found: | 57.71 | 8.07 | 6.20 | 0.13 | 0.55 | H₂O < 0.1 |

The ¹H-NMR, ¹³C-NMR, IR and MS spectra are consistent with the indicated structure.

### D) Gadolinium complex of [3β(S),5β] -3- [[4-[bis [2-[bis(carboxymethyl)amino]ethyl]amino]-4-carboxy-1-oxobutyl](carboxymethyl)amino]cholan-24-oic acid salified with 1-deoxy-1-(methylamino)-D-glucitol (1:4)

A suspension of the compound prepared at step C) (12.9 g; 14.6 mmol) in H₂O (100 mL) is added with 1 M meglumine aqueous solution (72.0 mL; 72.0 mmol) at room temperature to obtain a clear solution having pH 6.2. A 0.393 M GdCl₃ aqueous solution (37.2 mL; 14.6 mmol) is added dropwise, keeping pH 6.2 by addition of 1 M meglumine (30.0 mL; 30.0 mmol) by means of a pH-stat. At the end of the additions the reaction mixture is filtered through paper and then through Millipore® membrane (HAWP 0.45 µm), nanofiltered, adjusted to pH 7.0 by addition of 1 M meglumine (0.20 mL; 0.20 mmol) and evaporated. The solid is dried to afford 24.0 g of the desired product (13.2 mmol).
Yield: 91 % m.p.: 90-92 °C
HPLC assay : 100% (area%)
- Stationary phase:: Lichrospher 100 RP-8 5 µm; column Merck KGAa 250 x 4 mm;
- Temperature:: 40 °C;
- Mobile phase:: isocratic elution with premixed mobile phase: 1 g of n-octylamine is added to 400 mL of acetonitrile and 600 mL of water. The solution is buffered to pH 6 with H₃PO₄;
- Flow rate:: 1 mL min⁻¹;
- Detection (UV) :: 210 nm.

| Elemental anal. | C | H | N | Gd | Na,Cl | |
|---|---|---|---|---|---|---|
| % calc.: | 46.96 | 7.38 | 6.17 | 8.66 | | |
| % found: | 44.27 | 7.59 | 5.76 | 8.21 | <0.1 | H₂O 6.61 |

The IR and MS spectra are consistent with the indicated structure.

### EXAMPLE 8

### Gadolinium complex of [3β(R),5β,12α]-3-[[4-[bis[2-[bis(carboxymethyl)amino]ethyl]amino]-4-carboxy-1-oxobutyl]amino]-12-hydroxycholan-24-oic acid salified with sodium (1:3)

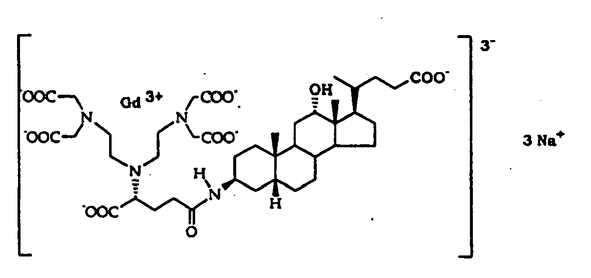

Following reaction scheme 3 and the experimental procedure given in example 5, (R)-5-oxo-1,2-pyrrolidinedicarboxylic acid 1-(phenylmethyl) ester (product commercially available) was esterified with methyl iodide in the presence of N,N-diisopropylethylamine and the (R) methyl ester thus obtained was reacted with (3β,5β,12α)-3-amino-12-hydroxycholan-24-oic acid methyl ester (prepared analogously to the cholic acid derivative described in WO-A-95/32741: example 5) to afford [3β(R),5β,12α]-3-[[4-[bis[2-[bis[2-(1,1-dimethylethoxy)-2-oxoethyl]amino]-ethyl]amino]-5-methoxy-1,5-dioxopentyl]amino]-12-hydroxy-cholan-24-oic acid methyl ester. After removal (H₂/Pd) of the Cbz protecting group and alkylation with N-(2-bromoethyl)-N-[2-(1,1-dimethylethoxy)-2-oxoethyl]-glycine 1,1-dimethylethyl ester (prepared according to the procedure described in WO-A-95/32741: example 15) in CH₃CN/phosphate buffer pH 8, an hexaester was obtained which was transformed (aq. LiOH/dioxane) into the related hexaacid. The latter was complexed, according to the procedure reported in example 4, point D), to obtain the desired product with 44% overall yield.
m.p.: > 300 °C

| Elemental anal. | C | H | N | Gd | Na | |
|---|---|---|---|---|---|---|
| % calc.: | 46.49 | 5.71 | 5.29 | 14.85 | 6.51 | |
| % found: | 44.02 | 6.13 | 5.10 | 14.09 | 6.17 | H₂O 4.50% |

The IR and MS spectra are consistent with the indicated structure.

### EXAMPLE 9

### Gadolinium complex of [3β(RS),5β,12α]-3-[[4-[bis[2-[bis(carboxymethyl)amino]ethyl]amino]-4-carboxy-1-oxobutyl]amino]-12-hydroxycholan-24-oic acid salified with sodium (1:3)

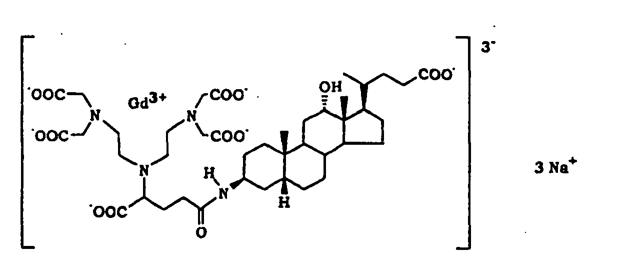

The compound was synthetized from (3β,5β,12α)-3-amino-12-hydroxycholan-24-oic acid methyl ester (prepared analogously to the cholic acid derivative described in WO-A-95/32741: example 5) and N,N-bis[2-[bis[2-(1,1-dimethylethoxy)-2-oxoethyl]amino]-ethyl]-DL-glutamic acid 1-(1,1-dimethylethyl) ester (prepared, from DL glutamic acid, as described in WO-A-95/32741: example 15 for the L isomer) according to the procedure given in detail in example 4. The product was obtained with 61% overall yield.
m.p.: > 300°C
HPLC assay: 99% (area%)
- Stationary phase:: Zorbax ECLIPSE XDB-C8 3.5 µm; 150 x 4.6 mm column Rockland Technologies, Inc.;
- Temperature:: 40°C;
- Mobile phase:: gradient elution;
A = 0.005 M KH₂PO₄, 0.005 M K₂HPO₄, 0.3
mM EDTA in water;
B = CH₃CN

| Gradient: | min | % A | % B |
|---|---|---|---|
| | 0 | 90 | 10 |
| | 5. | 90 | 10 |
| | 20 | 50 | 50 |

Flow rate: 1.0 mL min⁻¹;
Detection (UV): 210 nm;

The chromatographic method shows two peaks, in almost equal percentage, that are related to diastereoisomers due to the RS stereocenter in the DTPA residue.

| Elemental anal. | C | H | N | Gd | Na | |
|---|---|---|---|---|---|---|
| % calc.: | 46.49 | 5.71 | 5.29 | 14.85 | 6.51 | |
| % found: | 43.98 | 6.34 | 4.98 | 13.86 | 6.16 | H₂O 4.63% |

The IR and MS spectra are consistent with the indicated structure.

### EXAMPLE 10

### Gadolinium complex of [3α(S),5β,12α]-3-[[4-[bis[2-[bis(carboxymethyl)amino]ethyl]amino]-4-carboxy-1-oxobutyl]amino]-12-hydroxycholan-24-oic acid salified with sodium (1:3)

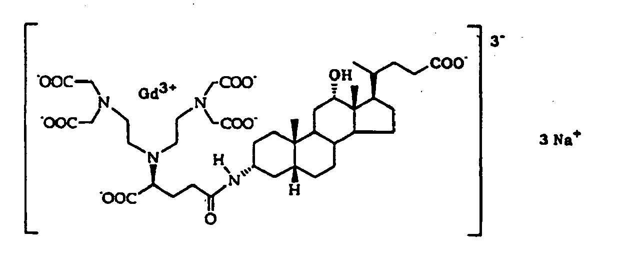

### 3α,5β,12α)-3-Amino-12-hydroxycholan-24-oic acid methyl ester

(3α,5β,12α)-3,12-Dihydroxycholan-24-oic acid methyl ester was reacted under Mitsunobu conditions (Mitsunobu, O. Synthesis 1981, 1-28; Denike, J. K. et al. chem. Phys. Lipids 1995, 77, 261-267) with triphenylphosphine, diethyl azodicarboxylate and formic acid in THF to afford (3β,5β,12α)-3-formyloxy-12-hydroxycholan-24-oic acid methyl ester. The latter was deprotected (MeOH/HCl) to (3β,5β,12α)-3,12-dihydroxycholan-24-oic acid methyl ester which was subjected to the series of reactions described in WO-A-95/32741: example 5 for the cholic acid derivative. (3α,5β,12α)-3-Amino-12-hydroxycholan-24-oic acid methyl ester was obtained with 32% overall yield.
m.p.: 90-92 °C
[α]²⁰D= + 53.46 (c 1.3, CH₃OH)

| Elemental analysis | C | H | N |
|---|---|---|---|
| % calc.: | 74.03 | 10.69 | 3.44 |
| % found: | 74.20 | 10.99 | 3.26 |

TLC: Stationary phase: silica gel plate 60F 254 Merck
Eluent: 9 : 1 : 0.15 CHCl₃/CH₃OH/25% NH₄OH R_{f}= 0.21
The ¹H-NMR, ¹³C-NMR, IR and MS spectra are consistent with the indicated structure.

Following reaction scheme 3 and the experimental procedure given in example 5, (S)-5-oxo-1,2-pyrrolidinedicarboxylic acid 2-methyl 1-(phenylmethyl) diester was reacted with compound A). The 3α(S),5β,12α] - 3-[[4-[bis[2-[bis[2-(1,1-dimethylethoxy)-2-oxoethyl]-amino]ethyl]amino]-5-methoxy-1,5-dioxopentyl]amino]-12-hy-droxycholan-24-oic acid methyl ester thus obtained was hydrogenated (H₂/Pd) to remove the Cbz protecting group.

The subsequent alkylation with N-(2-bromoethyl)-N-[2-(1,1-dimethylethoxy)-2-oxoethyl]glycine 1,1-dimethylethyl ester (prepared according to the procedure described in WO-A-95/32741: example 15) in CH₃CN/phosphate buffer pH 8, afforded the related hexaester. The latter was transformed (aq. LiOH/dioxane) into the hexaacid which was complexed, according to the procedure reported in example 4, point D), to obtain the desired product with 33% overall yield.
m.p.: > 300 °C
HPLC assay: 100.0% (area%)

| Elemental anal. | C | H | N | Gd | Na | |
|---|---|---|---|---|---|---|
| % calc.: | 46.49 | 5.71 | 5.29 | 14.85 | 6.51 | |
| % found: | 42.04 | 6.37 | 4.76 | 13.28 | 5.91 | H₂O 9.79% |

The IR and MS spectra are consistent with the indicated structure.

### EXAMPLE 11

### Gadolinium complex of [3β(S),5β,7α]-3-[[4-[bis[2-[bis(carboxymethyl)amino]ethyl]amino]-4-carboxy-1-oxobutyl]amino)-7-hydroxycholan-24-oic acid salified with sodium (1:3)

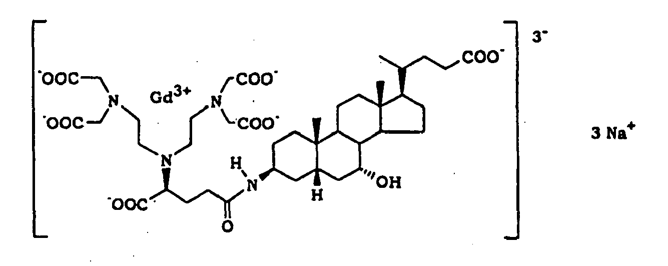

The compound was synthetized from (3β,5β,7α)-3-amino-7-hydroxycholan-24-oic acid methyl ester (prepared analogously to the cholic acid derivative described in WO-A-95/32741: example 5) and N,N-bis[2-[bis[2-(1,1-dimethylethoxy)-2-oxoethyl]amino]-ethyl]-L-glutamic acid 1-(1,1-dimethylethyl) ester (prepared as described in WO-A-95/32741: example 15) according to the procedure given in detail in example 4. The product was obtained with 89% overall yield.
m.p.: > 300 °C

| Elemental anal. | C | H | N | Gd | Na | |
|---|---|---|---|---|---|---|
| % calc.: | 46.49 | 5.71 | 5.29 | 14.85 | 6.51 | |
| % found: | 43.88 | 6.50 | 4.91 | 13.62 | 6.04 | H₂O 7.11% |

The IR and MS spectra are consistent with the indicated structure.

### EXAMPLE 12

### [3β(RS),5β,7α,12α]-3-[[4-(Bis[2-[bis(carboxymethyl) amino]ethyl]amino]-4-carboxy-1-oxobutyl]amino]-7,12-dihy-droxycholan-24-oic acid

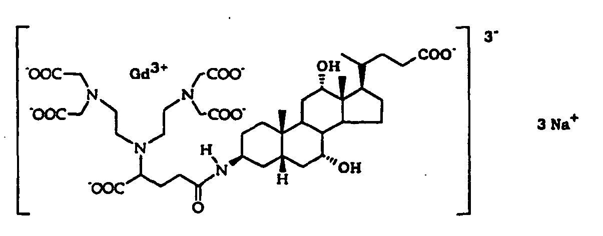

The compound was synthetized from (3β,5β,7α,12α)-3-amino-7,12-dihydroxycholan-24-oic acid methyl ester (prepared as described in WO-A-95/32741: example 5) and N,N-bis[2-[bis[2-(1,1-dimethylethoxy)-2-oxoethyl]amino]-ethyl]-DL-glutamic acid 1-(1,1-dimethylethyl) ester (prepared, from DL glutamic acid, as described in WO-A-95/32741: example 15 for the L isomer) according to the procedure given in detail in example 4 for the preparation of compound B). The product was obtained with 65% overall yield.
m.p.: 224 °C dec.
HPLC assay: 97% (area%)
- Stationary phase:: Zorbax ECLIPSE XDB-C8 3.5 µm; 150 x 4.6 mm column Rockland Technologies, Inc.;
- Temperature:: 40°C;
- Mobile phase:: gradient elution;
A = 0.017 M H₃PO₄, 0.3 mM EDTA in water;
B = CH₃CN

| Gradient: | min | % A | % B |
|---|---|---|---|
| | 0 | 95 | 5 |
| | 40 | 65 | 35 |
| | 50 | 65 | 35 |

Flow rate: 1.0 mL min⁻¹;
Detection (UV): 210 nm;

The chromatographic method shows two peaks, in almost equal percentage, that are related to diastereoisomers due to the RS stereocenter in the DTPA residue.

| Elemental anal. | C | H | N | Li,Cl | |
|---|---|---|---|---|---|
| % calc.: | 57.60 | 7.78 | 6.55 | | |
| % found: | 54.34 | 7.81 | 6.19 | < 0.1 | H₂O 5.12% |

The IR, NMR and MS spectra are consistent with the indicated structure.

### EXAMPLE 13

### Gadolinium complex of [3α(S),5β,7α,12α]-3-[[[[5-(bis[2-[bis(carboxymethyl)amino]ethyl]amino]-5-carboxypentyl] amino]carbonyl]oxy]-7,12-dihydroxycholan-24-oic acid salified with sodium (1:3)

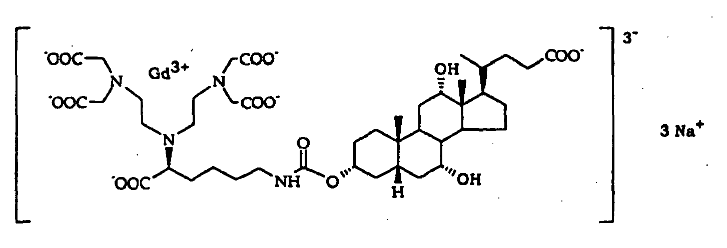

### A) (3α(S),5β,7α,12α]-3-[[[[6-(1,1-Dimethylethoxy)-5-[bis[2-[bis[2-(1,1-dimethylethoxy)-2-oxoethyl] amino]ethyl]amino]-6-oxohexyl]amino]carbonyl]oxy]-7,12-dihydroxy cholan-24-oic acid methyl ester

Method 1: a solution of bis(trichloromethyl) carbonate (2.9 g; 9.7 mmol) in anhydrous CH₂Cl₂ (40 mL) was added dropwise, under nitrogen, to a solution of (3α,5β,7α,12α)-3,7,12-trihydroxycholan-24-oic acid methyl ester (commercial product) (10.0 g; 23.7 mmol) and pyridine (2.3 mL; 28.4 mmol) in anhydrous CH₂Cl₂ (100 mL) cooled to 0°C. The mixture was then allowed to warm and after 1 h at room temperature the solution was cooled again to 0°C, N,N-diisopropylethylamine (7.9 mL; 47.3 mmol) was added then a solution of N²,N²-bis[2-[bis[2-(1,1-dimethylethoxy)-2-oxoethyl]amino]ethyl]-L-lysine (1,1-dimethylethyl) ester (Anelli, P. L. et al Bioconjugate Chem. 1999, 10, 137) (17.6 g; 23.7 mmol) in anhydrous CH₂Cl₂ (50 mL) was added dropwise. The solution was stirred 3 h at room temperature then washed with H₂O (2 x 100 mL), dried over Na₂SO₄ and evaporated. The crude was purified by flash chromatography to give the desidered product (14.8 g; 12.4 mmol).
Yield: 52%.
The ¹H-NMR, ¹³C-NMR, IR and MS spectra are consistent with the indicated structure.

Method 2 : A solution of [3α, 5β, 7α, 12α] -3-[(chlorocarbonyl)oxy]-7,12-dihydroxycholan-24-oic acid methyl ester (Janout, V., Lanier, M.; Regen, S. L. J. Am. Chem. Soc. 1997, 119, 640) (6.1 g; 12.6 mmol) in anhydrous CH₂Cl₂ (150 mL) was cooled to 0°C under nitrogen and then N,N-diisopropylethylamine (4.8 mL; 27.6 mmol) was added. Then a solution of N²,N²-bis[2-[bis[2-(1,1-dimethylethoxy)-2-oxoethyl]amino]ethyl]-L-lysine (1,1-dimethylethyl) ester (9.3 g; 12.6 mmol) in anhydrous CH₂Cl₂ (20 mL) was added dropwise. The solution was stirred 3 h at room temperature then washed with H₂O (2 x 100 mL), dried over Na₂SO₄ and evaporated. The crude was purified by flash chromatography to give the desidered product (11.9 g; 9.9 mmol).
Yield: 79%.
The ¹H-NMR, ¹³C-NMR, IR and MS spectra are consistent with the indicated structure.

### B) [3α(S),5β,7α,12α]-3-[[[[5-[Bis[2-[bis(carboxymethyl)amino]ethyl]amino]-5-carboxypentyl]amino] carbonyl]oxy]-7,12-dihydroxycholan-24-oic acid

A 2 M aq. solution of LiOH (141 mL) was added dropwise to a solution of compound A) (11.2 g; 9.4 mmol) in 1,4 -dioxane (141 mL) at room temperature. After 104 h the solution was concentrated (150 mL) and added dropwise to 2 M aq. HCl (175 mL): final pH was 1.9. The precipitate was filtered, washed with H₂O (5 x 50 mL) and vacuum dried. The crude was purified by flash chromatography. The solid obtained was dissolved in 10% aq. CH₃CN and pH adjusted to 1 by the addition of conc. HCl then the solution was loaded onto an Amberlite® XAD 16.00 resin column (250 mL) and eluted with a CH₃CN/H₂O gradient. The fractions containing the product were evaporated to give the desidered product (4.3 g; 4.8 mmol).
Yield: 51%. m.p.: 184-191°C
K.F.: 4.36%
[α]²⁰_{D}= + 19.5 (c 1, 1 M NaOH)
HPLC assay : 97.3 % (% area)
Stationary phase: Hypurity Elite C 18 5µm; 250 x 4.6 mm column packed by Hypersil;
Temperature: 40°C;
Mobile phase: gradient elution;
A = 0.01 M KH₂PO₄, 0.3 mM EDTA in water;
B = CH₃CN

| Gradient: | min | % A | % B |
|---|---|---|---|
| | 0 | 95 | 5 |
| | 20 | 65 | 35 |
| | 50 | 65 | 35 |

Flow rate: 1 mL min⁻¹;
Detection (UV): 200 nm;

| Elemental analysis | C | H | N | Cl |
|---|---|---|---|---|
| % calc.: | 57.45 | 7.85 | 6.23 | |
| % found: | 55.22 | 7.91 | 6.08 | <0.1 |

The ¹H-NMR, ¹³C-NMR, IR and MS spectra are consistent with the indicated structure.

### C) Gadolinium complex of [3α(S),5β,7α,12α]-3-[[[[5-[bis[2-[bis(carboxymethyl)amino]ethyl]amino]-5-carboxypentyl]amino]carbonyl]oxy]-7,12-dihydroxy-cholan-24-oic acid salified with sodium (1:3)

Compound prepared at step B) (4.7 g; 5.2 mmol) was suspended in H₂O (100 mL) and dissolved by addition of 1 M NaOH (10 mL) until pH was 6.5. A solution of GdCl₃ (1.9 g; 5.2 mmol) in H₂O (17 mL) was added maintaining pH 6.5 with 1 M NaOH (15.6 mL). After 1 h at room temperature, the solution was loaded onto an Amberlite® XAD 16.00 resin column (250 mL) which was eluted with a CH₃CN/H₂O gradient. The fractions containing the product were evaporated to give the desired product (4.2 g; 3.8 mmol).
Yield 72%. m.p.: >300°C
K.F. : 9.49%
[α]²⁰_{D}= + 2.63 (c 2, H₂O)
HPLC assay : 100 % (% area)(same method of step B)

| Elemental analysis | C | H | N | Gd | Na | Cl |
|---|---|---|---|---|---|---|
| % calc.: | 46.15 | 5.76 | 5.00 | 14.05 | 6.17 | |
| % found: | 41.80 | 6.28 | 4.54 | 12.64 | 5.73 | <0.1 |

The ¹H-NMR, ¹³C-NMR, IR and MS spectra are consistent with the indicated structure.

### EXAMPLE 14

### Manganese complex of [3β(S),5β,12α]-3- [[4-[[2-[bis-(carboxymethyl)amino]ethyl](carboxymethyl)amino]-4-car-boxy-1-oxobutyl]amino]-12-hydroxycholan-24-oic acid salified with sodium (1:3)

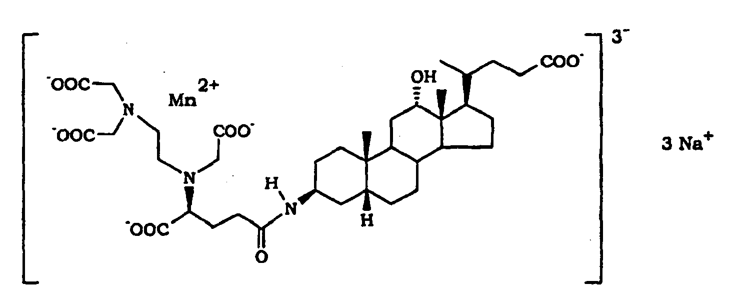

### A) [3β(S),5β,12α]-3-[[4-Amino-5-methoxy-1,5-dioxopen-tyl]amino]-12-hydroxycholan-24-oic acid methyl ester

3.4 g of 5 % Pd/C is added to a solution of [3β(S),5β,12α]-3-[[5-methoxy-1,5-dioxo-4-[[(phenylmetho-xy)carbonyl]amino]pentyl]amino]-12-hydroxycholan-24-oic acid methyl ester (example 5, product B) (34.14 g; 50 mmol) in MeOH (340 mL). The suspension was stirred, at r.t., over 3.5 h under a hydrogen atmosphere. After filtration through a Millipore® filter FH (0.45 µm) the solution was evaporated to dryness to give the desired product (27.1 g; 49.3 mmol).
Yield 98.6 %.
Weight loss (50°C; high vacuum): <0.1%

| Elemental analysis | C | H | N |
|---|---|---|---|
| % calc.: | 67.85 | 9.55 | 5.10 |
| % found: | 68.58 | 9.72 | 5.12 |

[α]²⁰_{D}= + 36.61 (c 2.01, CHCl₃)
Acidic titer (0.1 M HCl) : 94.6 %
The ¹H-NMR, ¹³C-NMR, IR and MS spectra are consistent
with the indicated structure.

### B) [3β(S),5β,12α]-3-[[4-[[2-[Bis[2-(1,1-dimethylethoxy)-2-oxoethyl]amino]ethyl][2-(1,1-dimethylethoxy)-2-oxoethyl]amino]-5-methoxy-1,5-dioxopentyl]amino]-12-hydroxycholan-24-oic acid methyl ester

Compound prepared at step A) (16.0 g; 29.1 mmol) and N-(2-bromoethyl)-N-[2-(1,1-dimethylethoxy)-2-oxoethyl]-glicine 1,1-dimethylethyl ester (13.3 g; 37.8 mmol) (prepared according to the procedure described in WO-A-95/32741: example 15) were dissolved in EtOAc (120 mL). After addition of 2 M phosphate buffer pH 8 (120 mL) the mixture was vigorously stirred for 2 h, then the aqueous phase was replaced with fresh 2 M phosphate buffer pH 8 (120 mL) and stirred for further 70 h. The mixture was warmed to 40°C for 12 h, cooled to room temperature, separated and the organic phase evaporated to give a residue which was dissolved in CH₂Cl₂ (150 mL), washed with water (2 x 100 mL), dried over Na₂SO₄ and evaporated. The crude was purified by flash chromatography to give the desidered product (12.3 g; 15.0 mmol).
Yield: 52%.

| Elemental analysis | C | H | N |
|---|---|---|---|
| % calc.: | 65.90 | 9.46 | 5.12 |
| % found: | 65.99 | 9.72 | 5.03 |

TLC: Stationary phase: silica gel plate 60F 254 Merck
Eluent: 1 : 1 = EtOAc/n-hexane R_{f}= 0.40
Detection: 1% KMnO₄ in 1 M NaOH
The ¹H-NMR, ¹³C-NMR, IR and MS spectra are consistent with the indicated structure.

### C) [3β(S),5β,12α]-3-[[4-[[2-[Bis[2-(1,1-dimethylethoxy)-2-oxoethyl]amino]ethyl][2-(1,1-dimethylethoxy)-2-oxoethyl]amino]-5-methoxy-1,5-dioxopentyl]amino]-12-hydroxycholan-24-oic acid methyl ester

Tert-butyl bromoacetate (3.9 g; 20.1 mmol) was added dropwise to a solution of compound prepared at step B) (11.0 g; 13.4 mmol) and N,N-diisopropylethylamine (3.5 mL; 20.1 mmol) in CH₃CN. The mixture was stirred 24 h at room temperature and then more N,N-diisopropylethylamine (0.9 mL; 4.0 mmol) and *tert*-butyl bromoacetate (0.8 g; 4.0 mmol) were added. The mixture was stirred for further 70 h, separated and the organic phase evaporated to give a residue which was dissolved in CH₂Cl₂ (150 mL), washed with water (2 x 100 mL), dried over Na₂SO₄ and evaporated. The crude was purified by flash chromatography to give the desired product (10.7·g; 11.5 mmol).
Yield: 85%.

| Elemental analysis | C | H | N |
|---|---|---|---|
| % calc.: | 65.57 | 9.39 | 4.50 |
| % found: | 66.27 | 9.62 | 4.52 |

TLC: Stationary phase: silica gel plate 60F 254 Merck
Eluent: 1 : 1 = EtOAc/n-hexane R_{f}= 0.46
Detection: 1% KMnO₄ in 1 M NaOH
The ¹H-NMR, ¹³C-NMR, IR and MS spectra are consistent with the indicated structure.

### D) [3β(S),5β,12α]-3-[[4-[[2-[Bis(carboxymethyl)amino] ethyl](carboxymethyl)amino]-4-carboxy-1-oxobutyl]-amino]-12-hydroxycholan-24-oic acid

A 2 M aq. solution of LiOH (120 mL) was added dropwise to a solution of compound prepared at step C)(9.2 g; 9.8 mmol) in 1,4-dioxane (120 mL) at room temperature. After 24 h the solution was concentrated (100 mL) and.added dropwise to 2 M aq. HCl (135 mL): final pH was 1.9. The precipitate was filtered, washed with H₂O (5 x 50 mL) and vacuum dried to give the desired product (7.3 g; 9.6 mmol).
Yield 98% m.p.: 163-168°C
K.F. : 1.81%
[α]²⁰D= + 29.03 (c 1, 1 M NaOH)
HPLC assay : 100% (area %)
Stationary phase: Zorbax Eclipse XDB-C8 3.5µm; 150 x 4.6 mm column packed by Rockland Technologies Inc.;
Temperature: 40°C;
- Mobile phase:: gradient elution;
A = 0.01 M KH₂PO₄, 0.01 M K₂HPO₄, 0.3 mM
EDTA in water;
B = CH₃CN

| Gradient: | min | % A | % B |
|---|---|---|---|
| | 0 | 93 | 7 |
| | 5 | 93 | 7 |
| | 50 | 60 | 40 |

Flow rate: 1 mL min⁻¹;
Detection (UV): 200 nm;

| Elemental analysis | C | H | N | Li | Cl |
|---|---|---|---|---|---|
| % calc.: | 60.23 | 8.06 | 5.69 | | |
| % found: | 58.60 | 8.25 | 5.49 | <0.1 | <0.1 |

The ¹H-NMR, ¹³C-NMR, IR and MS spectra are consistent with the indicated structure.

### E) Manganese complex of [3β(S),5β,12α]-3- [[4-[[2-[bis(carboxymethyl)amino]ethyl](carboxymethyl)amino] -4-carboxy-1-oxobutyl]amino]-12-hydroxycholan-24-oic acid salified with sodium (1:3)

The compound prepared at step D) (5.3 g; 7.2 mmol) was suspended in H₂O (250 mL) and dissolved by addition of 1 M NaOH (36 mL) until pH was 6.5. A solution of MnCl₂·4H₂O (1.4 g; 7.2 mmol) in H₂O (50 mL) was added maintaining pH 6.5 with 1 M NaOH (7.9 mL). After 1 h at room temperature, the solution was desalted by nanofiltration, then evaporated to give the desired product.
Yield: 98% m.p.: >300°C
K.F. : 13.54%
[α]²⁰D= + 2.63 (c 2, H₂O)
CE assay : 100% (area %)
- Capillary:: fused silica 0.72 m x 50 µm
- Voltage:: 30 kV
- Buffer:: 0.07 M borate, pH 9,3, 0.3 mM EDTA
- Temperature:: 25°C
- Stoptime:: 20 min;
- Detection (UV):: 200 nm;
- Injection:: hydrodynamic (50 mbar, 4 s);
- Sample concentration:: 1 mg mL⁻¹;
- Instrumentation: Hewlett: Packard 3D HPCE
- Preconditioning timetable:: t (min) action

| | |
|---|---|
| 2 | flush with H₂O |
| 2 | flush with 0.1 M NaOH |
| 1 | flush with H₂O |
| 5 | flush with buffer |

| | | | | | | |
|---|---|---|---|---|---|---|
| Elemental analysis | C | H | N | Mn | Na | Cl |

| % calc.: | 51.87 | 6.35 | 4.90 | 6.41 | 8.05 | |
|---|---|---|---|---|---|---|
| % found: | 45.50 | 6.95 | 4.30 | 5.28 | 6.86 | <0.1 |

The IR and MS spectra are consistent with the indicated structure.

In the same way starting from compound B) in example 2 the gadolinium complex of [3β(S),5β]-3-[[4-[[2-[[bis(carboxymethyl)amino]ethyl]-(carboxymethyl)amino]-4-carboxy-1-oxobutyl]amino]-12-oxocholan-24-oic acid was prepared.

### EXAMPLE 15

### Manganese complex of [3α(S),5β,12α]-3-[[[[5-[[2-[bis(carboxymethyl)amino]ethyl](carboxymethyl)amino]-5-carboxypentyl]amino]carbonyl]oxy]-12-hydroxycholan-24-oic acid salified with sodium (1:3)

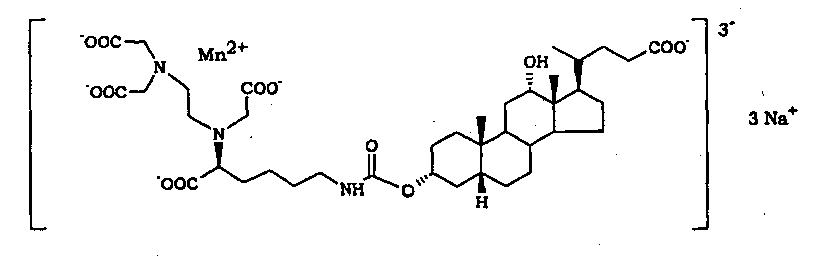

### A) N²-[2-[Bis[2-(1,1-dimethylethoxy)-2-oxoethyl]-amino]ethyl]-N²-[2-(1,1-dimethylethoxy)-2-oxoethyl]-N⁶-[(phenylmethoxy)carbonyl]-L-lysine methyl ester

N-(2-Bromoethyl)-N-[2-(1,1-dimethylethoxy)-2-oxoethyl]glycine 1,1-dimethylethyl ester (25.6 g; 72.55 mmol) (prepared according to the procedure described in WO-A-95/32741: example 15) dissolved in CH₃CN (25 mL) was added dropwise to a solution of *N*⁶-[(phenylmethoxy)carbonyl]-L-lysine methyl ester hydrochloride (20 g; 60.46 mmol) and N,N-diisopropylethylamine (12.64 mL; 72.55 mmol) in CH₃CN (250 mL). The reaction mixture was stirred at room temperature. After 5 days more N,N-diisopropylethylamine (17.7 mL; 101.6 mmol) and *tert*-butyl bromoacetate (18.8 g; 13.5 mL; 101.6 mmol) were added to the solution. After 24 h the solvent was evaporated and the residue was treated with Et₂O (200 mL). The mixture was filtered, the solution washed with 0.1 M HCl (2 x 100 mL), dried over Na₂SO₄ and evaporated under reduced pressure. The crude was purified by flash chromatography to give the desired product (13.03 g; 19.2 mmol).
Yield: 32%.
K.F. : <0.1%
[α]²⁰_{D}= - 22.47 (c 1.93, CHCl₃)

| Elemental analysis | C | H | N |
|---|---|---|---|
| % calc.: | 61.83 | 8.45 | 6.18 |
| % found: | 61.70 | 8.52 | 5.84 |

TLC: Stationary phase: silica gel plate 60F 254 Merck
Eluent: 3 : 7 = EtOAc/n-hexane R_{f}= 0.40
Detection: Ce(SO₄)₂, 4 H₂O (0.18%) and (NH₄)₆Mo₇O₂₄·4 H₂O (3.83%) in 10% H₂SO₄
The ¹H-NMR, ¹³C-NMR, IR and MS spectra are consistent with the indicated structure.

### B) (3α,5β,7α,12α)-3-[(Chlorocarbonyl)oxy]-12-hydroxy-cholan-24-oic acid methyl ester

### CAUTION: all the operations must be performed under a well ventilated fume hood

A 20% solution of phosgene in toluene (100 mL; 202.2 mmol) was added dropwise to a solution of (3α,5β,7α,12α)-3,12-dihydroxycholan-24-oic acid methyl ester (14.7 g; 36 mmol) in anhydrous CH₂Cl₂ (350 mL) cooled to 0°C under nitrogen. The solution was stirred 3 h at room temperature then evaporated (CAUTION) to give the desired product (15.2 g; 32.4 mmol).
Yield: 90%.
The ¹H-NMR, ¹³C-NMR, IR and MS spectra are consistent with the indicated structure.

### C) [3α(S),5β,12α]-3-[[[[5-[[2-[Bis[2-(1,1-dimethyletho-xy)-2-oxoethyl]amino]ethyl][2-(1,1-dimethylethoxy)-2-oxoethyl]amino]-6-methoxy-6-oxohexyl]amino]carbonyl]oxy]-12-hydroxycholan-24-oic acid methyl ester

5% Pd/C (1.3 g) was added to a solution of compound prepared at step A) (12.3 g; 18.1 mmol) in MeOH (120 mL) and the suspension was stirred for 3 h under hydrogen atmosphere at room temperature. After filtration through a Millipore^{®} filter (FT 0.45 µm) the solution was evaporated under reduced pressure. The crude was dissolved in anhydrous CH₂Cl₂ (20 mL) and added dropwise to a solution of compound prepared at step B) (8.7 g; 18.54 mmol) and N,N-diisopropylethylamine (DIEA) (6.5 mL; 37.07 mmol) in anhydrous CH₂Cl₂ (200 mL) at 0°C and under nitrogen. After 3 h the reaction mixture was washed with H₂O (2 x 100 mL), the organic phase was separated, dried over Na₂SO₄ and evaporated under reduced pressure. The crude was purified by flash chromatography to give the desired product(8.6 g; 8.8 mmol).
Yield: 49%.
K.F. : <0.1%

| Elemental analysis | C | H | N |
|---|---|---|---|
| % calc.: | 65.07 | 9.38 | 4.30 |
| % found: | 65.67 | 9.52 | 4.24 |

TLC: Stationary phase: silica gel plate 60F 254 Merck
Eluent: 3 : 7 = EtOAc/n-hexane R_{f}= 0.30
Detection: Ce(SO₄)₂,4 H₂O (0.18%) and (NH₄)₆Mo₇O₂₄·4 H₂O (3.83%) in 10% H₂SO₄
The ¹H-NMR, ¹³C-NMR, IR and MS spectra are consistent with the indicated structure.

### D) [3α(S),5β,12α]-3-[[[[5-[[2-[Bis(carboxymethyl)amino]ethyl](carboxymethyl)amino]-5-carboxypentyl]amino]carbonyl]oxy]-12-hydroxycholan-24-oic acid

2 M LiOH (133 mL; 266 mmol) was added to a solution of compound prepared at step C) (8.5 g; 10.64 mmol) in 1,4-dioxane (130 mL). The resulting solution was stirred at room temperature for 24 h and then neutralized to pH 7 by addition of 2 N HCl (120 mL). The solution was concentrated by evaporation under reduced pressure to half volume and very slowly acidified to pH 1.5 under. vigorous stirring to afford a white precipitate which was filtered, washed with H₂O (2 x 100 mL) and dried to give the desired product (6.45 g; 8.13 mmol).
Yield: 76%. m.p.: 188.9°C
K.F. : 1.44%
[α]²⁰D= + 35.47 (c 2.01, 1 M NaOH)
HPLC assay: 96.8% (area %)
Stationary phase: Zorbax Eclipse XDB-C₈ 3.5µm; 150 x 4.6 mm column packed by Rockland Technologies Inc.;
Temperature: 40°C;
Mobile phase: gradient elution;
A = 0.017 M H₃PO₄ in water;
B = CH₃CN

| Gradient: | min | % A | % B |
|---|---|---|---|
| | 0 | 65 | 35 |
| | 25 | 15 | 85 |
| | 30 | 15 | 85 |

Flow rate: 1 mL min⁻¹;
Detection (UV): 210 nm;

| Elemental analysis | C | H | N | Li | Cl |
|---|---|---|---|---|---|
| % calc.: | 59.91 | 8.12 | 5.37 | | |
| % found: | 58.44 | 8.04 | 5.03 | <0.1 | <0.1 |

The ¹H-NMR, ¹³C-NMR, IR and MS spectra are consistent with the indicated structure.

### E) Manganese complex of [3α(S),5β,12α]-3-[[[[5-[[2-[bis(carboxymethyl)amino]ethyl](carboxymethyl)amino] -5-carboxypentyl]amino]carbonyl]oxy]-12-hydroxy-cholan-24-oic acid salified with sodium (1:3)

The compound prepared at step D) (5 g; 6.3 mmol) was suspended in H₂O (50 mL) and dissolved by addition of 1 M NaOH (18 mL; 18 mmol). A 0.559 M aq. solution of MnCl₂ (11.27 mL; 6.3 mmol) was added over 3 h maintaining pH 6.5 by means of 1 M NaOH (7.8 mL). The solution was adjusted to pH 6.8 with 1 M NaOH (0.9 mL; 0.9 mmol), filtered (HA 0.45 µm Millipore® membrane) and desalted by nanofiltration. The solution was evaporated and dried to give the desired product (5.45 g; 6.05 mmol).
Yield: 96% m.p.: >300°C
K.F. : 3.78%
[α]²⁰D= + 2.74 (c 2, H₂O)
CE assay : 100% (area %)
- Capillary:: fused silica 0.72 m x 50 µm
- Voltage:: 30 kV
- Buffer:: 0.07 M borate, pH 9,3, 0.3 mM EDTA
- Temperature:: 25 °C
- Stoptime:: 20 min;
- Detection (UV):: 200 nm;
- Injection:: hydrodynamic (50 mbar, 4 s);
- Sample concentration: 1: mg mL⁻¹;
- Instrumentation:: Hewlett Packard 3D HPCE
- Preconditioning timetable:: t (min) action

| | |
|---|---|
| 2 | flush with H₂O |
| 2 | flush with 0.1 M NaOH |
| 1 | flush with H₂O |
| 5 | flush with buffer |

| Elemental analysis | C | H | N | Mn | Na | Cl |
|---|---|---|---|---|---|---|
| % calc.: | 52.00 | 6.49 | 4.66 | 6.10 | 7.66 | |
| % found: | 49.54 | 7.17 | 4.44 | 5.65 | 7.58 | <0.1 |

The IR and MS spectra are consistent with the indicated structure.

### EXAMPLE 16

### Gadolinium complex of N²-bis[2-[bis(carboxymethyl) amino]ethyl]-N-[(3β,5β,7α,12α)-7,12-dihydroxy-24-oxo-24-[(2-sulfoethyl)amino] cholan-3-yl]-L-glutamine salified with sodium (1:3)

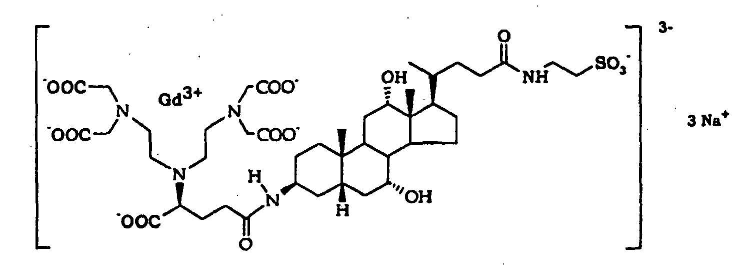

### A) [3β(S),5β,7α,12α]-3-[[4-[Bis[2-[bis[2-(1,1-dime-thylethoxy)-2-oxoethyl]amino]ethyl]amino]-5-(1,1-dimethylethoxy)-1,5-dioxopentyl]amino]-7,12-dihy-droxycholan-24-oic acid phenylmethyl ester

N,N-Bis[2-[bis[2-(1,1-dimethylethoxy)-2-oxoethyl]-amino]ethyl]-L-glutamic acid 1-(1,1-dimethyl ethyl) ester (Anelli, P. L. et al. Bioconjugate Chem. 1999, 10, 137) (37 g; 50 mmol), (3β,5β,7α,12α)-3-amino-7,12-dihydroxycholan-24-oic acid phenylmethyl ester (Anelli, P. L.; Lattuada, L.; Uggeri, F. Synth. Commun. 1998, 28, 109) (31 g; 55 mmol) and diethyl cyanophosphonate (commercial product) (9.6 g; 55 mmol; 9.2 mL) were dissolved in DMF (750 mL). The resulting solution was cooled to 0°C and Et₃N (7.3 mL) was added dropwise. After 1 h at room temperature the solution was,evaporated under reduced pressure, the residue was dissolved in EtOAc (300 mL), washed with a 5% aq. NaHCO₃ (2 x 200 mL) and then with brine (2 x 200 mL). The organic phase was separated, dried over Na₂SO₄ and then evaporated under reduced pressure. The crude was purified by flash chromatography to give the desired product (36 g; 29 mmol).
Yield 58%.
K.F. : 0.98%

| Elemental analysis | C | H | N |
|---|---|---|---|
| % calc.: | 65.64 | 9.21 | 4.57 |
| % found: | 66.31 | 9.20 | 4.51 |

TLC: Stationary phase: silica gel plate 60F 254 Merck
Eluent: 2 : 8 = EtOAc/*n*-hexane R_{f}= 0.3
Detection: AcOH/conc. H₂SO₄/p-anisaldehyde = 100:2:1
The ¹H-NMR, ¹³C-NMR, IR and MS spectra are consistent with the indicated structure.

### B) [3β(S),5β,7α,12α]-3- [[4-[Bis[2-[bis[2-(1,1-dimethyl-ethoxy)-2-oxoethyl]amino]ethyl] amino]-5-(1,1-dime-thylethoxy)-1,5-dioxopentyl]amino]-7,12-dihydroxy-cholan-24-oic acid triethylammonium salt

5% Pd/C (3.6 g) was added to a solution of compound prepared at step A) (36 g; 29.4 mmol) in EtOH (1.5 L) and the suspension was stirred for 3 h under hydrogen atmosphere at room temperature. After filtration (through a Millipore® filter FT 0.45 µm) the solution was evaporated under reduced pressure. The crude was purified by flash chromatography to give the desired product (22 g; 18 mmol).
Yield 60 %. m.p.: 58°C
K.F.: 1.34%

| Elemental analysis | C | H | N |
|---|---|---|---|
| % calc.: | 65.07 | 9.86 | 5.66 |
| % found: | 64.34 | 10.07 | 5.48 |

TLC: Stationary phase: silica gel plate 60F 254 Merck
Eluent: 1 : 5 : 95 = Et₃N/MeOH/CH₂Cl₂ R_{f}= 0.33
Detection: AcOH/conc. H₂SO₄/p-anisaldehyde = 100:2:1
The ¹H-NMR, ¹³C-NMR, IR and MS spectra are consistent with the indicated structure.

### C) N²-Bis[2-[bis(carboxymethyl)amino]ethyl]-N-[(3β,5β,7α,12α)-7,12-dihydroxy-24-oxo-24-[(2-sulfoethyl)amino]cholan-3-yl]-L-glutamine

Et₃N (1.2 g; 12 mmol; 1.7 mL) was added dropwise to a solution of compound B) (12.5 g; 11 mmol), (2-aminoethanesulfonic acid (commercial product) (1.5 g; 12 mmol) and diethyl cyanophosphonate (commercial product) (2.1 g; 12 mmol; 2 mL) in DMF (400 mL) at 0°C and under nitrogen. After 20 min the reaction mixture was allowed to rise to room temperature and stirred for 3 h. The solution was evaporated under reduced pressure, the residue was dissolved in dioxane (250 mL) and 0.5 M aq. H₂SO₄ (250 mL; 125 mmol) was added dropwise. The resulting mixture was heated at 90°C for 2 h. The pH of the solution was adjusted from 1.4 to 7 with 2 N NaOH and the solvent was evaporated under reduced pressure. The crude was purified by flash chromatography. The product was dissolved in H₂O (250 mL) and 2 N HCl (12.5 mL) and desalted by elution through an Amberlite® XAD-16.00 resin column with a CH₃CN/H₂O gradient to give the desired product (1.5 g; 1.6 mmol).
Yield 14%. m.p.: >200°C
K.F.: 5.87%

| Elemental analysis | C | . H | N | S |
|---|---|---|---|---|
| % calc.: | 53.68 | 7.44 | 7.28 | 3.33 |
| % found: | 50.34 | 7.71 | 6.64 | 2.99 |

The ¹H-NMR, ¹³C-NMR, IR and MS spectra are consistent with the indicated structure.

### D) Gadolinium complex of N²-bis[2-[bis(carboxymethyl) amino]ethyl]-N-[(3β,5β,7α,12α)-7,12-dihydroxy-24-oxo-24-[(2-sulfoethyl)amino] cholan-3-yl]-L-glutamine salified with sodium (1:3)

Product C) (880 mg; 0.915 mmol) was dissolved in H₂O (50 mL) and 1 N NaOH was added dropwise until pH 6.8 was reached. Gd₂O₃ (165 mg; 0.46 mmol) was added and the resulting suspension was heated at 50°C for 6 h. The reaction mixture was filtered through a Millipore® apparatus (HA 0.45 µm filter) and the filtrate was loaded onto a Dowex^{®} CCR 3LB weak cation exchange resin column (Na⁺ form, 20 mL). The eluate was evaporated under reduced pressure and dried to give the desired product (1.00 g; 0.75 mmol).
Yield 82%. m.p.: >250°C
K.F.: 13.95%
HPLC assay : 100% (area %)
Stationary phase: Lichrospher 100 RP-8 5µm; 250 x 4 mm column packed by Merck KGaA;
Temperature: 40°C;

Mobile phase: isocratic elution with premixed mobile phase: 1 g of n-octylamine is added to 300 mL of acetonitrile mixed with 700 mL of water. The solution is buffered to pH 6 with H₃PO₄;
Flow rate: 1 mL min⁻¹;
Detection (UV): 200 nm;

| | | | | | | |
|---|---|---|---|---|---|---|
| Elemental analysis | C | H | N | S | Gd | Na |
| % calc.: | 43.78 | 5.54 | 5.92 | 2.71 | 13.30 | 5.83 |
| % found: | 36.82 | 6.04 | 5.11 | 2.18 | 10.64 | 5.35 |

The IR and MS spectra are consistent with the indicated structure.

In the same way the gadolinium complex of N²-bis[2-[bis(carboxymethyl)amino]ethyl]-N-[(3(3,5(3)-24-oxo-24-((2-sulfoethyl)amino]cholan-3-yl]-L-glutamine was prepared.

### EXAMPLE 17

### Gadolinium complex of [3α(S),5β]-3-[2-[[5-[bis[2-[bis(carboxymethyl)amino]-ethyl)amino)-5-carboxypentyl] amino]-2-oxoethoxy]cholan-24-oic acid salified with sodium (1:3)

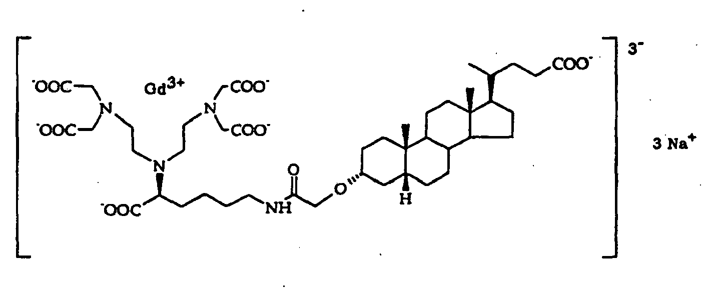

### A) (3α,5β)-3-(Carboxymethoxy)cholan-24-oic acid methyl ester

Benzyl glycolate triflate (Williams, M. A.; Rapoport, H. J. Org. Chem. 1994, 59, 3616) (16.8 g; 56.3 mmol)was added over a period of 15 min to a stirred solution of (3α,5β)-3-hydroxycholan-24-oic acid methyl ester (Dayal, B. et al. Steroids 1981, 37, 239) (20 g; 51.2 mmol) and N,N-diisopropylethylamine (10 mL; 57.4 mmol) in anhydrous CH₃CN (400 mL) at -20°C. After 4 h at -20°C the mixture was warmed to room temperature and stirred for additional 4 h. The solvent was evaporated and the residue partitioned between EtOAc (300 mL) and saturated NaHCO₃ (300 mL). The organic phase was dried over Na₂SO₄ and evaporated. The residue was dissolved in EtOH (200 mL) then 5 % Pd/C (3 g) was added and the mixture was stirred, at r.t., over 5 h under a hydrogen atmosphere. After filtration through a Millipore® filter FH (0.45 µm) the solution was evaporated and the residue purified by flash chromatography to give the desired product (14.2 g; 31.7 mmol). Yield: 62%
K.F. : <0.1

| Elemental analysis | C | H |
|---|---|---|
| % calc.: | 72.28 | 9.89 |
| % found: | 71.97 | 9:81 |

The ¹H-NMR, ¹³C-NMR, IR and MS spectra are consistent
with the indicated structure.

### B) [3α(S),5β]-3-[2-[[5-[bis[2-[bis(carboxymethyl)amino] ethyl]amino]-5-carboxypentyl]amino]-2-oxoethoxy]cho-lan-24-oic acid

N²,N²-bis[2-[bis[2-(1,1-dimethylethoxy)-2-oxoethyl]-amino]ethyl]-L-lysine (1,1-dimethylethyl) ester (Anelli, P. L. et al Bioconjugate Chem. 1999, 10, 137) (18.56 g; 25 mmol), (18.6 g; 25 mmol), compound A) (11.2 g; 25 mmol) and diethyl cyanophosphonate (commercial product) (4.9 g; 28 mmol) were dissolved in DMF (300 mL). The resulting solution was cooled to 0°C and Et₃N (4 mL) was added dropwise. After 6 h at room temperature the solution was evaporated under reduced pressure, the residue was dissolved in EtOAc (250 mL), washed with a 5% aq. NaHCO₃ (2 x 100 mL) and then with brine (2 x 100 mL). The organic phase was separated, dried over Na₂SO₄ and then evaporated under reduced pressure. The residue was dissolved in dioxane (300 mL) and 0.5 M aq. H₂SO₄ (300 mL; 150 mmol) was added dropwise. The resulting mixture was heated at 90°C for 2 h. The pH of the solution was adjusted to 7 with 2 N NaOH and the solvent was evaporated under reduced pressure. The crude was purified by flash chromatography. The product was dissolved in H₂O (300 mL) and 2 N HCl (30 mL) and desalted by elution through an Amberlite® XAD-16.00 resin column with a CH₃CN/H₂O gradient to give the desired product (9.03 g; 10.25 mmol). Yield 41%.
K.F.: 2.78%

| Elemental analysis | C | H | N |
|---|---|---|---|
| % calc.: | 59.98 | 8.24 | 6.36 |
| % found: | 58.11 | 8.28 | 6.14 |

The ¹H-NMR, ¹³C-NMR, IR and MS spectra are consistent with the indicated structure.

### C) Gadolinium complex of [3α(S),5β] -3- [2- [[5- [bis[2-[bis(carboxymethyl)aminoj-ethyl]amino]-5-carboxypentyl]amino]-2-oxoethoxy]cholan-24-oic acid salified with sodium (1:3)

Product B) (5 g; 5.67 mmol) was dissolved in H₂O (200 mL) by addition of 1 N NaOH until pH 6.8 was reached. Gd₂O₃ (1.03 g; 2.84 mmol) was added and the resulting suspension was heated at 50°C for 8 h. The reaction mixture was filtered through a Millipore® apparatus (HA 0.45 µm filter) and the filtrate was evaporated under reduced pressure and dried to give the desired product (5.74 g; 5.21 mmol).
Yield 92%. m.p.: >250°C
K.F.: 5.81%

| Elemental analysis | C | H | N | Gd | Na |
|---|---|---|---|---|---|
| % calc.: | 47.99 | 6.04 | 5.09 | 14.28 | 6.26 |
| % found: | 45.09 | 6.15 | 4.77 | 13.39 | 5.81 |

The IR and MS spectra are consistent with the indicated structure.

In the same way, starting from compound A) of example 15, the gadolinium complex of [3α(S),5β]-3-[2-[[5-[[2-[bis(carboxymethyl)amino]ethyl](carboxymethyl)-amino]-5-carboxypentyl]amino]-2-oxoethoxy]cholan-24-oic acid was prepared.

In the same way, starting from compound A) of example 15 and (3β,5β,7α,12α)-3-[(3-carboxy-1-oxopropyl)amino]-7,12-dihydroxycholan-24-oic acid methyl ester (prepared according to the procedure described in WO-A-95/32741: example 12), the gadolinium complex of [3β(S),5β,7α,12α]-3-[[4-[[5-[[2-[bis(carboxymethyl)-amino]ethyl](carboxymethyl)amino]-5-carboxypentyl]amino]-1,4-dioxobutyl]amino]-7,12-dihydroxycholan-,24-oic acid was prepared.

### EXAMPLE 18

### Measurements of the relaxation rate (Δ1/T₁)

The effectiveness of the compounds of the invention as blood pool agents was evaluated by plotting the progress of the longitudinal relaxation rate 1/T₁ versus the time elapsed after the administration. The proton relaxation rate 1/T₁ of blood samples, collected at predetermined times, was measured at 39°C by means of a Brucker Minispec PC120 instrumentation using "inversion recovery" three parameter sequences.

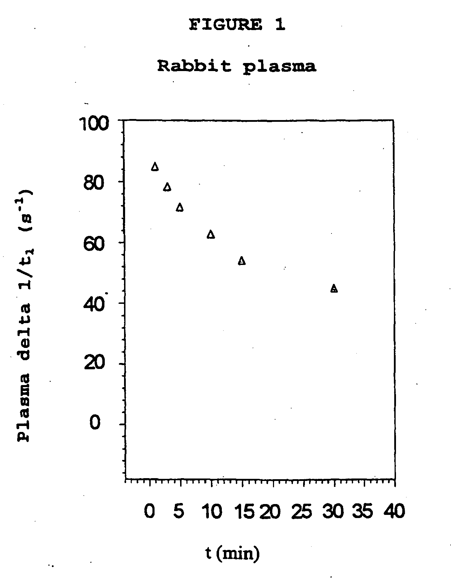

The compound prepared in Example 1, gadolinium complex of [3β(S),5β]-3-[[4-[bis[2-[bis(carboxymethyl)amino]ethyl]amino]-4-carboxy-1-oxobutyl]amino]cholan-24-oic acid salified with 1-deoxy-1-(methylamino)-D-glucitol (1:3), was administered to a rabbit at a dose of 0.1 mmol/kg. The diagram (fig. 1) represents the profile of the relaxation rate.

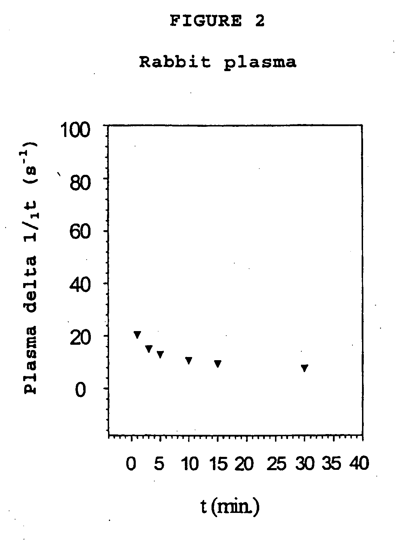

The compound prepared in Example 9 of patent application WO 95/32741, gadolinium complex of (3β,5β,7α,12α)-3-[[N-[N-[2-[[2-[bis (carboxymethyl)-amino]ethyl](carboxymethyl)amino]ethyl)-N-(carboxymethyl)-glycyl]glycyl]amino]-7,12-dihydroxy-cholan-24-oic acid salified with 1-deoxy-1-(methylamino)-D-glucitol (1:2), was administered to a rabbit at a dose of 0.1 mmol/kg. The diagram (fig. 2) represents the profile of the relaxation rate.

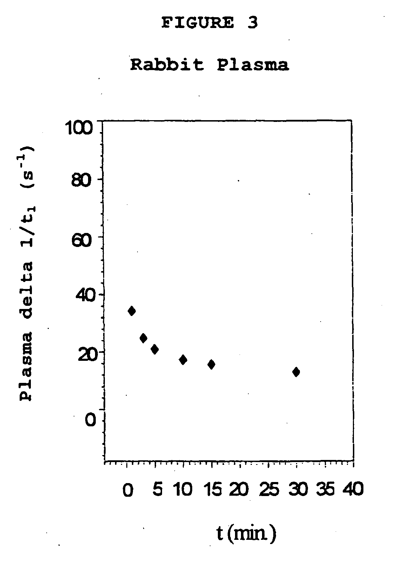

The compound prepared in Example 15 of patent application WO 95/32741, gadolinium complex of [3β(S),5β,7α,12α]-3-[[4-[bis[2-[bis(carboxymethyl)amino]ethyl]amino]-4-carboxy-1-oxobutyl]amino]-7,12-dihydro-xycholan-24-oic acid salified with 1-deoxy-1-(methylamino)-D-glucitol (1:3), was administered to the rabbit at a dose of 0.1 mmol/kg. The diagram (fig. 3) represents the profile of the relaxation rate.

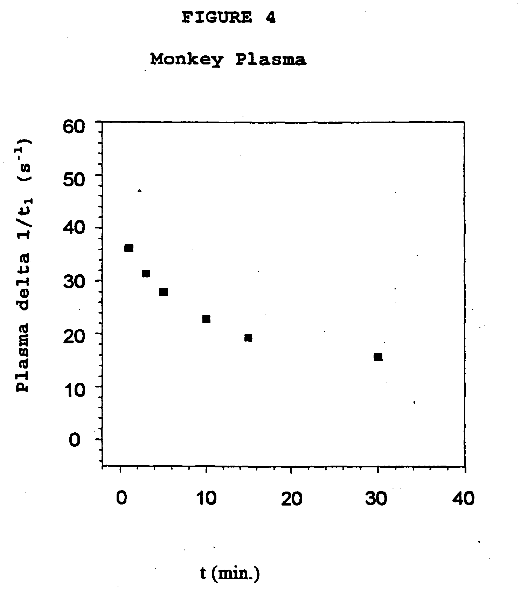

The compound prepared in Example 4, gadolinium complex of [[3β(S),5β,12α]-3-[[4-[bis[2-[bis(carboxymethyl)amino]ethyl]amino]-4-carboxy-1-oxobutyl]amino]-12-hydroxycholan-24-oic acid salified with 1-deoxy-1-(methylamino)-D-glucitol (1:3), was administered to the monkey at a dose of 0.05 mmol/kg. The diagram (fig. 4) is the profile of the relaxation rate.

### EXAMPLE 19

0.3 M Pharmaceutical formulation of the gadolinium complex with [3β(S),5β,12α]-3-[[4-[bis[2-[bis(carboxymethyl)amino]ethyl]amino]-4-carboxy-l-oxobutyl]amino]-12-hydroxycholan-24-oic acid salified with 1-deoxy-l-(methylamino)-D-glucitol (1:3)

31.775 kg of the gadolinium complex of [3β(S),5β,12α]-3- [[4-[bis[2-[bis(carboxymethyl)amino]-ethyl]amino]-4-carboxy-1-oxobutyl]ammino]-12-hydroxycho-lan-24-oic acid salified with 1-deoxy-1-(methylamino)-D-glucitol (1:3) (prepared as exemplified in Example 4) and 100 g of trometamol hydrochloride are dissolved in 100 L of sterile water at room temperature in a pharmaceutical stainless steel reactor. After dissolution, the pH of the solution is adjusted to 7.4 by addition of 1 M trometamol. The solution is sterile filtered through filters of 0.22 mm diameter and distributed in 20 mL vials, which are closed with halobutyl plugs, sealed with aluminium ring and vapour sterilized at Fo = 18. HPLC shows a 0.294 M titre.

### EXAMPLE 20

### Pre-filled plastic syringes containing the formulation of Example 19

20 mL Portions of the solution prepared in Example 19 are placed in a CZ plastic syringe with the tip closed by a cap. The piston is inserted under vacuum and the pre-filled syringe is sterilized in autoclave to a value of Fo = 18.

## Claims

1. The use of the chelated complexes of bi- trivalent paramagnetic metal ions which are Fe⁽²⁺⁾ or Fe⁽³⁺⁾ or Cu⁽²⁺⁾ or Cr₍₃₊₎ or Gd⁽³⁺⁾ or Eu⁽³⁺⁾ or Dy⁽³⁺⁾ or Yb⁽³⁺⁾ or Mn⁽²⁺⁾ with the compounds of formula (I), as well as the salts thereof with physiologically compatible organic bases which are primary, secondary, tertiary amines or basic amino acids, or with inorganic bases whose cations are sodium, potassium, magnesium, calcium or mixtures thereof:
X-L-Y (I),
wherein:
X is the residue of a polyammocarboxylic ligand or derivatives thereof, i.e. ethylenediaminotetraacetic acid (EDTA) or diethylenetriaminopentaacetic acid (DTPA) or 1,4,7,10-tetraazacyclododecane-1,4,7,10-tetraacetic acid (DOTA) or 1,4,7,10-tetraazacyclododecane-1,4,7-triacetic acid (DO3A) or (10-(2-hydroxypropyl)-1,4,7,10-tetraazacyclododecane-1,4,7-triacetic acid (HPDO3A) or 4-carboxy-5,8,11-this(carboxymethyl)-1-phenyl-2-oxa-5,8,11-triazatridecan-13-oic acid (BOPTA);
Y is the derivative of a bile acid, i.e a residue of cholic or chenodeoxycholic or deoxycholic or ursodeoxycholic or lithocholic acids, 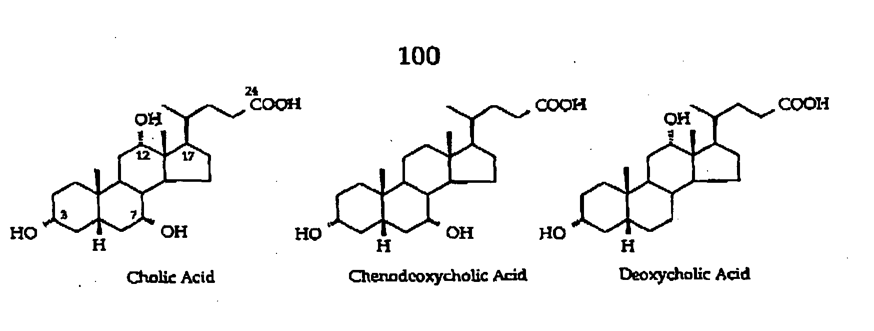 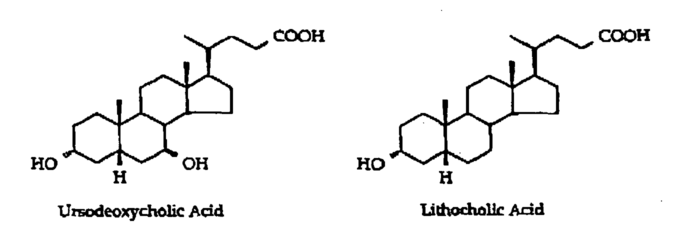 both as such and functionalized at the positions having the hydroxy group as the reactive group, independently of the stereochemistry of the final products,
said derivative also comprising the conjugate of the acid group at the 24 position with taurine and glycine;
L is a chain linked to any position of X, optionally comprising one of the carboxylic groups which is thus transformed into an amide group, and to the C-3, C-7, C-12 positions of Y, having the following formula (II) 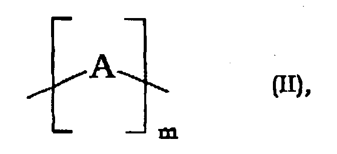
in which
m is an integer ranging from 1 to 10, wherein for values above 1, A can have different meanings,
A has the following formula (III), 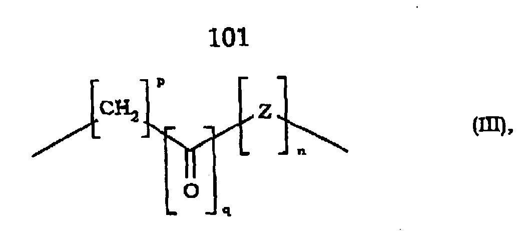
n and q can be 0 or 1, but they are not at the same time zero,
p can range from 0 to 10,
Z is an oxygen atom or a -NR group,
in which
R is a hydrogen atom, or a (C₁-C₅) alkyl group unsubstituted or substituted by a -COOH group;
for the preparation of diagnostic formulations for obtaining images of the blood system of the human and animal body, by means of nuclear magnetic resonance.

2. The use according to claim 1, in which the paramagnetic ions are Gadolinium or Manganese ions.

3. The use according to claim 1, in which the spacing chains L have the formulae (IIIa) and (IIIb).

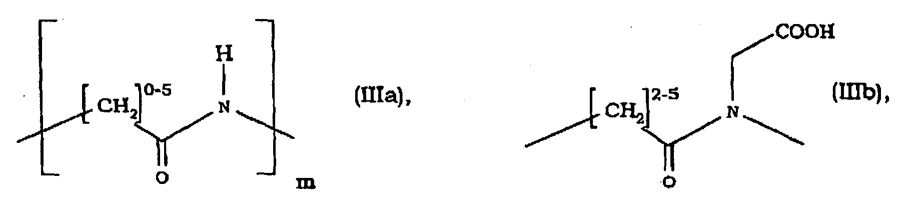

4. The use according to claim 1, in which Z is an oxygen atom and L is thereby formed through the hydroxy groups present at the 3, 7, 12 positions, independently of the stereochemistry of the final products.

5. The use according to claim 1, in which:
the residue X is selected from the group consisting of: EDTA, DTPA, DOTA, DO3A, BOPTA;
L is(IIIa) or (IIIb) 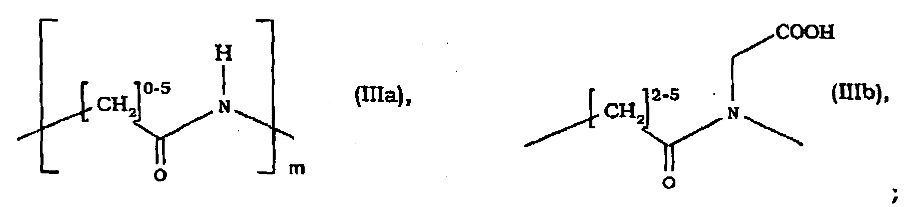 Y is a residue of cholic or deoxycholic or chenodeoxycholic or lithocholic acids, as they are or in which one or more hydroxy groups have been transformed into keto groups, linked to L by an amino group at the 3- position, the acid group at the 24 position being present as it is or as its taurine or glycine derivative;
the paramagnetic ions are gadolinium or manganese ions and
the organic bases are ethanolamine or diethanolamine or morpholine or glucamine or N-methylglucamine or N,N-dimethylglucamine.

6. The use according to claim 1, in which in the compounds of formula (I) the residue X is DTPA substituted on the central chain, resulting in compounds of formula (IV) 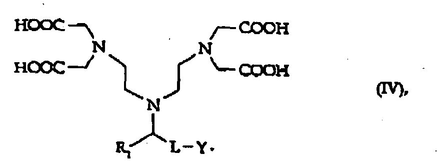 wherein:
R₁ is a hydrogen atom or the -COOH group,
Y is of a residue of cholic or deoxycholic or chenodeoxycholic or lithocholic acids and
L has the structure of formula (III).

7. T**he use according to claim 6, in which in the compounds of formula (IV) R₁ is the -COOH group, resulting in compounds of formula (IVa)** 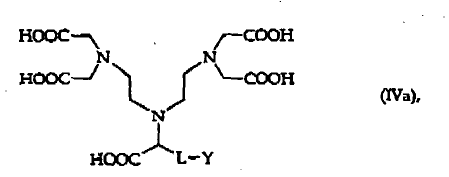 wherein Y is a residue of cholic or deoxycholic or chenodeoxycholic/lithocholic acids and L is formulae (IIIa) or (IIIb)

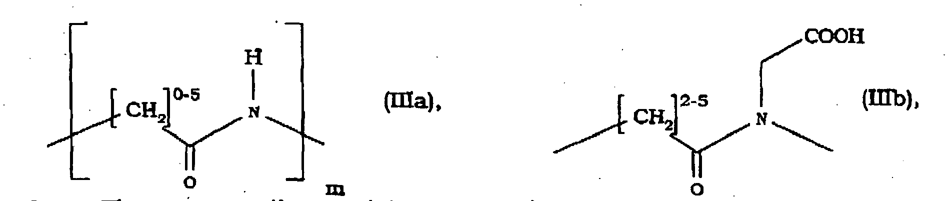

8. **The use according to claim 7, wherein the compound of formula (IVa) is**
[3β(S),5β]-3-[[4-[bis[2-[bis(carboxymethyl)amino]ethyl]-amino]-4-carboxy-1-oxobutyl](carboxymethyl)amino]cholan-24-oic acid or
[3β(S),5β]-3-[[4-[bis[2-(bis(carboxymethyl)amino]ethyl]-amino]-4-carboxy-1-oxobutyl]amino]cholan-24-oic acid or
[3β(S),5β]-3-[[4-[bis[2-[bis(carboxymethyl)amino]ethyl]amino]-4-carboxy-1-oxobutyl]amino]-12-oxocholan-24-oic acid or
[3β(S),5β,7α]-3-[[4-[bis[2-[bis(carboxymethyl)amino]ethyl]amino)-4-carboxy-1-oxobutyl]amino]-7-hydroxycholan-24-oic acid or
N²-bis[2-[bis(carboxymethyl)amino]ethyl]-N-[(3β,5β)-24-oxo-24-[(2-sulfoethyl) amino]cholan-3-yl]-L-glutamine or
N²-bis[2-[bis(carboxymethyl)amino]ethyl]-N-[(3β,5β,7α,12α)-7,12-dihydroxy-24-oxo-24-[(2-sulfoethyl)amino]-cholan-3-yl]-L-glutamine, or
[3β(S),5β,7β]-3-[[4-[bis(2-[bis(carboxymethyl)amino]ethyl]amino]-4-caxbooy-1-oxobutyl]amino]-7-hydroxycholan-24-oic acid or
[3β(R),5β,12α]-3-[[4-[bis[2-[bis(carboxyutethyl)amino]ethyl]amino]-4-carboxy-1-oxobutyl]amino]-12-hydroxycholan-24-oic acid or
[3β(RS),5β,12α]-3-[[4-[bis[2-[bis(carboxymethyl)amino]ethyl]amino]-4-carboxy-1-oxobutyl]amino]-12-hydroxycholan-24-oic acid or
[3β(RS),5β,7α,12α]-3-[[4-[bis[2-[bis(carboxynnethyl)-amino]ethyl]amino]-4-carboxy-1-oxobutyl]amino]-7,12-dihydroxycholan-24-oic acid or
[3β(S),5β,7α,12α]-3-[[4-[bis[2-[bis(carboxymethyl}amino]ethyl]amino]-4-carboxy-1-oxobutyl]amino-7,12-dihydroxycholan-24-oic acid or
[3α(S),5β]-3-[[4-[bis[2-[bis(carboxymethyl)amino]-ethyl]amino]-4-carboxy-1-oxobutyl]-amino]]cholan-24-oic acid or
[3β(S),5β,12α]-3-[[4-[bis[2-[bis(carboxymethyl}amino]-ethyl]amino]-4-carboxy-2-oxobutyl]amino]-12-hydroxyoholan-24-oic acid or
[3α(S),5β,12]-3-[[4-[bis[2-[bis(carboxymethyl)amino]ethyl]amino]-4-carboxy-1-oxobutyl]amino]-12-hydroxycholan-24-oic acid.

9. The use according to claim 6, in which in formula (IV) R₁ is hydrogen, resulting in compounds of formula (IVb) 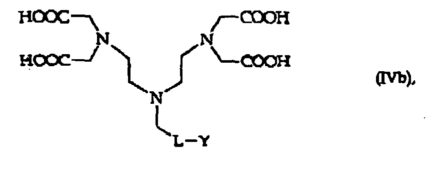 wherein Y is a residue of cholic, deoxycholic, chenodeoxycholic, lithocholic acids and L has the formula (IIIa)

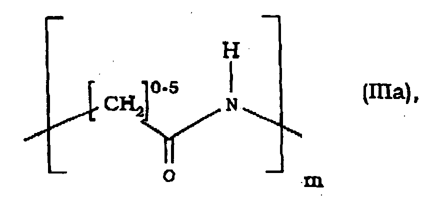

10. **The use according to claim 9, wherein the compound of formula (IVb) is**
(3β,5β,7α,12α)-3-[[[bis[2-[bis(carboxymethyl)amino]ethyl]amino]acetyl]amino]-7,12-dihydroxycholan-24-oic acid or
(3β,5β)-3-[[[[[bis[2-[bis(carboxymethyl)amino]ethyl]amino]acetyl]amino]acetyl] amino]cholan-24-oic acid or
(3β,5β,7α,12α)-3-[[[[[bis[2-[bis(carboxymethyl)amino]ethyl]amino]acetyl]amino] acetyl]amino]-7,12-dihydroxycholan-24-oic acid or
(3β,5β,7α,12α)-3-[[6-[[[bis[2-[bis(carboxymethyl)amino]ethyl]amino]acetyl]amino]-1-oxohexyl]amino]-7,12-dihydroxycholan-24-oic.

11. **The use according to claim 1, in which in the compounds of formula (I) the residue X is DTPA, resulting in compounds of formula (V)** 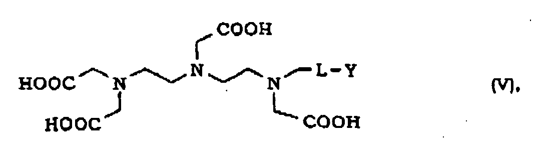 wherein Y is a residue of cholic of deoxycholic or chenodeoxycholic or lithocholic acids and L has the formula (IIIa).

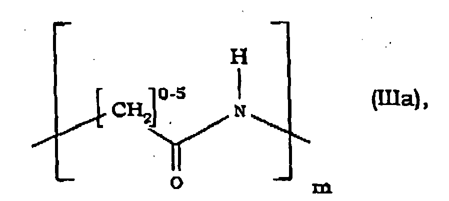

12. **The use according to claim 11, wherein the compound of formula (V) is**
(3β,5β,7α,12α)-3-[[N-[N-[2-[[2-[bis(carboxymethyl)amino] ethyl](carboxymethyl) amino]ethyl]-N-(carboxymethyl)glycyl]glycyl]amino]-7,12-dihydroxycholan-24-oic acid, or
18-[[(3β,5β,7α,12α)-23-carboxy-7,12-hydroxy-24-norcholan-3-yl]amino]-3,6,9-tris(carboxymethyl)-11,18-dioxo-3,6,9,12-tetraazaoctadecanoic acid.

13. The use according to claim 1, in which in the compounds of formula (I) the residue X is DO3A, **resulting in compounds of formula (VI)** 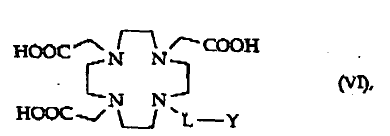 wherein Y is a residue of cholic or deoxycholic or chenodeoxycholic or lithocholic acids and L is formulae (IIIa) or (IIIb)

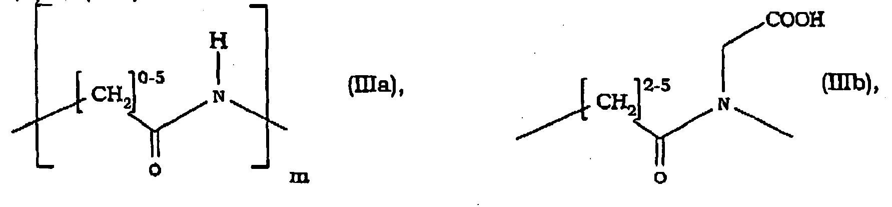

14. **The use according to claim 1**, in which in the compounds of formula (I) the residue X is EDTA, **resulting in compounds of formula (VII)** 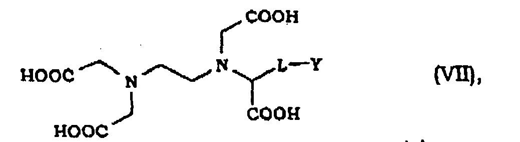 **wherein Y is** a residue of cholic or deoxycholic or chenodeoxycholic or lithocholic acids and L has the formula (III).

15. **The use according to claim 14, in which the compound of formula (VII).** is
[3α(S),5β,12α]-3-[[[[5-[[2-[bis(carboxymethyl)amino]ethyl](carboxymethyl)amino]-5-carboxypentyl]amino]carbonyl]oxy]-12-hydxoxycholan-24-oic acid or
[3β(S),5β,7α,12α]-3-[[4-[[5-[[2-[bis(carboxymethyl)amino]ethyl](carboxymethyl) amino]-5-carboxypentyl]amino]-1,4-dioxobutyl]amino]-7,12-di-hydroxycholan-24-oic acid or
[3β(S),5β]-3-[2-[[5-[[2-[bis(carboxymethyl)amino]ethyl](carboxymethyl)amino]-5-carboxypentyl]amino]-2-oxoethoxy]-cholan-24-oic acid or
[3β(S),5β,12α]-3-[[4-[[2-[[bis(carboxymethyl)amino]ethyl] (carboxymethyl)amino]-4-carboxy-1-oxobutyl]amino]-12-hydroxycholan-24-oic acid, or
[3β(S),5β]-3-[[4-[[2-[[bis(carboxymethyl)amino]ethyl]-(carboxymethyl)amino]-4-carboxy-1-oxobutyl]amino]-12-oxocholan-24-oic acid.

16. **The use according to claim 1 wherein the compound of formula (I) is**
[3α(S),5β,7α,12α]-3-[[[[5-[bis[2-[bis(carboxymethyl)amino]ethyl]amino]-5-carboxypentyl]amino]carbonyl]oxy]-7,12-dihydroxycholan-24-oic acid, or
[3α(S),5β]-3-[2-[[5-[bis[2-[bis(carboxymethyl)ammo]ethyl]amino]-5-carboxypenty]amino]-2-oxoethoxy]cholan-24-oic acid, or
[3β(S),5β,7α,12α]-3-[[4-[[5-[bis[2-[bis(carboxymethyl)amino]ethyl]amino]-5-carboxypentyl]amino]-1,4-dioxobutyl]amino]-7,12-dihydroxycholan-24-oic acid or
10-[3-[[(3α,5β,7α,12α)-23-carboxy-7,12-dihydroxy-24-norcholan-3-yl]oxy]-2-hydroxypropyl]-1,4,7,10-tetraazacyclododecane-1,4,7-triacetic acid

17. **The use according to any one of claims 1 to 16,** in which the chelated complex salts are formed with sodium or N-methylglucamine.

## Patentansprüche

1. Verwendung von Chelatkomplexen von bi- trivalenten paramagnetischen Metallionen, welche Fe⁽²⁺⁾ oder Fe⁽³⁺⁾ oder Cu⁽²⁺⁾ oder Cr⁽³⁺⁾ oder Gd⁽³⁺⁾ oder Eu⁽³⁺⁾ oder _{D}y (³⁺) oder Yb⁽³⁺⁾ oder Mn⁽²⁺⁾ sind, mit den Verbindungen der Formel (I) wie auch deren Salzen mit physiologisch verträglichen organischen Basen, welche primäre, sekundäre, tertiäre Amine oder basische Aminosäuren sind oder mit anorganischen Basen, deren Kationen Natrium, Kalium, Magnesium, Calcium sind oder deren Mischungen:
X-L-Y (I),
wobei:
X der Rest eines Polyaminocarbonsäure-Liganden oder dessen Derivaten ist, d.h. Ethylendiamintetraessigsäure (EDTA) oder Diethylentriaminpentaessigsäure (DTPA) oder 1,4,7,10-Tetraazacyclododecan-1,4,7,10-tetraessigsäure (DOTA) oder 1,4,7,10- Tetraazacyclododecan-1,4,7-triessigsäure (D03A) oder [10-(2-Hydroxypropyl)-1,4,7,10-tetraazacyclododecan-1,4,7-triessigsäure (HPD03A) oder 4-Carboxy-5,8,11-tris(carboxymethyl)-1-phenyl-2-oxa-5,8,11-triazatridecan-13-säure (BOPTA) ;
Y das Derivat einer Gallensäure ist, d.h. ein Rest aus Chol- oder Chenodesoxychol- oder Desoxycholoder Ursodesoxychol- oder Lithocholsäuren, 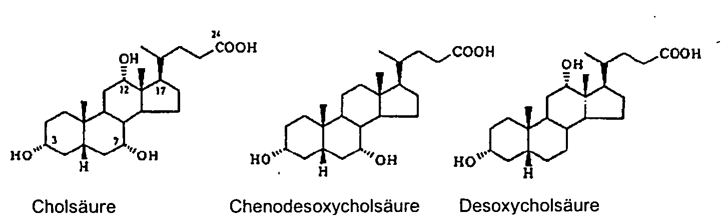 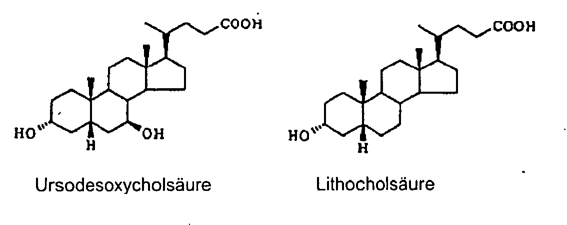
sowohl als solche als auch funktionalisiert an den Positionen, welche die Hydroxygruppe als die reaktive Gruppe aufweisen, unabhängig von der Stereochemie des Endproduktes,
wobei die genannten Derivate ebenfalls Konjugate der Säuregruppe an der Position 24 mit Taurin und Glycin umfassen;
L eine Kette ist, welche an jeder beliebigen Position von X, die gegebenenfalls eine der Carbonsäuregruppen umfasst, welche so in eine Amidgruppe transformiert ist und an die C-3, C-7, C-12 Positionen von Y gebunden ist, welche die folgende Formel (II) aufweist 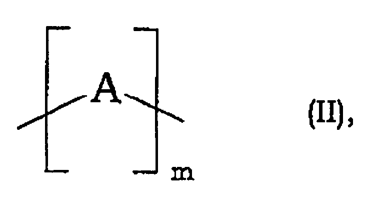
in welcher
m eine ganze Zahl von 1 bis 10 ist, wobei für Werte größer als 1 A verschiedene Bedeutungen haben kann,
A die nachfolgende Formel (III) aufweist, 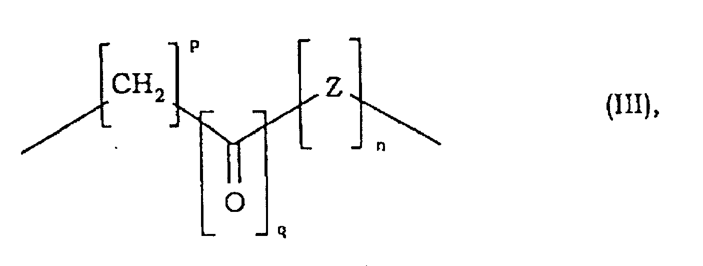
n und q 0 oder 1 sein können, aber nicht gleichzeitig 0 sind,
p von 0 bis 10 reichen kann,
Z ein Sauerstoffatom oder eine -NR-Gruppe ist,
in welcher
R ein Wasserstoffatom oder eine (C₁-C₅)-Alkylgruppe bedeutet, die unsubstituiert oder mit einer-COOH-Gruppe substituiert ist;
für die Herstellung von diagnostischen Formulierungen zum Erhalt von Bildern des Blutsystems des menschlichen und tierischen Körpers mittels Nuklearmagnetresonanz.

2. Verwendung gemäß Anspruch 1, wobei die paramagnetischen Ionen Gadolinium- oder Manganionen sind.

3. Verwendung gemäß Anspruch 1, wobei die Abstandsketten L die Formeln (IIIa) und (IIIb) aufweisen

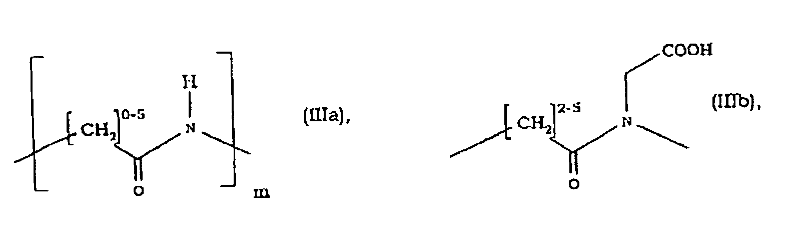

4. Verwendung gemäß Anspruch 1, wobei Z ein Sauerstoffatom ist und L **dadurch** durch die Hydroxygruppen, welche an den Positionen 3, 7, 12 vorliegen, gebildet wird, unabhängig von der Stereochemie des Endproduktes.

5. Verwendung gemäß Anspruch 1, wobei:
der Rest X ausgewählt ist aus der Gruppe bestehend aus: EDTA, DTPA, DOTA, DO3A, BOPTA; L (IIIa) oder (IIIb) ist 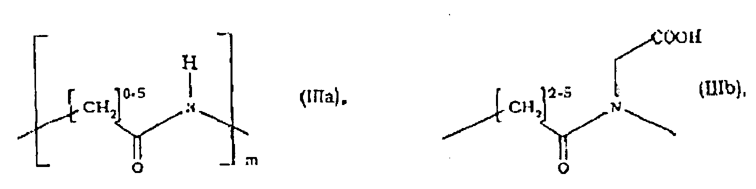
Y ein Rest aus Chol- oder Desoxychol- oder Chenodesoxychol- oder Lithocholsäuren ist, so wie sie sind oder in welchen eine oder mehr Hydroxygruppen zu Ketogruppen transformiert worden sind, verbunden mit L durch eine Aminogruppe an der 3-Position, wobei die Säuregruppe an der Position 24 so vorliegt, wie sie ist oder als ihr Taurin- oder Glycinderivat;
die paramagnetischen Ionen Gadolinium- oder Manganionen sind und die organischen Basen Ethanolamin oder Diethanolamin oder Morpholin oder Glucamin oder N-Methylglucamin oder N,N-Dimethylglucamin sind.

6. Verwendung gemäß Anspruch 1, wobei in den Verbindungen der Formel (I) der Rest X DTPA ist, substituiert an der zentralen Kette, welches Verbindungen der Formel (IV) ergibt 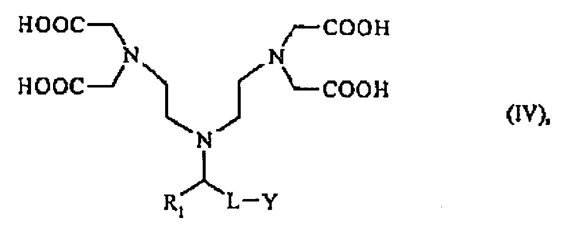 wobei:
R₁ ein Wasserstoffatom oder die -COOH-Gruppe ist,
Y ein Rest aus Chol- oder Desoxychol- oder Chenodesoxychol- oder Lithocholsäuren ist und
L die Struktur gemäß Formel (III) aufweist.

7. Verwendung gemäß Anspruch 6, wobei in den Verbindungen von Formel (IV) R₁ die -COOH-Gruppe ist, welches Verbindungen der Formel (IVa) ergibt 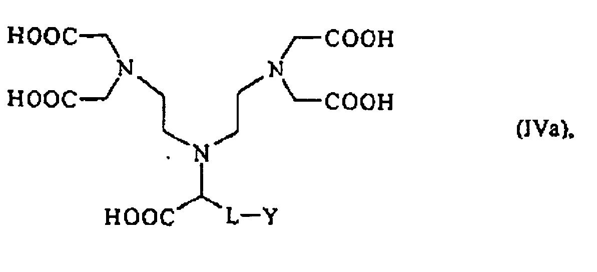 wobei Y ein Rest aus Chol- oder Desoxychol- oder Chenodesoxychol- oder Lithocholsäuren ist und L Formel (IIIa) oder (IIIb) ist

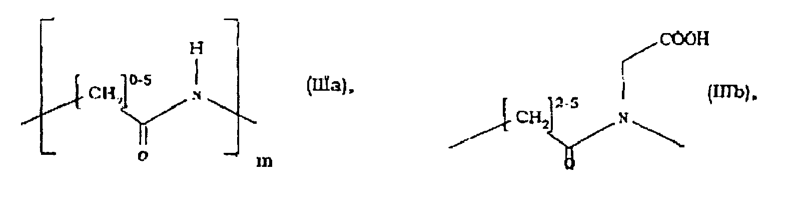

8. Verwendung gemäß Anspruch 7, wobei die Verbindung der Formel (IVa)
[3β(S),5β]-3-[[4-[Bis[2-[bis(carboxymethyl)amino]-ethyl]amino]-4-carboxy-1-oxobutyl](carboxymethyl)-amino]-cholan-24-säure oder
[3β(S),5β]-3-[[4-[Bis[2-[bis(carboxymethyl)amino]-ethylamino]-4-carboxy-1-oxobutyl]amino] cholan-24-säure oder
[3β(S),5β)-3-[[4-[Bis[2-[bis(carboxymethyl)amino]-ethylamino]-4-carboxy-1-oxobutyl]amino]-12-oxocholan-24-säure oder;
[3β(S),5β, 7α]-3-[[4-[Bis[2-[bis(carboxymethyl)amino]-ethyl]amino]-4-carboxy-1-oxobutyl]amino]-7-hydroxy-cholan24-säure oder
N²-Bis[2-[bis(carboxymethyl)amino]ethyl]-N-[(3β,5β)-24-oxo-24-[(2-sulfethyl)amino]cholan-3-yl]-L-glutamin oder
N²-Bis[2-[bis(carboxymethyl)amino]ethyl]-N-[(3β,5β,-7α,12α)-7,12-dihydroxy-24-oxo-24-[(2-sulfethyl)amino]-cholan-3-yl]-L-glutamin oder
[3β(S),5β,7β)-3-[[4-[Bis[2-[bis(carboxymethyl)aminoethyl]amino]-4-carboxy-1-oxobutyl]amino]-7-hydroxy-cholan24-säure oder
[3β(R),5β,12α]-3-[[4-[Bis[2-[bis(carboxymethyl)-amino]ethyl]amino]-4-carboxy-1-oxobutyl]amino]-12-hydroxycholan-24-säure oder
[3β(RS),5β,12α]-3-[[4-[Bis[2-[bis(carboxymethyl)-amino]ethyl]amino]-4-carboxy-1-oxobutyl]amino]-12-hydroxycholan-24-säure oder
[3β(RS),5β,7α,12α]-3-[[4-[Bis[2-[bis(carboxymethyl)-amino]ethyl]amino]-4-carboxy-1-oxobutyl]amino]-7,12-dihydroxycholan-24-säure oder
[3β(S),5β,7α,12α]-3-[[4-[Bis[2-[bis(carboxymethyl)-amino]ethyl]amino]-4-carboxy-1-oxobutyl]amino]-7,12-dihydroxycholan-24-säure oder
[3α(S),5β]-3-[[4-[Bis[2-[bis(carboxymethyl)amino]-ethyl]amino]-4-carboxy-1-oxobutyl]amino]]cholan-24-säure oder
[3β(S),5β,12α]-3-[[4-[Bis[2-[bis(carboxymethyl)amino]-ethyl]amino]-4-carboxy-1-oxobutyl]amino]-12-hydroxy-cholan-24-säure oder
[3α(S),5β,12α]-3-[[4-[Bis[2-[bis(carboxymethyl)amino]-ethyl]amino]-4-carboxy-1-oxobutyl]amino]-12-hydroxy-cholan-24-säure ist.

9. Verwendung gemäß Anspruch 6, wobei in Formel (IV) R₁ Wasserstoff ist, welches zu Verbindungen der Formel (IVb) führt 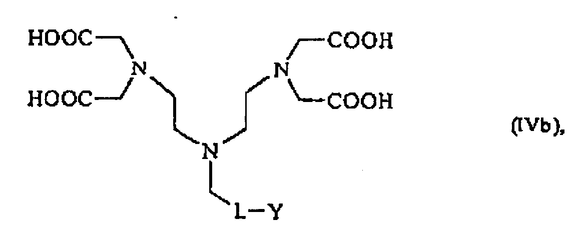 wobei Y ein Rest aus Chol-, Desoxychol-, Chenodesoxychol-, Lithocholsäuren ist und L die Formel (IIIa) aufweist

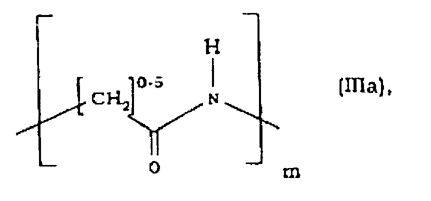

10. Verwendung nach Anspruch 9, wobei die Verbindung der Formel (IVb)
(3β,5β,7α,12α)-3-[[[Bis[2-[bis(carboxymethyl)amino]-ethyl]amino]acetyl]amino]-7,12-dihydroxycholan-24-säure oder
(3β,5β)-3-[[[[[Bis[2-[bis(carboxymethyl)amino]-ethyl]amino]acetyl]amino]acetyl]amino]cholan-24-säure oder
(3β,5β,7α,12α)-3-[[[[[Bis[2-[bis(carboxymethyl)amino]-ethyl]amino]acetyl]amino]acetyl]amino]-7,12-dihydroxy-cholan-24-säure oder
(3β,5β,7α,12α)-3-[[6[[[Bis[2-[bis(carboxymethyl)-amino]ethyl]amino]acetyl]amino]-1-oxohexyl]amino]-7,12-dihydroxycholan-24-säure ist.

11. Verwendung gemäß Anspruch 1, wobei in den Verbindungen der Formel (I) der Rest X DTPA ist, welches Verbindungen der Formel (V) ergibt 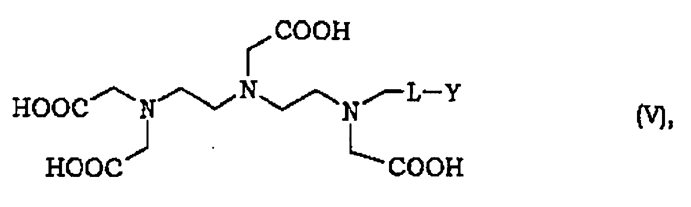 wobei Y ein Rest aus Chol- oder Desoxychol- oder Chenodesoxychol- oder Lithocholsäuren ist und L die Formel (IIIa) aufweist

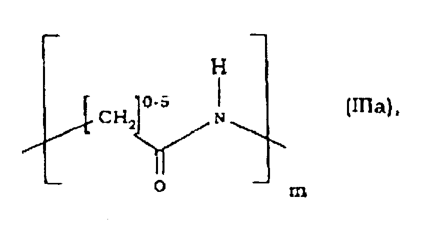

12. Verwendung gemäß Anspruch 11, wobei die Verbindung der Formel (V)
(3β,5β,7α,12α)-3-[[N-[N-[2-[[2-[Bis(carboxymethyl)-amino]ethyl] (carboxymethyl)amino]ethyl]-N-(carboxymethyl)glycyl]glycyl]amino]-7,12-dihydroxycholan-24-säure oder
18-[[(3β,5β,7α,12α)-23-Carboxy-7,12-hydroxy-24-norcholan-3-yl]amino]-3,6,9-tris(carboxymethyl)-11,18-dioxo-3,6,9,12-tetraazaoctadecansäure ist.

13. Verwendung gemäß Anspruch 1, wobei in den Verbindungen der Formel (I) der Rest X DO3A ist, welches Verbindungen der Formel (VI) ergibt 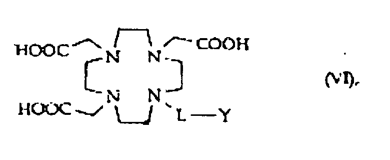 wobei Y ein Rest aus Chol- oder Desoxychol- oder Chenodesoxychol- oder Lithocholsäuren ist und L Formel (IIIa) oder (IIIb) ist

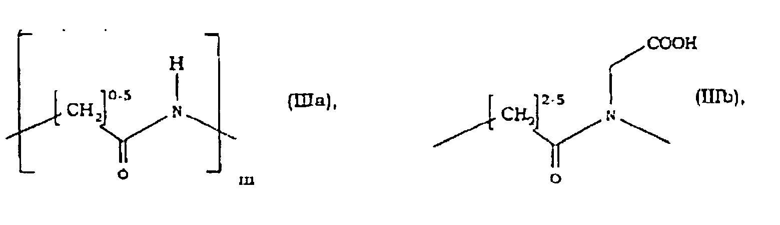

14. Verwendung gemäß Anspruch 1, wobei in den Verbindungen der Formel (I) der Rest X EDTA ist, welches Verbindungen der Formel (VII) ergibt 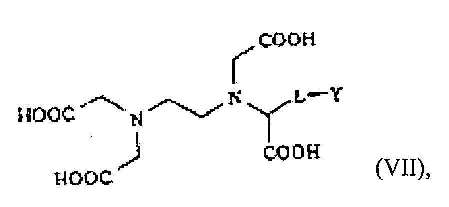 wobei Y ein Rest aus Chol- oder Desoxychol- oder Chenodesoxychol- oder Lithocholsäuren ist und L die Formel (III) aufweist.

15. Verwendung gemäß Anspruch 14, wobei die Verbindung der Formel (VII)
[3α(S),5β,12α]-3-[[[[5-[[2-[Bis(carboxymethyl)-amino]ethyl](carboxymethyl)amino]-5-carboxypentyl]-amino]carbonyl]oxy]-12-hydroxycholan-24-säure oder
[3β(S),5β,7α,12α]-3-[[4-[[5-[[2-[Bis(carboxymethyl)-amino]ethyl](carboxymethyl)amino]-5-carboxypentyl]-amino]-1,4-dioxobutyl]amino]-7,12-dihydröxycholan-24-säure oder
[3β(S),5β]-3-[2-[[5-[[2-[Bis(carboxymethyl)amino]-ethyl](carboxymethyl)amino]-5-carboxypentyl]amino]-2-oxoethoxy]-cholan-24-säure oder
[3β(S),5β,12α]-3-[[4-[[2-[[Bis(carboxymethyl)amino]-ethyl](carboxymethyl)amino]-4-carboxy-1-oxobutyl]-amino]-12-hydroxycholan-24-säure oder
[3β(S),5β]-3-[[4-[[2-[[Bis(carboxymethyl)amino]ethyl]-(carboxymethyl)amino]-4-carboxy-1-oxobutyl]amino]-12-oxocholan-24-säure ist.

16. Verwendung gemäß Anspruch 1, wobei die Verbindung der Formel (I)
[3α(S),5β,7α,12α]-3-[[[[5-[Bis[2-[bis(carboxymethyl)-amino]ethyl]amino]-5-carboxypentyl]amino]carbonyl]-oxy]-7,12-dihydroxycholan-24-säure oder
[3α(S),5β]-3-[2-[[5-[Bis[2-[bis(carboxymethyl)amino]-ethyl]amino]-5-carboxypentyl]amino]-2-oxoethoxy]-cholan-24-säure oder
[3β(S),5β,7α,12α]-3-[[4-[[5-[Bis[2-[bis(carboxymethyl)amino]ethyl]amino]-5-carboxypentyl]amino]1,4-dioxobutyl]amino]-7,12-dihydroxycholan-24-säure oder
10-[3-[[(3α,5β,7α,12α)-23-carboxy-7,12-dihydroxy-24-norcholan-3-yl]oxy]-2-hydroxypropyl]-1,4,7,10-tetra-azacyclododecan-1,4,7-triessigsäure ist.

17. Verwendung nach einem der Ansprüche 1 bis 16, wobei die Chelatkomplexsalze mit Natrium oder N-Methylglucamin ausgebildet sind.

## Revendications

1. Utilisation des complexes chélatés d'ions métalliques paramagnétiques bi- ou trivalents qui sont Fe⁽²⁺⁾ ou Fe⁽³⁺⁾ ou Cu⁽²⁺⁾ ou Cr⁽³⁺⁾ ou Gd⁽³⁺⁾ ou Eu⁽³⁺⁾ ou Dy⁽³⁺⁾ ou Yb ⁽³⁺⁾ ou Mn⁽²⁺⁾ avec les composés de formule (I), de même que les sels de ceux-ci avec des bases organiques physiologiquement compatibles qui sont des amines primaires, secondaires, tertiaires ou des acides aminés basiques, ou avec des bases inorganiques dont les cations sont le sodium, le potassium, le magnésium, le calcium ou des mélanges de ceux-ci :
X-L-Y (I),
dans laquelle :
X est le résidu d'un ligand polyaminocarboxylique ou de ses dérivés, c'est-à-dire l'acide éthylènediamine-tétraacétique (EDTA) ou l'acide diéthylène-triamine-pentaacétique (DTPA) ou l'acide 1,4,7,10-tétraazacyclododécane-1,4,7,10-tétraacétique (DOTA) ou l'acide 1,4,7,10-tétraazacyclododécane-1,4,7-triacétique (DO3A) ou l'acide [10-(2-hydroxypropyl)-1,4,7,10-tétraazacyclododécane-1,4,7-triacétique (HPDO3A) ou l'acide 4-carboxy-5,8,11-tris(carboxyméthyl)-1-phényl-2-oxa-5,8,11-triazatridécan-13-oïque (BOPTA) ;
Y est le dérivé d'un acide biliaire, c'est-à-dire un résidu d'acide cholique ou chénodésoxycholique ou désoxycholique ou ursodésoxycholique ou lithocholique, 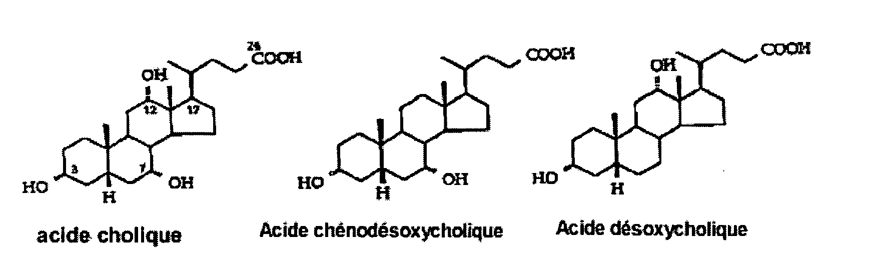 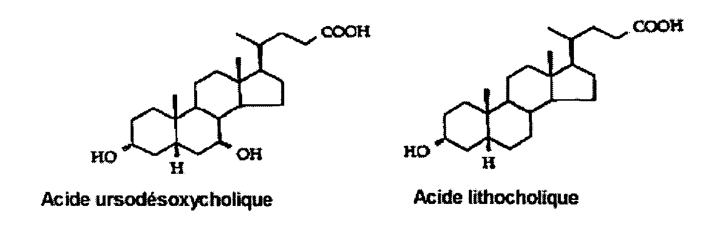 chacun tel quel, et fonctionnalisé aux positions ayant le groupement hydroxy comme groupement réactif, indépendamment de la stéréochimie des produits finaux,
ledit dérivé comprenant aussi le conjugué du groupement acide à la position 24 avec la taurine et la glycine ;
L est une chaîne liée à une quelconque position de X, comprenant éventuellement un des groupements carboxyliques qui est ainsi transformé en un groupement amide, et aux positions C-3, C-7, C-12 de Y, ayant la formule suivante (II), 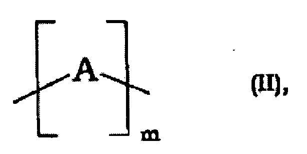 dans laquelle
m est un nombre entier compris entre 1 et 10, dans lequel pour les valeurs supérieures à 1, A peut avoir des significations différentes,
A a la formule suivante (III), 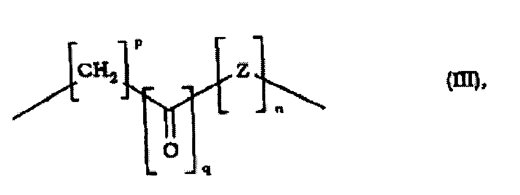
n et q peuvent être 0 ou 1, mais ne sont pas zéro en même temps,
p est compris entre 0 et 10,
Z est un atome d'oxygène ou un groupement -NR,
dans lequel,
R est un atome d'hydrogène, ou un groupement alkyle en C₁ à C₅ non substitué ou substitué par un groupement -COOH ;
pour la préparation de formulations de diagnostic destinées à l'obtention d'images du système sanguin du corps humain et animal, au moyen de la résonance magnétique nucléaire.

2. Utilisation selon la revendication 1, dans laquelle les ions paramagnétiques sont les ions Gadolinium ou Manganèse.

3. Utilisation selon la revendication 1, dans laquelle les chaînes d'espacement L ont les formules (IIIa) et (IIIb)

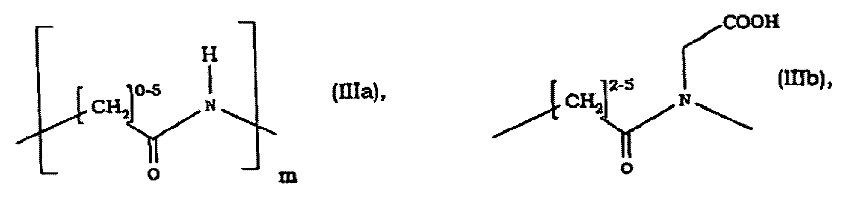

4. Utilisation selon la revendication 1, dans laquelle Z est un atome d'oxygène et L est en conséquence formé par les groupements hydroxy présents aux positions 3, 7, 12, indépendamment de la stéréochimie des produits finaux.

5. Utilisation selon la revendication 1, dans laquelle :
le résidu X est choisi dans le groupe constitué de : EDTA, DTPA, DOTA, D03A, BOPTA ; L est (IIIa) ou (IIIb) 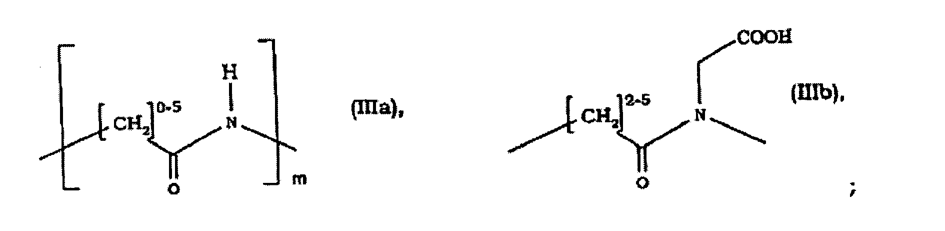
Y est un résidu d'acide cholique ou désoxycholique ou chénodésoxycholique ou lithocholique, tel quel ou dans lequel un ou plusieurs groupements hydroxy ont été transformés en groupements céto, lié à L par un groupement amino en position 3, le groupement acide en position 24 étant présent tel quel ou comme son dérivé taurine ou glycine ;
les ions paramagnétiques sont les ions gadolinium ou manganèse et les bases organiques sont l'éthanolamine ou la diéthanolamine ou la morpholine ou la glucamine ou la N-méthylglucamine ou la N,N-diméthylglucamine.

6. Utilisation selon la revendication 1, dans laquelle dans les composés de formule (I), le résidu X est le DTPA substitué sur la chaîne centrale, résultant dans les composés de formule (IV) : 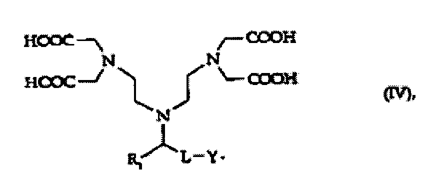 dans laquelle :
R₁ est un atome d'hydrogène ou le groupement -COOH,
Y est un résidu d'acide cholique ou désoxycholique
ou chénodésoxycholique ou lithocholique, et
L a la structure de formule (III).

7. Utilisation selon la revendication 6, dans laquelle dans les composés de formule (IV), R₁ est le groupement -COOH, résultant dans les composés de formule (IVa) : 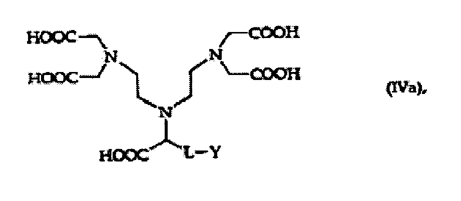 dans laquelle Y est un résidu d'acide cholique ou désoxycholique ou chénodésoxycholique ou lithocholique, et L est la formule (IIIa) ou (IIIb)

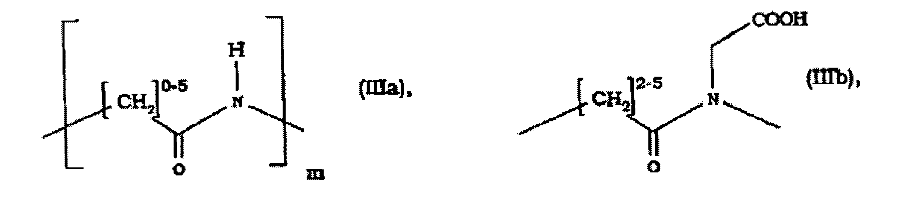

8. Utilisation selon la revendication 7, dans laquelle le composé de formule (IVa) est l'acide
[3β(S),5β]-3-[[4-[bis [2-[bis(carboxyméthyl)amino]éthyl]-amino]-4-carboxy-1-oxobutyl](carboxyméthyl)amino]cholan-24-oïque ou
l'acide[3β(S),5β]-3-[[4-[bis[2-[bis(carboxyméthyl)amino]éthyl]-amino]-4-carboxy-1-oxobutyl]amino]cholan-24-oïque ou l'acide [3β(S),5β]-3-[[4-[bis[2-[bis(carboxyméthyl)amino]éthyl]-amino]-4-carboxy-1-oxobutyl]amino]-12-oxocholan-24-oïque ou
l'acide [3β(S),5β,7α]-3-[[4-[bis[2-[bis(carboxyméthyl)amino]éthyl]-amino]-4-carboxy-1-oxobutyl]amino]-7-hydroxycholan-24-oïque oul a N²-bis[2-[bis(carboxyméthyl)amino]éthyl]-N-[(3β,5β)-24-oxo-24-[(2-sulfoéthyl)amino]cholan-3-yl]-L-glutamineou
la N²-bis[2-[bis(carboxyméthyl)amino]éthyl]-N-[(3β,5β,7α,12α)-7,12-dihydroxy-24-oxo-24-[(2-sulfoéthyl)amino]cholan-3-yl]-L-glutamine ou l'acide
[3β(S),5β,7β]-3-[[4-[bis[2-[bis(carboxyméthyl)amino]éthyl]-amino]-4-carboxy-1-oxobutyl]amino]-7-hydroxycholan-24-oïque ou l'acide
[3β(R),5β,12α]-3-[[4-[bis[2-[bis(carboxyméthyl)amino]éthyl]-amino]-4-carboxy-1-oxobutyl]amino]-12-hydroxycholan-24-oïque ou l'acide
[3β(RS),5β,12α]-3-[[4-[bis[2-[bis(carboxyméthyl)amino]éthyl]-amino]-4-carboxy-1-oxobutyl]amino]-12-hydroxycholan-24-oïque ou l'acide
[3β(RS),5β,7α,12α]-3-[[4-[bis[2-[bis(carboxyméthyl)amino]éthyl]-amino]-4-carboxy-1-oxobutyl]amino]-7,12-dihydroxycholan-24-oïque ou
l'acide [3β(S),5β,7α,12α]-3-[[4-[bis[2-[bis(carboxyméthyl)amino]éthyl]-amino]-4-carboxy-1-oxobutyl]amino]-7,12-dihydroxycholan-24-oïque ou
l'acide [3α(S),5β]-3-[[4-[bis[2-[bis(carboxyméthyl)amino]éthyl]-amino]-4-carboxy-1-oxobutyl]amino]-cholan-24-oïque ou l'acide
[3β(S),5β,12α]-3-[[4-[bis[2-[bis(carboxyméthyl)amino]éthyl]-amino]-4-carboxy-1-oxobutyl]amino]-12-hydroxycholan-24-oïque ou l'acide
[3α(S),5β,12α]-3-[[4-[bis[2-[bis(carboxyméthyl)amino]éthyl]-amino]-4-carboxy-1-oxobutyl]amino]-12-hydroxycholan-24-oïque.

9. Utilisation selon la revendication 6, dans laquelle dans la formule (IV), R₁ est l'hydrogène, résultant dans les composés de formule (IVb) : 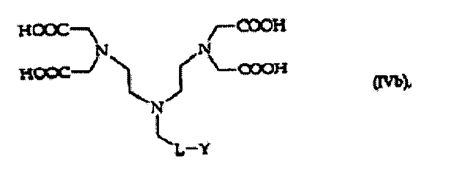 dans laquelle Y est un résidu d'acide cholique ou désoxycholique ou chénodésoxycholique ou lithocholique, et L a la formule (IIIa)

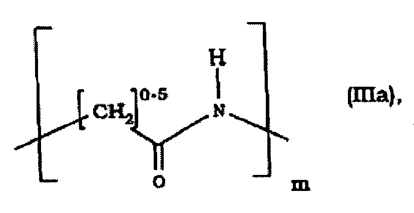

10. Utilisation selon la revendication 9, dans laquelle le composé de formule (IVb) est l'acide
[3β,5β,7α,12α]-3-[[[bis[2-[bis(carboxyméthyl)amino]éthyl]amino]acétyl]amino]-7,12-dihydroxycholan-24-oïque ou l'acide [3β,5β]-3-[[[[[bis[2-bis(carboxyméthyl)amino]éthyl]amino]acétyl]-amino]acétyl]amino]cholan-24-oïque ou l'acide
[3β,5β,7α,12α]-3-[[[[[bis[2bis(carboxyméthyl)amino]éthyl]-amino]acétyl]amino]acétyl]amino]-7,12-dihydroxycholan-24-oïque ou l'acide [3β,5β,7α,12α]-3-[[6- [[[bis[2-[bis(carboxyméthyl)amino]éthyl]amino]acétyl]amino]-1-oxohexyl]amino]-7,12-dihydroxycholan-24-oïque.

11. Utilisation selon la revendication 1, dans laquelle dans les composés de formule (I), le résidu X est DTPA, résultant dans les composés de formule (V) : 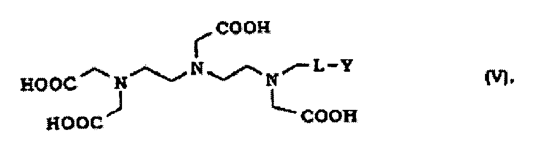 dans laquelle Y est un résidu d'acide cholique ou désoxycholique ou chénodésoxycholique ou lithocholique, et L a la formule (IIIa)

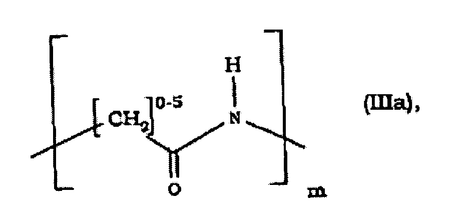

12. Utilisation selon la revendication 11, dans laquelle le composé de formule (V) est l'acide
[3β, 5β, 7α, 12α] -3- [[N- [N- [2- [[2-[bis(carboxyméthyl)amino]éthyl](carboxyméthyl)amino]-éthyl]-N-(carboxyméthyl)glycyl]glycyl]amino]-7,12-dihydroxycholan-24-oïque ou l'acide 18-[[(3β,5β,7α,12α)-23-carboxy-7,12-hydroxy-24-norcholan-3-yl]amino]-3,6,9-tris(carboxyméthyl)-11,18-dioxo-3,6,9,12-tétraazaoctadécanoïque.

13. Utilisation selon la revendication 1, dans laquelle dans les composés de formule (I), le résidu X est D03A, résultant dans les composés de formule (VI) : 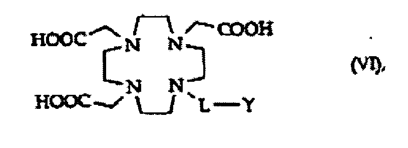 dans laquelle Y est un résidu d'acide cholique ou désoxycholique ou chénodésoxycholique ou lithocholique, et L est la formule (IIIa) ou (IIIb)

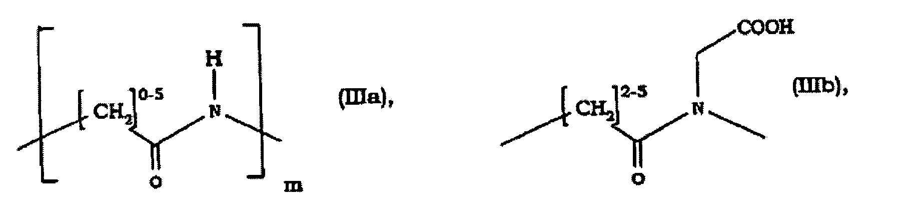

14. Utilisation selon la revendication 1, dans laquelle dans les composés de formule (I), le résidu X est EDTA, résultant dans les composés de formule (VII) : 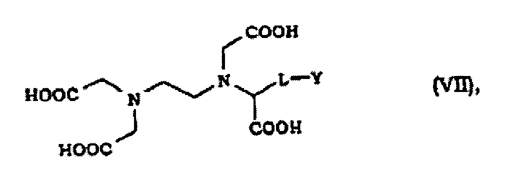 dans laquelle Y est un résidu d'acide cholique ou désoxycholique ou chénodésoxycholique ou lithocholique, et L est la formule (III).

15. Utilisation selon la revendication 14, dans laquelle le composé de formule (VII) est l'acide
[3α(S),5β,12α]-3-[[[[5-[[2-[bis(carboxyméthyl)amino]éthyl](carboxyméthyl)amino]-5-(carboxypentyl]amino]carbonyl]oxy]-12-hydroxycholan-24-oïque ou l'acide [3β(S),5β,7α,12α]-3-[[4-[[5-[[2-[bis(carboxyméthyl)amino]éthyl](carboxyméthyl)amino]-5-(carboxypentyl]amino]-1,4-dioxobutyl]amino]-7,12-di-hydroxycholan-24-oïque ou l'acide [3β(S),5β]-3-[2-[[5-[[2-[bis(carboxyméthyl)amino]éthyl](carboxyméthyl)amino]-5-(carboxypentyl]amino]-2-oxoéthoxy]-cholan-24-oïque ou l'acide [3β(S),5β,12α]-3-[[4-[[2-[bis(carboxyméthyl)amino]éthyl](carboxyméthyl)amino]-4-carboxy-1-oxobutyl]amino]-12-hydroxycholan-24-oïque ou l'acide [3β (S) , 5β] -3- [ [4- [[2-[bis(carboxyméthyl)amino]éthyl](carboxyméthyl)amino]-4-carboxy-1-oxobutyl]amino]-12-oxocholan-24-oïque.

16. Utilisation selon la revendication 1, dans laquelle le composé de formule (I) est l'acide [3α(S),5β,7α,12α]-3- [[[[5-[bis [2-[bis(carboxyméthyl)amino]éthyl]amino]-5-(carboxypentyl]amino]carbonyl]oxy]-7,12-dihydroxycholan-24-oïque ou l'acide [3α(S),5β]-3-[2-[[5-[bis[2-[bis(carboxyméthyl)amino]éthyl]amino]-5-(carboxypentyl]amino]-2-oxoéthoxy]cholan-24-oïque ou l'acide [3β(S),5β,7α,12α]-3-[[4-[[5-[bis(carboxyméthyl)amino]éthyl]-5-carboxypentyl)amino]-1,4-dioxobutyl]amino]-7,12-dihydroxycholan-24-oïque ou l'acide 10-[3-[[(3β,5β,7α,12α)-23-carboxy-7,12-dihydroxy-24-norcholan-3-yl]oxyl]-2-hydroxypropyl]-1,4,7,10-tétraazacyclododécane-1,4,7-triacétique.

17. Utilisation selon l'une quelconque des revendications 1 à 16, dans laquelle les sels complexes chélatés sont formés avec le sodium et la N-méthylglucamine.
